# EUROPEAN PATENT SPECIFICATION

(11) **EP 0 700 444 B1**
(45) Date of publication and mention of the grant of the patent: **02.04.2003**
(21) Application number: 94917380.1
(22) Date of filing: 12.05.1994
(51) Int. Cl.: C12N 15/62, C07K 16/46, C12N 1/21

(54) **IMMUNOTOXINS COMPRISING GELONIN AND AN ANTIBODY**
GELONIN UND EINEN ANTIKÖRPER ENTAHLTENDE IMMUNTOXINE
IMMUNOTOXINES COMPRENANT DE LA GELONINE ET UN ANTICORPS

(30) Priority: 12.05.1993 US 64691
(43) Date of publication of application: 13.03.1996
(73) Proprietor: XOMA Corporation, Berkeley, California 94710 (US)
(72) Inventor: BETTER, Marc, D., Los Angeles, CA 90046 (US); CARROLL, Stephen, F., Walnut Creek, CA 94596 (US); STUDNICKA, Gary, M., Santa Monica, CA 90404 (US)
(74) Representative: Ritter, Stephen David
(86) International application number: US9405348
(87) International publication number: WO94026910

(56) References cited:
- EP-A- 0 506 124
- WO-A-92/22324
- WO-A-93/05168
- WO-A-93/09130
- PROC. NATL. ACAD. SCI. USA vol. 90 , 1993 pages 457 - 461 M. BETTER ET AL.; 'Potent ant-CD5 ricin A immunoconjugates from bacterially produced Fab' and F(ab')2' cited in the application
- J. BIOL. CHEM. vol. 269 , 1994 pages 9644 - 9650 M. BETTER ET AL.; 'Gelonin analogs with engineered cysteine residues form antibody immunoconjugates with unique properties'

### SEQUENCE LISTING

(1) GENERAL INFORMATION:
   (i) APPLICANT: Xoma Corporation
   (ii) TITLE OF INVENTION: Immunotoxins Comprising Ribsome-Inactivating Proteins
   (iii) NUMBER OF SEQUENCES: 144
   (iv) CORRESPONDENCE ADDRESS:
      (A) ADDRESSEE: Marshall, O'Toole, Gerstein, Murray & Borun
      (B) STREET: 6300 Sears Tower, 233 South Wacker Drive
      (C) CITY: Chicago
      (D) STATE: Illinois
      (E) COUNTRY: USA
      (F) ZIP: 60606-6402
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
   (vii) PRIOR APPLICATION DATA:
      (A) APPLICATION NUMBER: US 08/064,691
      (B) FILING DATE: 12-MAY-1993
   (vii) PRIOR APPLICATION DATA:
      (A) APPLICATION NUMBER: US 07/988,430
      (B) FILING DATE: 09-DEC-1992
   (vii) PRIOR APPLICATION DATA:
      (A) APPLICATION NUMBER: PCT US92/09487
      (B) FILING DATE: 04-NOV-1992
   (vii) PRIOR APPLICATION DATA:
      (A) APPLICATION NUMBER: US 07/901,707
      (B) FILING DATE: 19-JUN-1992
   (vii) PRIOR APPLICATION DATA:
      (A) APPLICATION NUMBER: US 07/787,567
      (B) FILING DATE: 04-NOV-1991
   (viii) ATTORNEY/AGENT INFORMATION:
      (A) NAME: Noland, Greta E.
      (B) REGISTRATION NUMBER: 35,302
      (C) REFERENCE/DOCKET NUMBER: 32088
   (ix) TELECOMMUNICATION INFORMATION:
      (A) TELEPHONE: 312/474-6300
      (B) TELEFAX: 312/474-0448
      (C) TELEX: 25-3856
(2) INFORMATION FOR SEQ ID NO:1:
   (i) SEQUENCE CHARACTERISTICS:
      (A) LENGTH: 267 amino acids
      (B) TYPE: amino acid
      (D) TOPOLOGY: linear
   (ii) MOLECULE TYPE: protein
   (xi) SEQUENCE DESCRIPTION: SEQ ID NO:1:
   Ile Phe Pro Lys Gln Tyr Pro Ile Ile Asn Phe Thr Thr Ala Gly Ala
   1               5                   10                  15
   Thr Val Gln Ser Tyr Thr Asn Phe Ile Arg Ala Val Arg Gly Arg Leu
               20                  25                  30
   Thr Thr Gly Ala Asp Val Arg His Glu Ile Pro Val Leu Pro Asn Arg
           35                  40                  45
   Val Gly Leu Pro Ile Asn Gln Arg Phe Ile Leu Val Glu Leu Ser Asn
       50                  55                  60
   His Ala Glu Leu Ser Val Thr Leu Ala Leu Asp Val Thr Asn Ala Tyr
   65                  70                  75                  80
   Val Val Gly Tyr Arg Ala Gly Asn Ser Ala Tyr Phe Phe His Pro Asp
                   85                  90                  95
   Asn Gln Glu Asp Ala Glu Ala Ile Thr His Leu Phe Thr Asp Val Gln
               100                 105                 110
   Asn Arg Tyr Thr Phe Ala Phe Gly Gly Asn Tyr Asp Arg Leu Glu Gln
           115                 120                 125
   Leu Ala Gly Asn Leu Arg Glu Asn Ile Glu Leu Gly Asn Gly Pro Leu
       130                 135                 140
   Glu Glu Ala Ile Ser Ala Leu Tyr Tyr Tyr Ser Thr Gly Gly Thr Gln
   145                 150                 155                 160
   Leu Pro Thr Leu Ala Arg Ser Phe Ile Ile Cys Ile Gln Met Ile Ser
                   165                 170                 175
   Glu Ala Ala Arg Phe Gln Tyr Ile Glu Gly Glu Met Arg Thr Arg Ile
               180                 185                 190
   Arg Tyr Asn Arg Arg Ser Ala Pro Asp Pro Ser Val Ile Thr Leu Glu
           195                 200                 205
   Asn Ser Trp Gly Arg Leu Ser Thr Ala Ile Gln Glu Ser Asn Gln Gly
       210                 215                 220
   Ala Phe Ala Ser Pro Ile Gln Leu Gln Arg Arg Asn Gly Ser Lys Phe
   225                 230                 235                 240
   Ser Val Tyr Asp Val Ser Ile Leu Ile Pro Ile Ile Ala Leu Met Val
                   245                 250                 255
   Tyr Arg Cys Ala Pro Pro Pro Ser Ser Gln Phe
               260                 265
(2) INFORMATION FOR SEQ ID NO:2:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Cys Asp Asp Pro Gly
           35                  40                  45
   Lys Cys Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Asp Lys Asp Pro Lys
                   245                 250
(2) INFORMATION FOR SEQ ID NO:3:
   (i) SEQUENCE CHARACTERISTICS:
      (A) LENGTH: 280 amino acids
      (B) TYPE: amino acid
      (D) TOPOLOGY: linear
   (ii) MOLECULE TYPE: protein
   (xi) SEQUENCE DESCRIPTION: SEQ ID NO:3:
   Ala Ala Lys Met Ala Lys Asn Val Asp Lys Pro Leu Phe Thr Ala Thr
   1               5                   10                  15
   Phe Asn Val Gln Ala Ser Ser Ala Asp Tyr Ala Thr Phe Ile Ala Gly
               20                  25                  30
   Ile Arg Asn Lys Leu Arg Asn Pro Ala His Phe Ser His Asn Arg Pro
           35                  40                  45
   Val Leu Pro Pro Val Glu Pro Asn Val Pro Pro Ser Arg Trp Phe His
       50                  55                  60
   Val Val Leu Lys Ala Ser Pro Thr Ser Ala Gly Leu Thr Leu Ala Ile
   65                  70                  75                  80
   Arg Ala Asp Asn Ile Tyr Leu Glu Gly Phe Lys Ser Ser Asp Gly Thr
                   85                  90                  95
   Trp Trp Glu Leu Thr Pro Gly Leu Ile Pro Gly Ala Thr Tyr Val Gly
               100                 105                 110
   Phe Gly Gly Thr Tyr Arg Asp Leu Leu Gly Asp Thr Asp Lys Leu Thr
           115                 120                 125
   Asn Val Ala Leu Gly Arg Gln Gln Leu Ala Asp Ala Val Thr Ala Leu
       130                 135                 140
   His Gly Arg Thr Lys Ala Asp Lys Ala Ser Gly Pro Lys Gln Gln Gln
   145                 150                 155                 160
   Ala Arg Glu Ala Val Thr Thr Leu Val Leu Met Val Asn Glu Ala Thr
                   165                 170                 175
   Arg Phe Gln Thr Val Ser Gly Phe Val Ala Gly Leu Leu His Pro Lys
               180                 185                 190
   Ala Val Glu Lys Lys Ser Gly Lys Ile Gly Asn Glu Met Lys Ala Gln
           195                 200                 205
   Val Asn Gly Trp Gln Asp Leu Ser Ala Ala Leu Leu Lys Thr Asp Val
       210                 215                 220
   Lys Pro Pro Pro Gly Lys Ser Pro Ala Lys Phe Ala Pro Ile Glu Lys
   225                 230                 235                 240
   Met Gly Val Arg Thr Ala Glu Gln Ala Ala Asn Thr Leu Gly Ile Leu
                   245                 250                 255
   Leu Phe Val Glu Val Pro Gly Gly Leu Thr Val Ala Lys Ala Leu Glu
               260                 265                 270
   Leu Phe His Ala Ser Gly Gly Lys
           275                 280
(2) INFORMATION FOR SEQ ID NO:4:
   (i) SEQUENCE CHARACTERISTICS:
      (A) LENGTH: 263 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Asp Val Asn Phe Asp Leu Ser Thr Ala Thr Ala Lys Thr Tyr Thr Lys
   1               5                   10                  15
   Phe Ile Glu Asp Phe Arg Ala Thr Leu Pro Phe Ser His Lys Val Tyr
               20                  25                  30
   Asp Ile Pro Leu Leu Tyr Ser Thr Ile Ser Asp Ser Arg Arg Phe Ile
           35                  40                  45
   Leu Leu Asp Leu Thr Ser Tyr Ala Tyr Glu Thr Ile Ser Val Ala Ile
       50                  55                  60
   Asp Val Thr Asn Val Tyr Val Val Ala Tyr Arg Thr Arg Asp Val Ser
   65                  70                  75                  80
   Tyr Phe Phe Lys Glu Ser Pro Pro Glu Ala Tyr Asn Ile Leu Phe Lys
                   85                  90                  95
   Gly Thr Arg Lys Ile Thr Leu Pro Tyr Thr Gly Asn Tyr Glu Asn Leu
               100                 105                 110
   Gln Thr Ala Ala His Lys Ile Arg Glu Asn Ile Asp Leu Gly Leu Pro
           115                 120                 125
   Ala Leu Ser Ser Ala Ile Thr Thr Leu Phe Tyr Tyr Asn Ala Gln Ser
       130                 135                 140
   Ala Pro Ser Ala Leu Leu Val Leu Ile Gln Thr Thr Ala Glu Ala Ala
   145                 150                 155                 160
   Arg Phe Lys Tyr Ile Glu Arg His Val Ala Lys Tyr Val Ala Thr Asn
                   165                 170                 175
   Phe Lys Pro Asn Leu Ala Ile Ile Ser Leu Glu Asn Gln Trp Ser Ala
               180                 185                 190
   Leu Ser Lys Gln Ile Phe Leu Ala Gln Asn Gln Gly Gly Lys Phe Arg
           195                 200                 205
   Asn Pro Val Asp Leu Ile Lys Pro Thr Gly Glu Arg Phe Gln Val Thr
       210                 215                 220
   Asn Val Asp Ser Asp Val Val Lys Gly Asn Ile Lys Leu Leu Leu Asn
   225                 230                 235                 240
   Ser Arg Ala Ser Thr Ala Asp Glu Asn Phe Ile Thr Thr Met Thr Leu
                   245                 250                 255
   Leu Gly Glu Ser Val Val Asn
               260
(2) INFORMATION FOR SEQ ID NO:5:
   (i) SEQUENCE CHARACTERISTICS:
      (A) LENGTH: 248 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Asp Val Arg Phe Ser Leu Ser Gly Ser Ser Ser Thr Ser Tyr Ser Lys
   1               5                   10                  15
   Phe Ile Gly Asp Leu Arg Lys Ala Leu Pro Ser Asn Gly Thr Val Tyr
               20                  25                  30
   Asn Leu Thr Ile Leu Leu Ser Ser Ala Ser Gly Ala Ser Arg Tyr Thr
           35                  40                  45
   Leu Met Thr Leu Ser Asn Tyr Asp Gly Lys Ala Ile Thr Val Ala Val
       50                  55                  60
   Asp Val Ser Gln Leu Tyr Ile Met Gly Tyr Leu Val Asn Ser Thr Ser
   65                  70                  75                  80
   Tyr Phe Phe Asn Glu Ser Asp Ala Lys Leu Ala Ser Gln Tyr Val Phe
                   85                  90                  95
   Lys Gly Ser Thr Ile Val Thr Leu Pro Tyr Ser Gly Asn Tyr Glu Lys
               100                 105                 110
   Leu Gln Thr Ala Ala Gly Lys Ile Arg Glu Lys Ile Pro Leu Gly Phe
           115                 120                 125
   Pro Ala Leu Asp Ser Ala Leu Thr Thr Ile Phe His Tyr Asp Ser Thr
       130                 135                 140
   Ala Ala Ala Ala Ala Phe Leu Val Ile Leu Gln Thr Thr Ala Glu Ala
   145                 150                 155                 160
   Ser Arg Phe Lys Tyr Ile Glu Gly Gln Ile Ile Glu Arg Ile Ser Lys
                   165                 170                 175
   Asn Gln Val Pro Ser Leu Ala Thr Ile Ser Leu Glu Asn Ser Leu Trp
               180                 185                 190
   Ser Ala Leu Ser Lys Gln Ile Gln Leu Ala Gln Thr Asn Asn Gly Thr
           195                 200                 205
   Phe Lys Thr Pro Val Val Ile Thr Asp Asp Lys Gly Gln Arg Val Glu
       210                 215                 220
   Ile Thr Asn Val Thr Ser Lys Val Val Thr Lys Asn Ile Gln Leu Leu
   225                 230                 235                 240
   Leu Asn Tyr Lys Gln Asn Val Ala
                   245
(2) INFORMATION FOR SEQ ID NO:6:
   (i) SEQUENCE CHARACTERISTICS:
      (A) LENGTH: 255 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Asp Val Ser Phe Arg Leu Ser Gly Ala Thr Ser Ser Ser Tyr Gly Val
   1               5                   10                  15
   Phe Ile Ser Asn Leu Arg Lys Ala Leu Pro Asn Glu Arg Lys Leu Tyr
               20                  25                  30
   Asp Leu Pro Leu Ile Arg Ser Ser Leu Pro Gly Ser Gln Arg Tyr Ala
           35                  40                  45
   Ile Ile His Leu Thr Asn Tyr Ala Asp Glu Val Ala Leu Asp Val Thr
       50                  55                  60
   Asn Val Asp Ala Gly Leu Pro Arg Asn Ala Val Leu Tyr Ile Met Gly
   65                  70                  75                  80
   Tyr Arg Ala Gly Asp Thr Ser Tyr Phe Phe Asn Glu Ala Ser Ala Thr
                   85                  90                  95
   Glu Ala Ala Lys Tyr Val Phe Lys Asp Ala Met Arg Lys Val Thr Leu
               100                 105                 110
   Pro Tyr Ser Gly Asn Tyr Glu Arg Leu Gln Thr Ala Ala Gly Gly Leu
           115                 120                 125
   Arg Glu Asn Ile Pro Leu Gly Leu Pro Ala Leu Asp Ser Ala Ile Thr
       130                 135                 140
   Thr Leu Phe Tyr Tyr Asn Ala Asn Ser Ala Ala Ser Ala Leu Met Val
   145                 150                 155                 160
   Leu Ile Gln Ser Thr Ser Glu Ala Ala Arg Tyr Lys Phe Ile Glu Gln
                   165                 170                 175
   Gln Ile Gly Ser Arg Val Asp Lys Thr Phe Leu Pro Ser Leu Ala Ile
               180                 185                 190
   Ile Ser Leu Glu Asn Ser Leu Trp Leu Ala Leu Ser Lys Gln Ile Gln
           195                 200                 205
   Ile Ala Ser Thr Asn Asn Gly Glu Phe Glu Thr Pro Val Val Leu Ile
       210                 215                 220
   Asn Ala Gln Asn Gln Arg Val Thr Ile Thr Asn Val Asp Ala Gly Val
   225                 230                 235                 240
   Val Thr Ser Asn Ile Ala Leu Leu Leu Asn Arg Asn Asn Met Ala
                   245                 250                 255
(2) INFORMATION FOR SEQ ID NO:7:
   (i) SEQUENCE CHARACTERISTICS:
      (A) LENGTH: 263 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Asp Val Ser Phe Arg Leu Ser Gly Ala Asp Pro Arg Ser Tyr Gly Met
   1               5                   10                  15
   Phe Ile Lys Asp Leu Arg Asn Ala Leu Pro Phe Arg Glu Lys Val Tyr
               20                  25                  30
   Asn Ile Pro Leu Leu Leu Pro Ser Val Ser Gly Ala Gly Arg Tyr Leu
           35                  40                  45
   Leu Met His Leu Phe Asn Tyr Asp Gly Lys Thr Ile Thr Val Ala Val
       50                  55                  60
   Asp Val Thr Asn Val Tyr Ile Met Gly Tyr Leu Ala Asp Thr Thr Ser
   65                  70                  75                  80
   Tyr Phe Phe Asn Glu Pro Ala Ala Glu Leu Ala Ser Gln Tyr Val Phe
                   85                  90                  95
   Arg Asp Ala Arg Arg Lys Ile Thr Leu Pro Tyr Ser Gly Asn Tyr Glu
               100                 105                 110
   Arg Leu Gln Ile Ala Ala Gly Lys Pro Arg Glu Lys Ile Pro Ile Gly
           115                 120                 125
   Leu Pro Ala Leu Asp Ser Ala Ile Ser Thr Leu Leu His Tyr Asp Ser
       130                 135                 140
   Thr Ala Ala Ala Gly Ala Leu Leu Val Leu Ile Gln Thr Thr Ala Glu
   145                 150                 155                 160
   Ala Ala Arg Phe Lys Tyr Ile Glu Gln Gln Ile Gln Glu Arg Ala Tyr
                   165                 170                 175
   Arg Asp Glu Val Pro Ser Leu Ala Thr Ile Ser Leu Glu Asn Ser Trp
               180                 185                 190
   Ser Gly Leu Ser Lys Gln Ile Gln Leu Ala Gln Gly Asn Asn Gly Ile
           195                 200                 205
   Phe Arg Thr Pro Ile Val Leu Val Asp Asn Lys Gly Asn Arg Val Gln
       210                 215                 220
   Ile Thr Asn Val Thr Ser Lys Val Val Thr Ser Asn Ile Gln Leu Leu
   225                 230                 235                 240
   Leu Asn Thr Arg Asn Ile Ala Glu Gly Asp Asn Gly Asp Val Ser Thr
                   245                 250                 255
   Thr His Gly Phe Ser Ser Thr
               260
(2) INFORMATION FOR SEQ ID NO:8:
   (i) SEQUENCE CHARACTERISTICS:
      (A) LENGTH: 250 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Ala Pro Thr Leu Glu Thr Ile Ala Ser Leu Asp Leu Asn Asn Pro Thr
   1               5                   10                  15
   Thr Tyr Leu Ser Phe Ile Thr Asn Ile Arg Thr Lys Val Ala Asp Lys
               20                  25                  30
   Thr Glu Gln Cys Thr Ile Gln Lys Ile Ser Lys Thr Phe Thr Gln Arg
           35                  40                  45
   Tyr Ser Tyr Ile Asp Leu Ile Val Ser Ser Thr Gln Lys Ile Thr Leu
       50                  55                  60
   Ala Ile Asp Met Ala Asp Leu Tyr Val Leu Gly Tyr Ser Asp Ile Ala
   65                  70                  75                  80
   Asn Asn Lys Gly Arg Ala Phe Phe Phe Lys Asp Val Thr Glu Ala Val
                   85                  90                  95
   Ala Asn Asn Phe Phe Pro Gly Ala Thr Gly Thr Asn Arg Ile Lys Leu
               100                 105                 110
   Thr Phe Thr Gly Ser Tyr Gly Asp Leu Glu Lys Asn Gly Gly Leu Arg
           115                 120                 125
   Lys Asp Asn Pro Leu Gly Ile Phe Arg Leu Glu Asn Ser Ile Val Asn
       130                 135                 140
   Ile Tyr Gly Lys Ala Gly Asp Val Lys Lys Gln Ala Lys Phe Phe Leu
   145                 150                 155                 160
   Leu Ala Ile Gln Met Val Ser Glu Ala Ala Arg Phe Lys Tyr Ile Ser
                   165                 170                 175
   Asp Lys Ile Pro Ser Glu Lys Tyr Glu Glu Val Thr Val Asp Glu Tyr
               180                 185                 190
   Met Thr Ala Leu Glu Asn Asn Trp Ala Lys Leu Ser Thr Ala Val Tyr
           195                 200                 205
   Asn Ser Lys Pro Ser Thr Thr Thr Ala Thr Lys Cys Gln Leu Ala Thr
       210                 215                 220
   Ser Pro Val Thr Ile Ser Pro Trp Ile Phe Lys Thr Val Glu Glu Ile
   225                 230                 235                 240
   Lys Leu Val Met Gly Leu Leu Lys Ser Ser
                   245                 250
(2) INFORMATION FOR SEQ ID NO:9:
   (i) SEQUENCE CHARACTERISTICS:
      (A) LENGTH: 261 amino acids
      (B) TYPE: amino acid
      (D) TOPOLOGY: linear
   (ii) MOLECULE TYPE: protein
   (xi) SEQUENCE DESCRIPTION: SEQ ID NO:9:
   Ile Asn Thr Ile Thr Phe Asp Ala Gly Asn Ala Thr Ile Asn Lys Tyr
   1               5                   10                  15
   Ala Thr Phe Met Glu Ser Leu Arg Asn Glu Ala Lys Asp Pro Ser Leu
               20                  25                  30
   Lys Cys Tyr Gly Ile Pro Met Leu Pro Asn Thr Asn Ser Thr Ile Lys
           35                  40                  45
   Tyr Leu Leu Val Lys Leu Gln Gly Ala Ser Leu Lys Thr Ile Thr Leu
       50                  55                  60
   Met Leu Arg Arg Asn Asn Leu Tyr Val Met Gly Tyr Ser Asp Pro Tyr
   65                  70                  75                  80
   Asp Asn Lys Cys Arg Tyr His Ile Phe Asn Asp Ile Lys Gly Thr Glu
                   85                  90                  95
   Tyr Ser Asp Val Glu Asn Thr Leu Cys Pro Ser Ser Asn Pro Arg Val
               100                 105                 110
   Ala Lys Pro Ile Asn Tyr Asn Gly Leu Tyr Pro Thr Leu Glu Lys Lys
           115                 120                 125
   Ala Gly Val Thr Ser Arg Asn Glu Val Gln Leu Gly Ile Gln Ile Leu
       130                 135                 140
   Ser Ser Lys Ile Gly Lys Ile Ser Gly Gln Gly Ser Phe Thr Glu Lys
   145                 150                 155                 160
   Ile Glu Ala Asp Phe Leu Leu Val Ala Ile Gln Met Val Ser Glu Ala
                   165                 170                 175
   Ala Arg Phe Lys Tyr Ile Glu Asn Gln Val Lys Thr Asn Phe Asn Arg
               180                 185                 190
   Asp Phe Ser Pro Asn Asp Lys Val Leu Asp Leu Glu Glu Asn Trp Gly
           195                 200                 205
   Lys Ile Ser Thr Ala Ile His Asn Ser Lys Asn Gly Ala Leu Pro Lys
       210                 215                 220
   Pro Leu Glu Leu Lys Asn Ala Asp Gly Thr Lys Trp Ile Val Leu Arg
   225                 230                 235                 240
   Val Asp Glu Ile Lys Pro Asp Val Gly Leu Leu Asn Tyr Val Asn Gly
                   245                 250                 255
   Thr Cys Gln Ala Thr
               260
(2) INFORMATION FOR SEQ ID NO:10:
   (i) SEQUENCE CHARACTERISTICS:
      (A) LENGTH: 259 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Val Thr Ser Ile Thr Leu Asp Leu Val Asn Pro Thr Ala Gly Gln Tyr
   1               5                   10                  15
   Ser Ser Phe Val Asp Lys Ile Arg Asn Asn Val Lys Asp Pro Asn Leu
               20                  25                  30
   Lys Tyr Gly Gly Thr Asp Ile Ala Val Ile Gly Pro Pro Ser Lys Glu
           35                  40                  45
   Lys Phe Leu Arg Ile Asn Phe Gln Ser Ser Arg Gly Thr Val Ser Leu
       50                  55                  60
   Gly Leu Lys Arg Asp Asn Leu Tyr Val Val Ala Tyr Leu Ala Met Asp
   65                  70                  75                  80
   Asn Thr Asn Val Asn Arg Ala Tyr Tyr Phe Arg Ser Glu Ile Thr Ser
                   85                  90                  95
   Ala Glu Ser Thr Ala Leu Phe Pro Glu Ala Thr Thr Ala Asn Gln Lys
               100                 105                 110
   Ala Leu Glu Tyr Thr Glu Asp Tyr Gln Ser Ile Glu Lys Asn Ala Gln
           115                 120                 125
   Ile Thr Gln Gly Asp Gln Ser Arg Lys Glu Leu Gly Leu Gly Ile Asp
       130                 135                 140
   Leu Leu Ser Thr Ser Met Glu Ala Val Asn Lys Lys Ala Arg Val Val
   145                 150                 155                 160
   Lys Asp Glu Ala Arg Phe Leu Leu Ile Ala Ile Gln Met Thr Ala Glu
                   165                 170                 175
   Ala Ala Arg Phe Arg Tyr Ile Gln Asn Leu Val Ile Lys Asn Phe Pro
               180                 185                 190
   Asn Lys Phe Asn Ser Glu Asn Lys Val Ile Gln Phe Glu Val Asn Trp
           195                 200                 205
   Lys Lys Ile Ser Thr Ala Ile Tyr Gly Asp Ala Lys Asn Gly Val Phe
       210                 215                 220
   Asn Lys Asp Tyr Asp Phe Gly Phe Gly Lys Val Arg Gln Val Lys Asp
   225                 230                 235                 240
   Leu Gln Met Gly Leu Leu Met Tyr Leu Gly Lys Pro Lys Ser Ser Asn
                   245                 250                 255
   Glu Ala Asn
(2) INFORMATION FOR SEQ ID NO:11:
   (i) SEQUENCE CHARACTERISTICS:
      (A) LENGTH: 813 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:11:
   GGGCTAGATA CCGTGTCATT CTCAACCAAA GGTGCCACTT ATATTACCTA CGTGAATTTC      60
   TTGAATGAGC TACGAGTTAA ATTGAAACCC GAAGGTAACA GCCATGGAAT CCCATTGCTG     120
   CGCAAAAAAT GTGATGATCC TGGAAAGTGT TTCGTTTTGG TAGCGCTTTC AAATGACAAT     180
   GGACAGTTGG CGGAAATAGC TATAGATGTT ACAAGTGTTT ATGTGGTGGG CTATCAAGTA     240
   AGAAACAGAT CTTACTTCTT TAAAGATGCT CCAGATGCTG CTTACGAAGG CCTCTTCAAA     300
   AACACAATTA AAACAAGACT TCATTTTGGC GGCAGCTATC CCTCGCTGGA AGGTGAGAAG     360
   GCATATAGAG AGACAACAGA CTTGGGCATT GAACCATTAA GGATTGGCAT CAAGAAACTT     420
   GATGAAAATG CGATAGACAA TTATAAACCA ACGGAGATAG CTAGTTCTCT ATTGGTTGTT     480
   ATTCAAATGG TGTCTGAAGC AGCTCGATTC ACCTTTATTG AGAACCAAAT TAGAAATAAC     540
   TTTCAACAGA GAATTCGCCC GGCGAATAAT ACAATCAGCC TTGAGAATAA ATGGGGTAAA     600
   CTCTCGTTCC AGATCCGGAC ATCAGGTGCA AATGGAATGT TTTCGGAGGC AGTTGAATTG     660
   GAACGTGCAA ATGGCAAAAA ATACTATGTC ACCGCAGTTG ATCAAGTAAA ACCCAAAATA     720
   GCACTCTTGA AGTTCGTCGA TAAAGATCCT AAAACGAGCC TTGCTGCTGA ATTGATAATC     780
   CAGAACTATG AGTCATTAGT GGGCTTTGAT TAG                                  813
(2) INFORMATION FOR SEQ ID NO:12:
   (i) SEQUENCE CHARACTERISTICS:
      (A) LENGTH: 846 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:12:
   ATGGCGGCAA AGATGGCGAA GAACGTGGAC AAGCCGCTCT TCACCGCGAC GTTCAACGTC 60
   CAGGCCAGCT CCGCCGACTA CGCCACCTTC ATCGCCGGCA TCCGCAACAA GCTCCGCAAC 120
   CCGGCGCACT TCTCCCACAA CCGCCCCGTG CTGCCGCCGG TCGAGCCCAA CGTCCCGCCG 180
   AGCAGGTGGT TCCACGTCGT GCTCAAGGCC TCGCCGACCA GCGCCGGGCT CACGCTGGCC 240
   ATCCGCGCGG ACAACATCTA CCTGGAGGGC TTCAAGAGCA GCGACGGCAC CTGGTGGGAG 300
   CTCACCCCGG GCCTCATCCC CGGCGCCACC TACGTCGGGT TCGGCGGCAC CTACCGCGAC 360
   CTCCTCGGCG ACACCGACAA GCTAACCAAC GTCGCTCTCG GCCGACAGCA GCTGGCGGAC 420
   GCGGTGACCG CGCTCCACGG GCGCACCAAG GCCGACAAGG CCTCCGGCCC GAAGCAGCAG 480
   CAGGCGAGGG AGGCGGTGAC GACGCTGGTC CTCATGGTGA ACGAGGCCAC GCGGTTCCAG 540
   ACGGTGTCTG GGTTCGTGGC CGGGTTGCTG CACCCCAAGG CGGTGGAGAA GAAGAGCGGG 600
   AAGATCGGCA ATGAGATGAA GGCCCAGGTG AACGGGTGGC AGGACCTGTC CGCGGCGCTG 660
   CTGAAGACGG ACGTGAAGCC TCCGCCGGGA AAGTCGCCAG CGAAGTTCGC GCCGATCGAG 720
   AAGATGGGCG TGAGGACGGC TGAACAGGCC GCCAACACGC TGGGGATCCT GCTGTTCGTG 780
   GAGGTGCCGG GTGGGTTGAC GGTGGCCAAG GCGCTGGAGC TGTTCCATGC GAGTGGTGGG 840
   AAATAG 846
(2) INFORMATION FOR SEQ ID NO:13:
   (i) SEQUENCE CHARACTERISTICS:
      (A) LENGTH: 913 base pairs
      (B) TYPE: nucleic acid
      (C) STRANDEDNESS: single
      (D) TOPOLOGY: linear
   (ii) MOLECULE TYPE; cDNA
   (xi) SEQUENCE DESCRIPTION: SEQ ID NO:13:
   CGTCCGAAAA TGGTGAAATG CTTACTACTT TCTTTTTTAA TTATCGCCAT CTTCATTGGT 60
   GTTCCTACTG CCAAAGGCGA TGTTAACTTC GATTTGTCGA CTGCCACTGC AAAAACCTAC 120
   ACAAAATTTA TCGAAGATTT CAGGGCGACT CTTCCATTTA GCCATAAAGT GTATGATATA 180
   CCTCTACTGT ATTCCACTAT TTCCGACTCC AGACGTTTCA TACTCCTCGA TCTTACAAGT 240
   TATGCATATG AAACCATCTC GGTGGCCATA GATGTGACGA ACGTTTATGT TGTGGCGTAT 300
   CGCACCCGCG ATGTATCCTA CTTTTTTAAA GAATCTCCTC CTGAAGCTTA TAACATCCTA 360
   TTCAAAGGTA CGCGGAAAAT TACACTGCCA TATACCGGTA ATTATGAAAA TCTTCAAACT 420
   GCTGCACACA AAATAAGAGA GAATATTGAT CTTGGACTCC CTGCCTTGAG TAGTGCCATT 480
   ACCACATTGT TTTATTACAA TGCCCAATCT GCTCCTTCTG CATTGCTTGT ACTAATCCAG 540
   ACGACTGCAG AAGCTGCAAG ATTTAAGTAT ATCGAGCGAC ACGTTGCTAA GTATGTTGCC 600
   ACTAACTTTA AGCCAAATCT AGCCATCATA AGCTTGGAAA ATCAATGGTC TGCTCTCTCC 660
   AACAAATCTT TTTGGCGCAG AATCAAGGAG GAAAATTTAG AAATCCTGTC GACCTTATAA 720
   AACCTACCGG GGAACGGTTT CAAGTAACCA ATGTTGATTC AGATGTTGTA AAAGGTAATA 780
   TCAAACTCCT GCTGAACTCC AGAGCTAGCA CTGCTGATGA AAACTTTATC ACAACCATGA 840
   CTCTACTTGG GGAATCTGTT GTGAATTGAA AGTTTAATAA TCCACCCATA TCGAAATAAG 900
   GCATGTTCAT GAC 913
(2) INFORMATION FOR SEQ ID NO:14:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:14:
   TTYAARGAYG CNCCNGAYGC NGCNTAYGAR GG 32
(2) INFORMATION FOR SEQ ID NO:15:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:15:
   TTYTTYATRA TRCANTGNCG NCANCTRGTY CA 32
(2) INFORMATION FOR SEQ ID NO:16:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:16:
   GGNYTNGAYA CNGTNWSNTT YWSNACNAAR GG 32
(2) INFORMATION FOR SEQ ID NO:17:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:17:
   TGTCTGAACC CGTAACTTGG TAA 23
(2) INFORMATION FOR SEQ ID NO:18:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:18:
   CACTCTTCCG TATCTCTCTG T 21
(2) INFORMATION FOR SEQ ID NO:19:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:19:
   TCAACCCGGG CTAGATACCG TGTCATTCTC AACCAAAGGT GCCACTTATA TTA 53
(2) INFORMATION FOR SEQ ID NO:20:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:20:
   CTTCATTTTG GCGGCACGTA TCC 23
(2) INFORMATION FOR SEQ ID NO:21:
   (i) SEQUENCE CHARACTERISTICS:
      (A) LENGTH: 46 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:21:
   CTCGAGGCTG CAAGCTTACG TGGGATTTTT TTTTTTTTTT TTTTTT 46
(2) INFORMATION FOR SEQ ID NO:22:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:22:
   CTCGCTGGAA GGTGAGAA 18
(2) INFORMATION FOR SEQ ID NO:23:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:23:
   CTCGAGGCTG CAAGCTTACG TGGGA 25
(2) INFORMATION FOR SEQ ID NO:24:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:24:
   TGATCTCGAG TACTATTTAG GATCTTTATC GACGA 35
(2) INFORMATION FOR SEQ ID NO:25:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:25:
   GTAAGCAGCA TCTGGAGCAT CT 22
(2) INFORMATION FOP. SEQ ID NO:26:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:26:
(2) INFORMATION FOR SEQ ID NO:27:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:27:
   GGCCTGGACA CCGTGAGCTT TAG 23
(2) INFORMATION FOR SEQ ID NO:28:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:28:
   TCGATTGCGA TCCTAAATAG TACTC 25
(2) INFORMATION FOR SEQ ID NO:29:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA
   (xi) SEOUENCE DESCRIPTION: SEO ID NO:29:
(2) INFORMATION FOR SEQ ID NO:30:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:30:
   GTTCGTCTGT AAAGATCCTA AATAGTACTC GA 32
(2) INFORMATION FOR SEQ ID NO:31:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:31:
   GGATCTTTAC AGACGAACTT CAAGAGT 27
(2) INFORMATION FOR SEQ ID NO:32:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:32:
   TCTTGTGCTT CGTCGATAAA GATCC 25
(2) INFORMATION FOR SEQ ID NO:33:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:33:
   ATCGACGAAG CACAAGAGTG CTATTTT 27
(2) INFORMATION FOR SEQ ID NO:34:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:34:
   GTAAAACCAT GCATAGCACT CTTGAAGTTC GT 32
(2) INFORMATION FOR SEQ ID NO:35:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:35:
   AGTGCTATGC ATGGTTTTAC TTGATCAACT GC   32
(2) INFORMATION FOR SEQ ID NO:36:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:36:
   AGCACATGTG GTGCCACTTA TATTACCTA 29
(2) INFORMATION FOR SEQ ID NO:37:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:37:
   TAAGTGGCAC CACATGTGCT AAAGCTCACG GTG 33
(2) INFORMATION FOR SEQ ID NO:38:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:38:
   TGACTGTGGA CAGTTGGCGG AAATA 25
(2) INFORMATION FOR SEQ ID NO:39:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:39:
   GCCACTGTCC ACAGTCATTT GAAAGCGCTA CC 32
(2) INFORMATION FOR SEQ ID NO:40:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:40:
   GATGATCCTG GAAAGGCTTT CGTTTTGGTA GCGCTT 36
(2) INFORMATION FOR SEQ ID NO:41:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:41:
   AAGCCTTTCC AGGATCATCA GCTTTTTTGG GCAGCAATGG G 41
(2) INFORMATION FOR SEQ ID NO:42:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:42:
   AAGCCTTTCC AGGATCATCA CAT 23
(2) INFORMATION FOR SEQ ID NO:43:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:43:
   GCGACTCTCT ACTGTTTC 18
(2) INFORMATION FOR SEQ ID NO:44:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:44:
   CGTTAGCAAT TTAACTGTGA T 21
(2) INFORMATION FOR SEQ ID NO:45:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:45:
   AACAGCTATG ACCATG 16
(2) INFORMATION FOR SEQ ID NO:46:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:46:
   TGAACTCGAG GAAACTACCT ATTTCCCAC 29
(2) INFORMATION FOR SEQ ID NO:47:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:47:
   GCATTACATC CATGGCGGC 19
(2) INFORMATION FOR SEQ ID NO:48:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:48:
   GATATCTCGA GTTAACTATT TCCCACCACA CGCATGGAAC AGCTCCAGCG CCTTGGCCAC 60
   CGTC 64
(2) INFORMATION FOR SEQ ID NO:49:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:49:
   TGTCTGTTCG TGGAGGTGCC G 21
(2) INFORMATION FOR SEQ ID NO:50:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:50:
   CCAAGTGTCT GGAGCTGTTC CATGCGA 27
(2) INFORMATION FOR SEQ ID NO:51:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:51:
   GATGTTAAYT TYGAYTTGTC NACDGCTAC 29
(2) INFORMATION FOR SEQ ID NO:52:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:52:
   ATTGGNAGDG TAGCCCTRAA RTCYTCDAT 29
(2) INFORMATION FOR SEQ ID NO:53:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:53:
   GCCACTGCAA AAACCTACAC AAAATTTATT GA 32
(2) INFORMATION FOR SEQ ID NO:54:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:54:
   GATGTTAACT TCGATTTGTC GA 22
(2) INFORMATION FOR SEQ ID NO:55:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:55:
   TCAACTCGAG GTACTCAATT CACAACAGAT TCC 33
(2) INFORMATION FOR SEQ ID NO:56:
   (i) SEQUENCE CHARACTERISTICS:
      (A) LENGTH: 20 amino acids
      (B) TYPE: amino acid
      (D) TOPOLOGY: linear
   (ii) MOLECULE TYPE: peptide
   (xi) SEQUENCE DESCRIPTION: SEQ ID NO:56:
   Cys His His His Ala Ser Arg Val Ala Arg Met Ala Ser Asp Glu Phe
   1               5                   10                  15
   Pro Ser Met Cys
               20
(2) INFORMATION FOR SEQ ID NO:57:
   (i) SEQUENCE CHARACTERISTICS:
      (A) LENGTH: 20 amino acids
      (B) TYPE: amino acid
      (D) TOPOLOGY: linear
   (ii) MOLECULE TYPE: peptide
   (xi) SEQUENCE DESCRIPTION: SEQ ID NO:57:
   Pro Ser Gly Gln Ala Gly Ala Ala Ala Ser Glu Ser Leu Phe Ile Ser
   1               5                   10                  15
   Asn His Ala Tyr
               20
(2) INFORMATION FOR SEQ ID NO:58:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:58:
   CAGCCATGGA ATCCCATTGC TG                                        22
(2) INFORMATION FOR SEQ ID NO:59:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:59:
   CACATGTAAA ACAAGACTTC ATTTTGGC 28
(2) INFORMATION FOR SEQ ID NO:60:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:60:
   TGAAGTCTTG TTTTAGATGT GTTTTTGAAG AGGCCT 36
(2) INFORMATION FOR SEQ ID NO:61:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:61:
   ATGCCATATG CAATTATAAA CCAACGGAGA 30
(2) INFORMATION FOR SEQ ID NO:62:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:62:
   GGTTTATAAT TGCATATGGC ATTTTCATCA AGTTTCTTG 39
(2) INFORMATION FOR SEQ ID NO:63:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:63:
   CTTTCAACAA TGCATTCGCC CGGCGAATAA TAC 33
(2) INFORMATION FOR SEQ ID NO:64:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:64:
   GCGAATGCAT TGTTGAAAGT TATTTCTAAT TTG 33
(2) INFORMATION FOR SEQ ID NO:65:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:65:
   GTTTTGTGAG GCAGTTGAAT TGGAAC 26
(2) INFORMATION FOP SEQ ID NO:66:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:66:
(2) INFORMATION FOR SEQ ID NO:67:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:67:
   AAAAGCTGAT GATCCTGGAA AGTG 24
(2) INFORMATION FOR SEQ ID NO:68:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:68:
   TCCAGGATCA TCAGCTTTTT TGCGCAGCAA TGGGA 35
(2) INFORMATION FOR SEQ ID NO:69:
   (i) SEQUENCE CHARACTERISTICS:
      (A) LENGTH: 321 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:69:
   GACATCCAGA TGACTCAGTC TCCATCTTCC ATGTCTGCAT CTCTGGGAGA CAGAGTCACT 60
   ATCACTTGCC GGGCGAGTCA GGACATTAAT AGCTATTTAA GCTGGTTCCA GCAGAAACCA 120
   GGGAAATCTC CTAAGACCCT GATCTATCGT GCAAACAGAT TGGTAGATGG GGTCCCATCA 180
   AGGTTCAGTG GCAGTGGATC TGGGACAGAT TATACTCTCA CCATCAGCAG CCTGCAATAT 240
   GAAGATTTTG GAATTTATTA TTGTCAACAG TATGATGAGT CTCCGTGGAC GTTCGGTGGA 300
   GGCACCAAGC TTGAAATCAA A 321
(2) INFORMATION FOR SEQ ID NO:70:
   (i) SEQUENCE CHARACTERISTICS:
      (A) LENGTH: 354 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:70:
   CAGATCCAGT TGGTGCAGTC TGGACCTGGC CTGAAGAAGC CTGGAGGGTC CGTCAGAATC 60
   TCCTGCGCAG CTTCTGGGTA TACCTTCACA AACTATGGAA TGAACTGGGT GAAGCAGGCT 120
   CCAGGAAAGG GTTTAAGGTG GATGGGCTGG ATAAACACCC ACACTGGAGA GCCAACATAT 180
   GCTGATGACT TCAAGGGACG GTTTACCTTC TCTTTGGACA CGTCTAAGAG CACTGCCTAT 240
   TTACAGATCA ACAGCCTCAG AGCCGAGGAC ACGGCTACAT ATTTCTGTAC AAGACGGGGT 300
   TACGACTGGT ACTTCGATGT CTGGGGCCAA GGGACCACGG TCACCGTCTC CTCC 354
(2) INFORMATION FOR SEQ ID NO:71:
   (i) SEQUENCE CHARACTERISTICS:
      (A) LENGTH: 354 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:71:
   GAGATCCAGT TGGTGCAGTC TGGAGGAGGC CTGGTGAAGC CTGGAGGGTC CGTCAGAATC 60
   TCCTGCGCAG CTTCTGGGTA TACCTTCACA AACTATGGAA TGAACTGGGT GCGCCAGGCT 120
   CCAGGAAAGG GTTTAGAGTG GATGGGCTGG ATAAACACCC ACACTGGAGA GCCAACATAT 180
   GCTGATTCTT TCAAGGGACG GTTTACCTTC TCTTTGGACG ATTCTAAGAA CACTGCCTAT 240
   TTACAGATCA ACAGCCTCAG AGCCGAGGAC ACGGCTGTGT ATTTCTGTAC AAGACGGGGT 300
   TACGACTGGT ACTTCGATGT CTGGGGCCAA GGGACCACGG TCACCGTCTC CTCC 354
(2) INFORMATION FOR SEQ ID NO:72:
   (i) SEQUENCE CHARACTERISTICS:
      (A) LENGTH: 321 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:72:
   GACATCCAGA TGACTCAGTC TCCATCTTCC CTGTCTGCAT CTGTAGGAGA CAGAGTCACT 60
   ATCACTTGCC GGGCGAGTCA GGACATTAAT AGCTATTTAA GCTGGTTCCA GCAGAAACCA 120
   GGGAAAGCTC CTAAGACCCT GATCTATCGT GCAAACAGAT TGGAATCTGG GGTCCCATCA 180
   AGGTTCAGTG GCAGTGGATC TGGGACAGAT TATACTCTCA CCATCAGCAG CCTGCAATAT 240
   GAAGATTTTG GAATTTATTA TTGTCAACAG TATGATGAGT CTCCGTGGAC GTTCGGTGGA 300
   GGCACCAAGC TTGAAATCAA A 321
(2) INFORMATION FOR SEQ ID NO:73:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:73:
   TGTCATCATC ATGCATCGCG AGTTGCCAGA ATGGCATCTG ATGAGTTTCC TTCTATGTGC 60
   GCAAGTACTC 70
(2) INFORMATION FOR SEQ ID NO:74:
   (i) SEQUENCE CHARACTERISTICS:
      (A) LENGTH: 78 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:74:
   TCGAGAGTAC TTGCGCACAT AGAAGGAAAC TCATCAGATG CCATTCTGGC AACTCGCGAT 60
   GCATGATGAT GACATGCA 78
(2) INFORMATION FOR SEQ ID NO:75:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:75:
   TGTTCGGCCG CATGTCATCA TCATGCATCG 30
(2) INFORMATION FOR SEQ ID NO:76:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:76:
   AGTCATGCCC CGCGC 15
(2) INFORMATION FOR SEQ ID NO:77:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:77:
   TCCCGGCTGT CCTACAGT 18
(2) INFORMATION FOR SEQ ID NO:78:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:78:
   TCCAGCCTGT CCAGATGGTG TGTGAGTTTT GTCACAA 37
(2) INFORMATION FOR SEQ ID NO:79:
   (i) SEQUENCE CHARACTERISTICS:
      (A) LENGTH: 76 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:79:
   CTAACTCGAG AGTACTGTAT GCATGGTTCG AGATGAACAA AGATTCTGAG GCTGCAGCTC 60
   CAGCCTGTCC AGATGG 76
(2) INFORMATION FOR SEQ ID NO:80:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:80:
   CTAACTCGAG AGTACTGTAT 20
(2) INFORMATION FOR SEQ ID NO:81:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:81:
   TCCAGCCTGT CCAGATGGAC ACTCTCCCCT GTTGAA 36
(2) INFORMATION FOR SEQ ID NO:82:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:82:
   GTACAGTGGA AGGTGGAT 18
(2) INFORMATION FOR SEQ ID NO:83:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:83:
   CATGCGGCCG ATTTAGGATC TTTATCGACG A 31
(2) INFORMATION FOR SEQ ID NO:84:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:84:
   AACATCCAGT TGGTGCAGTC TG 22
(2) INFORMATION FOR SEQ ID NO:85:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:85:
   GAGGAGACGG TGACCGTGGT 20
(2) INFORMATION FOR SEQ ID NO:86:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:86:
   GACATCAAGA TGACCCAGT 19
(2) INFORMATION FOR SEQ ID NO:87:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:87:
   GTTTGATTTC AAGCTTGGTG C 21
(2) INFORMATION FOR SEQ ID NO:88:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:88:
   ACTTCGGCCG CACCATCTGG ACAGGCTGGA G 31
(2) INFORMATION FOR SEQ ID NO:89:
   (i) SEQUENCE CHARACTERISTICS:
      (A) LENGTH: 723 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:89:
   GACATCCAGA TGACTCAGTC TCCATCTTCC CTGTCTGCAT CTGTAGGAGA CAGAGTCACT 60
   ATCACTTGCC GGGCGAGTCA GGACATTAAT AGCTATTTAA GCTGGTTCCA GCAGAAACCA 120
   GGGAAAGCTC CTAAGACCCT GATCTATCGT GCAAACAGAT TGGAATCTGG GGTCCCATCA 180
   AGGTTCAGTG GCAGTGGATC TGGGACAGAT TATACTCTCA CCATCAGCAG CCTGCAATAT 240
   GAAGATTTTG GAATTTATTA TTGTCAACAG TATGATGAGT CTCCGTGGAC GTTCGGTGGA 300
   GGCACCAAGC TTGAGATGAA AGGTGGCGGT GGATCTGGTG GAGGTGGGTC CGGAGGTGGA 360
   GGATCTGAGA TCCAGTTGGT GCAGTCTGGA GGAGGCCTGG TGAAGCCTGG AGGGTCCGTC 420
   AGAATCTCCT GCGCAGCTTC TGGGTATACC TTCACAAACT ATGGAATGAA CTGGGTGCGC 480
   CAGGCTCCAG GAAAGGGTTT AGAGTGGATG GGCTGGATAA ACACCCACAC TGGAGAGCCA 540
   ACATATGCTG ATTCTTTCAA GGGACGGTTT ACCTTCTCTT TGGACGATTC TAAGAACACT 600
   GCCTATTTAC AGATCAACAG CCTCAGAGCC GAGGACACGG CTGTGTATTT CTGTACAAGA 660
   CGGGGTTACG ACTGGTACTT CGATGTCTGG GGCCAAGGGA CCACGGTCAC CGTCTCCTCA 720
   TGA 723
(2) INFORMATION FOR SEQ ID NO:90:
   (i) SEQUENCE CHARACTERISTICS:
      (A) LENGTH: 723 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:90:
   GAGATCCAGT TGGTGCAGTC TGGAGGAGGC CTGGTGAAGC CTGGAGGGTC CGTCAGAATC    60
   TCCTGCGCAG CTTCTGGGTA TACCTTCACA AACTATGGAA TGAACTGGGT GCGCCAGGCT   120
   CCAGGAAAGG GTTTAGAGTG GATGGGCTGG ATAAACACCC ACACTGGAGA GCCAACATAT   180
   GCTGATTCTT TCAAGGGACG GTTTACCTTC TCTTTGGACG ATTCTAAGAA CACTGCCTAT   240
   TTACAGATCA ACAGCCTCAG AGCCGAGGAC ACGGCTGTGT ATTTCTGTAC AAGACGGGGT   300
   TACGACTGGT ACTTCGATGT CTGGGGCCAA GGGACCACGG TCACCGTCTC CTCAGGTGGC   360
   GGTGGATCTG GTGGAGGTGG GTCCGGAGGT GGAGGATCTG ACATCCAGAT GACTCAGTCT   420
   CCATCTTCCC TGTCTGCATC TGTAGGAGAC AGAGTCACTA TCACTTGCCG GGCGAGTCAG   480
   GACATTAATA GCTATTTAAG CTGGTTCCAG CAGAAACCAG GGAAAGCTCC TAAGACCCTG   540
   ATCTATCGTG CAAACAGATT GGAATCTGGG GTCCCATCAA GGTTCAGTGG CAGTGGATCT   600
   GGGACAGATT ATACTCTCAC CATCAGCAGC CTGCAATATG AAGATTTTGG AATTTATTAT   660
   TGTCAACAGT ATGATGAGTC TCCGTGGACG TTCGGTGGAG GCACCAAGCT TGAGATGAAA   720
   TGA                                                                 723
(2) INFORMATION FOR SEQ ID NO:91:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:91:
   CGGACCCACC TCCACCAGAT CCACCGCCAC CTTTCATCTC AAGCTTGGTG C    51
(2) INFORMATION FOR SEQ ID NO:92:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:92:
   GACATCCAGA TGACTCAGT    19
(2) INFORMATION FOR SEQ ID NO:93:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:93:
   GGTGGAGGTG GGTCCGGAGG TGGAGGATCT GAGATCCAGT TGGTGCAGT 49
(2) INFORMATION FOR SEQ ID NO:94:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:94:
   TGTACTCGAG CCCATCATGA GGAGACGGTG ACCGT 35
(2) INFORMATION FOR SEQ ID NO:95:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:95:
(2) INFORMATION FOR SEQ ID NO:96:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:96:
   TGTACTCGAG CCCATCATTT CATCTCAAGC TTGGTGC 37
(2) INFORMATION FOR SEQ ID NO:97:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:97:
   GAGATCCAGT TGGTGCAGTC TG 22
(2) INFORMATION FOR SEQ ID NO:98:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:98:
   CGGACCCACC TCCACCAGAT CCACCGCCAC CTGAGGAGAC GGTGACCGT 49
(2) INFORMATION FOR SEQ ID NO:99:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:99:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Cys Asp Asp Pro Gly
           35                  40                  45
   Lys Ala Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Asp Lys Asp Pro Lys
                   245                 250
(2) INFORMATION FOR SEQ ID NO:100:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:100:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Ala Asp Asp Pro Gly
           35                  40                  45
   Lys Cys Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Asp Lys Asp Pro Lys
                   245                 250
(2) INFORMATION FOR SEQ ID NO:101:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:101:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Ala Asp Asp Pro Gly
           35                  40                  45
   Lys Ala Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Asp Lys Asp Pro Lys
                   245                 250
(2) INFORMATION FOR SEQ ID NO:102:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:102:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Cys Asp Asp Pro Gly
           35                  40                  45
   Lys Cys Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Cys Lys Asp Pro Lys
                   245                 250
(2) INFORMATION FOR SEQ ID NO:103:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:103:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Cys Asp Asp Pro Gly
           35                  40                  45
   Lys Cys Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Asp Cys Asp Pro Lys
                   245                 250
(2) INFORMATION FOR SEQ ID NO:104:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:104:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Cys Asp Asp Pro Gly
           35                  40                  45
   Lys Cys Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Cys Ile
   225                 230                 235                 240
   Ala Leu Leu Lys Phe Val Asp Lys Asp Pro Lys
                   245                 250
(2) INFORMATION FOR SEQ ID NO:105:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:105:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Cys Asp Asp Pro Gly
           35                  40                  45
   Lys Cys Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Cys Phe Val Asp Lys Asp Pro Lys
                   245                 250
(2) INFORMATION FOR SEQ ID NO:106:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:106
   Gly Leu Asp Thr Val Ser Phe Ser Thr Cys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Cys Asp Asp Pro Gly
           35                  40                  45
   Lys Cys Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Asp Lys Asp Pro Lys
                   245                 250
2) INFORMATION FOR SEQ ID NO:107:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:107:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Cys Asp Asp Pro Gly
           35                  40                  45
   Lys Cys Phe Val Leu Val Ala Leu Ser Asn Asp Cys Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Asp Lys Asp Pro Lys
                   245                 250
(2) INFORMATION FOR SEQ ID NO:108:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:108:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Cys Asp Asp Pro Gly
           35                  40                  45
   Lys Cys Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Cys Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Asp Lys Asp Pro Lys
                   245                 250
(2) INFORMATION FOR SEQ ID NO:109:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:109:
   Gly Leu Asp Thr Val Ser Phe Ser Thr Lys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Cys Asp Asp Pro Gly
           35                  40                  45
   Lys Cys Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Cys Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Asp Lys Asp Pro Lys
                   245                 250
(2) INFORMATION FOR SEQ ID NO:110:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:110:
   Gly Leu Asp Thr Val Ser Phe Ser Thr Cys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Ala Asp Asp Pro Gly
           35                  40                  45
   Lys Ala Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Asp Lys Asp Pro Lys
                   245                 250
(2) INFORMATION FOR SEQ ID NO:111:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:111:
   Gly Leu Asp Thr Val Ser Phe Ser Thr Cys Gly Ala Thr Tyr Ile Thr
   1               5                   10                  15
   Tyr Val Asn Phe Leu Asn Glu Leu Arg Val Lys Leu Lys Pro Glu Gly
               20                  25                  30
   Asn Ser His Gly Ile Pro Leu Leu Arg Lys Lys Ala Asp Asp Pro Gly
           35                  40                  45
   Lys Ala Phe Val Leu Val Ala Leu Ser Asn Asp Asn Gly Gln Leu Ala
       50                  55                  60
   Glu Ile Ala Ile Asp Val Thr Ser Val Tyr Val Val Gly Tyr Gln Val
   65                  70                  75                  80
   Arg Asn Arg Ser Tyr Phe Phe Lys Asp Ala Pro Asp Ala Ala Tyr Glu
                   85                  90                  95
   Gly Leu Phe Lys Asn Thr Ile Lys Thr Arg Leu His Phe Gly Gly Ser
               100                 105                 110
   Tyr Pro Ser Leu Glu Gly Glu Lys Ala Tyr Arg Glu Thr Thr Asp Leu
           115                 120                 125
   Gly Ile Glu Pro Leu Arg Ile Gly Ile Lys Lys Leu Asp Glu Asn Ala
       130                 135                 140
   Ile Asp Asn Tyr Lys Pro Thr Glu Ile Ala Ser Ser Leu Leu Val Val
   145                 150                 155                 160
   Ile Gln Met Val Ser Glu Ala Ala Arg Phe Thr Phe Ile Glu Asn Gln
                   165                 170                 175
   Ile Arg Asn Asn Phe Gln Gln Arg Ile Arg Pro Ala Asn Asn Thr Ile
               180                 185                 190
   Ser Leu Glu Asn Lys Trp Gly Lys Leu Ser Phe Gln Ile Arg Thr Ser
           195                 200                 205
   Gly Ala Asn Gly Met Phe Ser Glu Ala Val Glu Leu Glu Arg Ala Asn
       210                 215                 220
   Gly Lys Lys Tyr Tyr Val Thr Ala Val Asp Gln Val Lys Pro Lys Ile
   225                 230                 235                 240
   Ala Leu Leu Lys Phe Val Cys Lys Asp Pro Lys
                   245                 250
(2) INFORMATION FOR SEQ ID NO:112:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:112:
   TGATGCGGCC GACATCTCAA GCTTGGTGC 29
(2) INFORMATION FOR SEQ ID NO:113:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:113:
   TGATGCGGCC GACATCTCAA GCTTGGTGC 29
(2) INFORMATION FOR SEQ ID NO:114:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:114:
   TCTAGGTCAC CGTCTCCTCA CCATCTGGAC AGGCTGGA 38
(2) INFORMATION FOR SEQ ID NO:115:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:115:
   TTCGAAGCTT GAGATGAAAC CATCTGGACA GGCTGGA 37
(2) INFORMATION FOR SEQ ID NO:116:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:116:
   GAGATCCAGT TGGTGCAGTC TG 22
(2) INFORMATION FOR SEQ ID NO:117:
   (i) SEQUENCE CHARACTERISTICS:
      (A) LENGTH: 98 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:117:
   AGTCGTCGAC ACGATGGACA TGAGGACCCC TGCTCAGTTT CTTGGCATCC TCCTACTCTG 60
   GTTTCCAGGT ATCAAATGTG ACATCCAGAT GACTCAGT 98
(2) INFORMATION FOR SEQ ID NO:118:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:118:
(2) INFORMATION FOR SEQ ID NO:119:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:119:
   TGACTCGCCC GGCAAGTGAT AGTGACTCTG TCTCCTACAG ATGCAGACAG GGAAGATGGA 60
   GACTGAGTCA TCTGGATGTC 80
(2) INFORMATION FOR SEQ ID NO:120:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:120:
   GATCCACTGC CACTGAACCT TGATGGGACC CCAGATTCCA ATCTGTTTGC ACGATAGATC 60
   AGGGTCTTAG GAGCTTTCC 79
(2) INFORMATION FOR SEQ ID NO:121:
   (i) SEQUENCE CHARACTERISTICS:
      (A) LENGTH: 82 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:121:
   GGTTCAGTGG CAGTGGATCT GGGACAGATT ATACTCTCAC CATCAGCAGC CTGCAATATG 60
   AAGATTTTGG AATTTATTAT TG 82
(2) INFORMATION FOR SEQ ID NO:122:
   (i) SEQUENCE CHARACTERISTICS:
      (A) LENGTH: 82 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:122:
   GTTTGATTTC AAGCTTGGTG CCTCCACCGA ACGTCCACGG AGACTCATCA TACTGTTGAC        60
   AATAATAAAT TCCAAAATCT TC
(2) INFORMATION FOR SEQ ID NO:123:
   (i) SEQUENCE CHARACTERISTICS:
      (A) LENGTH: 107 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:123:
   Asp Ile Lys Met Thr Gln Ser Pro Ser Ser Met Tyr Ala Ser Leu Gly
   1               5                   10                  15
   Glu Arg Val Thr Ile Thr Cys Lys Ala Ser Gln Asp Ile Asn Ser Tyr
               20                  25                  30
   Leu Ser Trp Phe His His Lys Pro Gly Lys Ser Pro Lys Thr Leu Ile
           35                  40                  45
   Tyr Arg Ala Asn Arg Leu Val Asp Gly Val Pro Ser Arg Phe Ser Gly
       50                  55                  60
   Ser Gly Ser Gly Gln Asp Tyr Ser Leu Thr Ile Ser Ser Leu Asp Tyr
   65                  70                  75                  80
   Glu Asp Met Gly Ile Tyr Tyr Cys Gln Gln Tyr Asp Glu Ser Pro Trp
                   85                  90                  95
   Thr Phe Gly Gly Gly Thr Lys Leu Glu Ile Lys
               100                 105
(2) INFORMATION FOR SEQ ID NO:124:
   (i) SEQUENCE CHARACTERISTICS:
      (A) LENGTH: 118 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:124:
   Gln Ile Gln Leu Val Gln Ser Gly Pro Glu Leu Lys Lys Pro Gly Glu
   1 5 10 15
   Thr Val Lys Ile Ser Cys Lys Ala Ser Gly Tyr Thr Phe Thr Asn Tyr
   20 25 30
   Gly Met Asn Trp Val Lys Gln Ala Pro Gly Lys Gly Leu Arg Trp Met
   35 40 45
   Gly Trp Ile Asn Thr His Thr Gly Glu Pro Thr Tyr Ala Asp Asp Phe
   50 55 60
   Lys Gly Arg Phe Ala Phe Ser Leu Glu Thr Ser Ala Ser Thr Ala Tyr
   65 70 75 80
   Leu Gln Ile Asn Asn Leu Lys Asn Glu Asp Thr Ala Thr Tyr Phe Cys
   85 90 95
   Thr Arg Arg Gly Tyr Asp Trp Tyr Phe Asp Val Trp Gly Ala Gly Thr
   100 105 110
   Thr Val Thr Val Ser Ser
   115
(2) INFORMATION FOR SEQ ID NO:125:
   (i) SEQUENCE CHARACTERISTICS:
      (A) LENGTH: 107 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:125:
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Asn Ser Tyr
   20 25 30
   Leu Ser Trp Phe Gln Gln Lys Pro Gly Lys Ala Pro Lys Thr Leu Ile
   35 40 45
   Tyr Arg Ala Asn Arg Leu Glu Ser Gly Val Pro Ser Arg Phe Ser Gly
   50 55 60
   Ser Gly Ser Gly Thr Asp Tyr Thr Leu Thr Ile Ser Ser Leu Gln Tyr
   65 70 75 80
   Glu Asp Phe Gly Ile Tyr Tyr Cys Gln Gln Tyr Asp Glu Ser Pro Trp
   85 90 95
   Thr Phe Gly Gly Gly Thr Lys Leu Glu Ile Lys
   100 105
(2) INFORMATION FOR SEQ ID NO:126:
   (i) SEQUENCE CHARACTERISTICS:
      (A) LENGTH: 118 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:126:
   Glu Ile Gln Leu Val Gln Ser Gly Gly Gly Leu Val Lys Pro Gly Gly
   1               5                   10                  15
   Ser Val Arg Ile Ser Cys Ala Ala Ser Gly Tyr Thr Phe Thr Asn Tyr
               20                  25                  30
   Gly Met Asn Trp Val Arg Gln Ala Pro Gly Lys Gly Leu Glu Trp Met
           35                  40                  45
   Gly Trp Ile Asn Thr His Thr Gly Glu Pro Thr Tyr Ala Asp Ser Phe
       50                  55                  60
   Lys Gly Arg Phe Thr Phe Ser Leu Asp Asp Ser Lys Asn Thr Ala Tyr
   65                  70                  75                  80
   Leu Gln Ile Asn Ser Leu Arg Ala Glu Asp Thr Ala Val Tyr Phe Cys
                   85                  90                  95
   Thr Arg Arg Gly Tyr Asp Trp Tyr Phe Asp Val Trp Gly Gln Gly Thr
               100                 105                 110
   Thr Val Thr Val Ser Ser
           115
(2) INFORMATION FOR SEQ ID NO:127:
   (i) SEQUENCE CHARACTERISTICS:
      (A) LENGTH: 280 amino acids
      (B) TYPE: amino acid
      (D) TOPOLOGY: linear
   (ii) MOLECULE TYPE: protein
   (xi) SEQUENCE DESCRIPTION: SEQ ID NO:127:
   Ala Ala Lys Met Ala Lys Asn Val Asp Lys Pro Leu Phe Thr Ala Thr
   1               5                   10                  15
   Phe Asn Val Gln Ala Ser Ser Ala Asp Tyr Ala Thr Phe Ile Ala Gly
               20                  25                  30
   Ile Arg Asn Lys Leu Arg Asn Pro Ala His Phe Ser His Asn Arg Pro
           35                  40                  45
   Val Leu Pro Pro Val Glu Pro Asn Val Pro Pro Ser Arg Trp Phe His
       50                  55                  60
   Val Val Leu Lys Ala Ser Pro Thr Ser Ala Gly Leu Thr Leu Ala Ile
   65                  70                  75                  80
   Arg Ala Asp Asn Ile Tyr Leu Glu Gly Phe Lys Ser Ser Asp Gly Thr
                   85                  90                  95
   Trp Trp Glu Leu Thr Pro Gly Leu Ile Pro Gly Ala Thr Tyr Val Gly
               100                 105                 110
   Phe Gly Gly Thr Tyr Arg Asp Leu Leu Gly Asp Thr Asp Lys Leu Thr
           115                 120                 125
   Asn Val Ala Leu Gly Arg Gln Gln Leu Ala Asp Ala Val Thr Ala Leu
       130                 135                 140
   His Gly Arg Thr Lys Ala Asp Lys Ala Ser Gly Pro Lys Gln Gln Gln
   145                 150                 155                 160
   Ala Arg Glu Ala Val Thr Thr Leu Val Leu Met Val Asn Glu Ala Thr
                   165                 170                 175
   Arg Phe Gln Thr Val Ser Gly Phe Val Ala Gly Leu Leu His Pro Lys
               180                 185                 190
   Ala Val Glu Lys Lys Ser Gly Lys Ile Gly Asn Glu Met Lys Ala Gln
           195                 200                 205
   Val Asn Gly Trp Gln Asp Leu Ser Ala Ala Leu Leu Lys Thr Asp Val
       210                 215                 220
   Lys Pro Pro Pro Gly Lys Ser Pro Ala Lys Phe Ala Pro Ile Glu Lys
   225                 230                 235                 240
   Met Gly Val Arg Thr Ala Glu Gln Ala Ala Asn Thr Leu Gly Ile Leu
                   245                 250                 255
   Leu Phe Val Glu Val Pro Gly Gly Leu Thr Val Ala Lys Ala Leu Glu
               260                 265                 270
   Leu Phe His Ala Cys Gly Gly Lys
           275                 280
(2) INFORMATION FOR SEQ ID NO:128:
   (i) SEQUENCE CHARACTERISTICS:
      (A) LENGTH: 280 amino acids
      (B) TYPE: amino acid
      (D) TOPOLOGY: linear
   (ii) MOLECULE TYPE: protein
   (xi) SEQUENCE DESCRIPTION: SEQ ID NO:128:
   Ala Ala Lys Met Ala Lys Asn Val Asp Lys Pro Leu Phe Thr Ala Thr
   1               5                   10                  15
   Phe Asn Val Gln Ala Ser Ser Ala Asp Tyr Ala Thr Phe Ile Ala Gly
               20                  25                  30
   Ile Arg Asn Lys Leu Arg Asn Pro Ala His Phe Ser His Asn Arg Pro
           35                  40                  45
   Val Leu Pro Pro Val Glu Pro Asn Val Pro Pro Ser Arg Trp Phe His
       50                  55                  60
   Val Val Leu Lys Ala Ser Pro Thr Ser Ala Gly Leu Thr Leu Ala Ile
   65                  70                  75                  80
   Arg Ala Asp Asn Ile Tyr Leu Glu Gly Phe Lys Ser Ser Asp Gly Thr
                   85                  90                  95
   Trp Trp Glu Leu Thr Pro Gly Leu Ile Pro Gly Ala Thr Tyr Val Gly
               100                 105                 110
   Phe Gly Gly Thr Tyr Arg Asp Leu Leu Gly Asp Thr Asp Lys Leu Thr
           115                 120                 125
   Asn Val Ala Leu Gly Arg Gln Gln Leu Ala Asp Ala Val Thr Ala Leu
       130                 135                 140
   His Gly Arg Thr Lys Ala Asp Lys Ala Ser Gly Pro Lys Gln Gln Gln
   145                 150                 155                 160
   Ala Arg Glu Ala Val Thr Thr Leu Val Leu Met Val Asn Glu Ala Thr
                   165                 170                 175
   Arg Phe Gln Thr Val Ser Gly Phe Val Ala Gly Leu Leu His Pro Lys
               180                 185                 190
   Ala Val Glu Lys Lys Ser Gly Lys Ile Gly Asn Glu Met Lys Ala Gln
           195                 200                 205
   Val Asn Gly Trp Gln Asp Leu Ser Ala Ala Leu Leu Lys Thr Asp Val
       210                 215                 220
   Lys Pro Pro Pro Gly Lys Ser Pro Ala Lys Phe Ala Pro Ile Glu Lys
   225                 230                 235                 240
   Met Gly Val Arg Thr Ala Glu Gln Ala Ala Asn Thr Leu Gly Ile Leu
                   245                 250                 255
   Leu Phe Val Glu Val Pro Gly Gly Leu Thr Val Ala Lys Cys Leu Glu
               260                 265                 270
   Leu Phe His Ala Ser Gly Gly Lys
           275                 280
(2) INFORMATION FOR SEQ ID NO:129:
   (i) SEQUENCE CHARACTERISTICS:
      (A) LENGTH: 280 amino acids
      (B) TYPE: amino acid
      (D) TOPOLOGY: linear
   (ii) MOLECULE TYPE: protein
   (xi) SEQUENCE DESCRIPTION: SEQ ID NO:129:
   Ala Ala Lys Met Ala Lys Asn Val Asp Lys Pro Leu Phe Thr Ala Thr
   1               5                   10                  15
   Phe Asn Val Gln Ala Ser Ser Ala Asp Tyr Ala Thr Phe Ile Ala Gly
               20                  25                  30
   Ile Arg Asn Lys Leu Arg Asn Pro Ala His Phe Ser His Asn Arg Pro
           35                  40                  45
   Val Leu Pro Pro Val Glu Pro Asn Val Pro Pro Ser Arg Trp Phe His
       50                  55                  60
   Val Val Leu Lys Ala Ser Pro Thr Ser Ala Gly Leu Thr Leu Ala Ile
   65                  70                  75                  80
   Arg Ala Asp Asn Ile Tyr Leu Glu Gly Phe Lys Ser Ser Asp Gly Thr
                   85                  90                  95
   Trp Trp Glu Leu Thr Pro Gly Leu Ile Pro Gly Ala Thr Tyr Val Gly
               100                 105                 110
   Phe Gly Gly Thr Tyr Arg Asp Leu Leu Gly Asp Thr Asp Lys Leu Thr
           115                 120                 125
   Asn Val Ala Leu Gly Arg Gln Gln Leu Ala Asp Ala Val Thr Ala Leu
       130                 135                 140
   His Gly Arg Thr Lys Ala Asp Lys Ala Ser Gly Pro Lys Gln Gln Gln
   145                 150                 155                 160
   Ala Arg Glu Ala Val Thr Thr Leu Val Leu Met Val Asn Glu Ala Thr
                   165                 170                 175
   Arg Phe Gln Thr Val Ser Gly Phe Val Ala Gly Leu Leu His Pro Lys
               180                 185                 190
   Ala Val Glu Lys Lys Ser Gly Lys Ile Gly Asn Glu Met Lys Ala Gln
           195                 200                 205
   Val Asn Gly Trp Gln Asp Leu Ser Ala Ala Leu Leu Lys Thr Asp Val
       210                 215                 220
   Lys Pro Pro Pro Gly Lys Ser Pro Ala Lys Phe Ala Pro Ile Glu Lys
   225                 230                 235                 240
   Met Gly Val Arg Thr Ala Glu Gln Ala Ala Asn Thr Leu Gly Ile Cys
                   245                 250                 255
   Leu Phe Val Glu Val Pro Gly Gly Leu Thr Val Ala Lys Ala Leu Glu
               260                 265                 270
   Leu Phe His Ala Ser Gly Gly Lys
               275                 280
(2) INFORMATION FOR SEQ ID NO:130:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:130:
   Ser Cys Asp Lys Thr His Thr
   1               5
(2) INFORMATION FOR SEQ ID NO:131:
   (i) SEQUENCE CHARACTERISTICS:
      (A) LENGTH: 85 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:131:
(2) INFORMATION FOR SEQ ID NO:132:
   (i) SEQUENCE CHARACTERISTICS:
      (A) LENGTH: 86 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:132:
   AAGGTATACC CAGAAGCTGC GCAGGAGATT CTGACGGACC CTCCAGGCTT CACCAGGCCT    60
   CCTCCAGACT GCACCAACTG GATCTC                                         86
(2) INFORMATION FOR SEQ ID NO:133:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:133:
   GCAGCTTCTG GGTATACCTT CACAAACTAT GGAATGAACT GGGTGCGCCA GGCTCCAGGA 60
   AAGAATTTAG AGTGGATGGG CTGG 84
(2) INFORMATION FOR SEQ ID NO:134:
   (i) SEQUENCE CHARACTERISTICS:
      (A) LENGTH: 85 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:134:
   AAAGAGAAGG TAAACCGTCC CTTGAAAGAA TCAGCATATG TTGGCTCTCC AGTGTGGGTG 60
   TTTATCCAGC CCATCCACTC TAAAC 85
(2) INFORMATION FOR SEQ ID NO:135:
   (i) SEQUENCE CHARACTERISTICS:
      (A) LENGTH: 87 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:135:
   GACGGTTTAC CTTCTCTTTG GACGATTCTA AGAACACTGC CTATTTACAG ATCAACAGCC 60
   TCAGAGCCGA GGACACGGCT GTGTATT 87
(2) INFORMATION FOR SEQ ID NO:136:
   (i) SEQUENCE CHARACTERISTICS:
      (A) LENGTH: 92 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:136:
   GAGGAGACGG TGACCGTGGT CCCTTGGCCC CAGACATCGA AGTACCAGTC GTAACCCCGT 60
   CTTGTACAGA AATACACAGC CGTGTCCTCG GC 92
(2) INFORMATION FOR SEQ ID NO:137:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:137:
   GCAGCTTCTG GGTATACCTT CACAAACTAT GGAATGAACT GGGTGAAGCA GGCTCCAGGA 60
   AAGGGTTTAA GGTGGATGGG CTGG 84
(2) INFORMATION FOR SEQ ID NO:138:
   (i) SEQUENCE CHARACTERISTICS:
      (A) LENGTH: 85 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:138:
   AAAGAGAAGG TAAACCGTCC CTTGAAGTCA TCAGCATATG TTGGCTCTCC AGTGTGGGTG 60
   TTTATCCAGC CCATCCACCT TAAAC 85
(2) INFORMATION FOR SEQ ID NO:139:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:139:
   GACGGTTTAC CTTCTCTTTG GACACGTCTA AGTGCACTGC CTATTTACAG ATCAACAGCC 60
   TCAGAGCCGA GGACACGGCT ACAT 84
(2) INFORMATION FOR SEQ ID NO:140:
   (i) SEQUENCE CHARACTERISTICS:
      (A) LENGTH: 91 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:140:
   AGGAGACGGT GACCGTGGTC CCTTGGCCCC AGACATCGAA GTACCAGTCG TAACCCCGTC 60
   TTGTACAGAA ATATGTAGCC GTGTCCTCGG C 91
(2) INFORMATION FOR SEQ ID NO:141:
   (i) SEQUENCE CHARACTERISTICS:
      (A) LENGTH: 8 amino acids
      (B) TYPE: amino acid
      (D) TOPOLOGY: linear
   (ii) MOLECULE TYPE: protein
   (xi) SEQUENCE DESCRIPTION: SEQ ID NO:141:
   Lys Pro Ala Lys Phe Phe Arg Leu
   1 5
(2) INFORMATION FOR SEQ ID NO:142:
   (i) SEQUENCE CHARACTERISTICS:
      (A) LENGTH: 8 amino acids
      (B) TYPE: amino acid
      (D) TOPOLOGY: linear
   (ii) MOLECULE TYPE: protein
   (xi) SEQUENCE DESCRIPTION: SEQ ID NO:142:
   Lys Pro Ala Lys Phe Leu Arg Leu
   1 5
(2) INFORMATION FOR SEQ ID NO:143:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:143:
   GGCCGCAAAG CCGGCTAAGT TCTTMCGTCT GAGT 34
(2) INFORMATION FOR SEQ ID NO:144:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:144:

## Description

### FIELD OF THE INVENTION

The present invention generally relates to materials useful as components of cytotoxic therapeutic agents. More particularly, the invention relates to fusion proteins comprising gelonin.

### BACKGROUND

Ribosome-inactivating proteins (RIPs) comprise a class of proteins which is ubiquitous in higher plants. However, such proteins have also been isolated from bacteria. RIPs are potent inhibitors of eukaryotic protein synthesis. The N-glycosidic bond of a specific adenine base is hydrolytically cleaved by RIPs in a highly conserved loop region of the 28S rRNA of eukaryotic ribosomes thereby inactivating translation.

Plant RIPs have been divided into two types. Stirpe *et al*., *FEBS Lett., 195(1,2)*:1-8 (1986). Type I proteins each consist of a single peptide chain having ribosome-inactivating activity, while Type II proteins each consist of an A-chain, essentially equivalent to a Type I protein, disulfide-linked to a B-chain having cell-binding properties. Gelonin, dodecandrin, tricosanthin, tricokirin, bryodin, *Mirabilis* antiviral protein (MAP), barley ribosome-inactivating protein (BRIP), pokeweed antiviral proteins (PAPs), saporins, luffins, and momordins are examples of Type I RIPs; whereas Ricin and abrin are examples of Type II RIPs.

Amino acid sequence information is reported for various ribosome-inactivating proteins. It appears that at least the tertiary structure of RIP active sites is conserved among Type I RIPS, bacterial RIPs, and A-chains of Type II RIPs. In many cases, primary structure homology is also found. Ready *et al*., *J. Biol. Chem*., *259(24)*:15252-15256 (1984) and other reports suggest that the two types of RIPS are evolutionarily related.

Type I plant ribosome-inactivating proteins may be particularly suited for use as components of cytotoxic therapeutic agents. A RIP may be conjugated to a targeting agent which will deliver the RIP to a particular cell type *in vivo* in order to selectively kill those cells. Typically, the targeting agent (*e*.*g*., an antibody) is linked to the toxin by a disulfide bond which is reduced *in vivo* allowing the protein toxin to separate from the delivery antibody and become active intracellularly. Another strategy for producing targeted cytotoxic proteins is to express a gene encoding a cytotoxic protein fused to a gene encoding a targeting moiety. The resulting protein product is composed of one or more polypeptides containing the cytotoxic protein linked to, for example, at least one chain of an antibody.

A variety of such gene fusions are discussed in Pastan *et al., Science*, *254*:1173-1177 (1991). However, these fusion proteins have been constructed with sequences from diphtheria toxin or *Pseudomonas aeruginosa* exotoxin A, both of which are ADP-ribosyltransferases of bacterial origin. These protein toxins are reported to intoxicate cells and inhibit protein synthesis by mechanisms which differ from those of the RIPs. Moreover, diphtheria toxin and exotoxin A are structurally different from, and show little amino acid sequence similarity with, RIPs. In general, fusion proteins made with diphtheria toxin or exotoxin A have been immunogenic and toxic in animals, and are produced intracellularly in relatively low yield. Another strategy for producing a cytotoxic agent is to express a gene encoding a RIP fused to a gene encoding a targeting moiety. The resulting protein product is a single polypeptide containing a RIP linked to, for example, at least one chain of an antibody.

Because some RIPs, such as the Type I RIP gelonin, are primarily available from scarce plant materials, it is desirable to clone the genes encoding the RIPs to enable recombinant production of the proteins. It is also desirable to develop analogs of the natural proteins which may be easily conjugated to targeting molecules while retaining their natural biological activity because most Type I RIPs have no natural sites (*i.e*. available cysteine residues) for conjugation to targeting agents. Alternatively, it is desirable to develop gene fusion products including Type I RIPs as a toxic moiety and antibody substances as a targeting moiety.

The present invention also provides novel humanized or human-engineered antibody fusion proteins. Such fusions are useful in the treatment of various disease states, including autoimmune diseases and cancer.

There are several reports relating to replacement of amino acids in a mouse antibody with amino acids normally occurring at the analogous position in the human form of the antibody. *See, e.g.*, Junghaus, *et al., Cancer Res., 50*: 1495-1502 (1990) and other publications which describe genetically-engineered mouse/human chimeric antibodies. Also by genetic engineering techniques, the genetic information from murine hypervariable complementarity determining regions (hereinafter referred to as "CDRs") may be inserted in place of the DNA encoding the CDRs of a human monoclonal antibody to generate a construct encoding a human antibody with murine CDRs. *See, e.g.,* Jones, *et al., Nature, 321*: 522-525 (1986).

Protein structure analysis on such "CDR-grafted" antibodies may be used to "add back" murine residues in order to restore lost antigen-binding capability, as described in Queen, *et al*, *Proc. Natl. Acad. Sci. (USA), 86*:10029-10033 (1989); Co, *et al*., *Proc. Nat. Acad. Sci. (USA)*, *88*: 2869-2873 (1991). However, a frequent result of CDR-grafting is that the specific binding acitvity of the resulting humanized antibodies may be diminished or completely abolished.

As demonstrated by the foregoing, there exists a need in the art for cloned genes encoding Type I RIPs, gene fusion products comprising Type I RIPs, and especially wherein such gene fusions also comprise an humanized antibody portion.

### SUMMARY OF THE INVENTION

According to one aspect of the invention, there are provided fusion proteins comprising gelonin fused to the carboxy terminal end of an antigen-binding portion of the antibody designated he3 obtainable from cell line ATTC HB11206.

The fusion proteins may lack a cleavable peptide segment linking said gelonin and said antibody portion, in which case they may be of the structure V_{L}V_{H}::gelonin or V_{H}V_{L}::gelonin.

The invention further provides fusion proteins comprising gelonin fused to the amino terminal end of an antigen-binding portion of the antibody designated he3 obtainable from cell line ATTC 11206, said fusion protein having the structure Gelonin::kappa,Fd or gelonin::Fd,kappa.

The invention also provides fusion proteins comprising gelonin fused to the amino or carboxy terminal end of an antigen-binding portion of an antibody, said fusion protein further comprising the cleavage site-containing peptide segment KPAKFLRL or KPAKFFRL between said gelonin and said antibody portion.

The invention further provides the following fusion proteins:
- the fusion protein (Gelonin::RMA::kappa,Fd')₂, wherein "RMA" represents the sequence PSGQAGAAASESLFISNHAY.
- the fusion protein (Gelonin::RMA::Fd',kappa)₂, wherein "RMA" represents the sequence PSGQAGAAASESLFISNHAY.

The invention also provides fusion proteins comprising gelonin fused to the amino terminal end of an antigen-binding portion of the antibody designated he3 obtainable from cell line ATTC 11206, said fusion protein having the structure Gelonin::RMA::kappa,Fd, wherein "RMA" represents the sequence PSGQAGAAASESLFISNHAY, or PSGQAGAAASESLFISNHAY.

The invention also provides fusion proteins comprising gelonin fused to the amino terminal end of an antigen-binding portion of the antibody designated he3 obtainable from cell line ATTC 11206, said fusion protein having the structure Gelonin::SLT::kappa,Fd or Gelonin::SLT::Fd,kappa, wherein SLT represents the sequence CHHHASRVARMASDEFPSMC.

The invention further provides the fusion proteins V_{H}V_{L}::SLT::Gelonin and V_{L}V_{H}::SLT::Gelonin, wherein "SLT" represents the sequence CHHHASRVARMASDEFPSMC.

The invention also provides the fusion proteins V_{H}V_{L}::RMA::Gelonin and V_{L}V_{H}::RMA::Gelonin, wherein "RMA" represents the sequence PSGQAGAAASESLFISNHAY.

Polynucleotides encoding the above fusion proteins as well as host cells transformed or transfected with these polynucleotides form further aspects of the invention.

A purified and isolated polynucleotide encoding natural sequence gelonin (SEQ ID NO: 11), and a host cell including a vector encoding gelonin of the type deposited as ATCC Accession No. 68721 are provided.

Some of the polynucleotides mentioned above encode fusion proteins of the present invention comprising gelonin (SEQ ID NO:2) and an antibody or a fragment comprising an antigen-binding portion thereof. Several alternate forms of fusion proteins comprising gelonin are contemplated herein. For example, the fusion protein may contain a single RIP fused to a monovalent antibody binding moiety, such as a Fab or single chain antibody. Alternatively, multivalent forms of the fusion proteins may be made and may have greater activity than the monovalent forms. In preferred embodiments of the invention, gelonin may be fused to either the carboxy or the amino terminus of the antibody or antigen-binding portion of thereof. Also in a preferred embodiment of the invention, the antibody or fragment thereof comprises an antigen-binding portion may be an he3 antibody, an he3-Fab, an he3 Fd, single-chain antibody, or an he3 kappa fragment. The antibody or antigen-binding portion thereof may be fused to gelonin by means of a linker peptide, preferably a peptide segment of shiga-like toxin as shown in SEQ ID NO: 56 or a peptide segment of Rabbit muscle aldolase as shown in SEQ ID NO: 57 or a human muscle aldolase, an example of which is reported in Izzo, *et al., Eur. J. Biochem, 174*: 569-578 (1988), incorporated by reference herein.

The fusion proteins of the invention may be used for preparing an agent toxic to a cell.

The fusion proteins of the invention may be used for treating a disease in which elimination of particular cells is a goal may include the step of administering to a patient having the disease a therapeutically effective amount thereof to the cells.

Useful target cells for immunotoxins of the present invention include, but are not limited to, cells which are pathogenic, such as cancer cells, autoimmune cells, and virally-infected cells. Such pathogenic cells may be targeted by antibodies or other targeting agents of the invention which are joined, either by genetic engineering techniques or by chemical cross-linking, to an RIP. Specifically useful targets include tumor-associated antigens (*e.g*., on cancer cells), cell differentiation markers (*e.g*., on autoimmune cells), parasite-specific antigens, bacteria-specific antigens, and virus-specific antigens.

The fusion proteins of the invention are particularly suited for use as components of cytotoxic therapeutic agents. Cytotoxic agents according to the present invention may be used *in vivo* to selectively eliminate any cell type to which the RIP component is targeted by the specific binding capacity of the second component.

The gelonin of the invention may preferably be conjugated or fused to humanized or human engineered antibodies, such as the he3 antibody described herein. Such humanized antibodies may be constructed from mouse antibody variable domains, such as the mouse antibody H65 (SEQ ID NOS: 123 and 124). Specifically gelonin according to the present invention may be fused to he3 human-engineered antibody light and heavy chain variable regions (SEQ ID NO: 125 and 126, respectively) or fragments thereof. A cell line producing an intact he3 immunoglobulin was deposited as ATCC Accession No. HB11206 with the American Type Culture Collection, 12301 Parklawn Drive, Rockville, Maryland 20852.

Gelonin may also be conjugated to targeting agents other than antibodies, for example, lectins which bind to cells having particular surface carbohydrates, hormones, lymphokines, growth factors or other polypeptides which bind specifically to cells having particular receptors. Immunoconjugates including RIPs may be described as immunotoxins. An immunotoxin may also consist of a fusion protein rather than an immunoconjugate.

The present invention provides gene fusions of an antigen-binding portion of an antibody (*e.g.*, an antibody. light chain or truncated heavy chain, or a single chain antibody) or any targeting agent listed in the foregoing paragraph, linked to a Type I RIP. Preferably, the antigen-binding portion of an antibody or fragment thereof comprises a single chain antibody, a Fab fragment, or a F(ab')₂ fragment. Active antibodies generally have a conserved three-dimensional folding pattern and it is expected that any antibody which maintains that folding pattern will retain binding specificity. Such antibodies are expected to retain target enzymatic activity when incorporated into a fusion protein according to the present invention.

The fusion proteins of the invention may include cleavable linkers (*i.e.*, disulfides, acid-sensitive linkers, and the like) in order to allow intracellular release of the cytotoxic component. Such intracellular release allows the cytotoxic component to function unhindered by possible negative kinetic or steric effects of the attached antibody. Accordingly, gene fusions of the present invention may comprise gelonin fused, via a DNA segment encoding a linker protein as described above, to either the 5' or the 3' end of a gene encoding an antibody. If a linker is used, it preferably encodes a polypeptide which contains two cysteine residues participating in a disulfide bond and forming a loop which includes a protease-sensitive amino acid sequence (*e.g.*, a segment of *E. coli* shiga-like toxin as in SEQ ID NO: 56) or a segment which contains several cathepsin cleavage sites (*e.g*., a segment of rabbit muscle aldolase as in SEQ ID NO: 57; a segment of human muscle aldolase; or a synthetic peptide including a cathepsin cleavage sites such as in SEQ ID NOs: 141 or 142). A linker comprising cathepsin cleavage sites as exemplified herein comprises the C-terminal 20 amino acids of RMA. However, that sequence differs by only one amino acid from human muscle aldolase and it is contemplated that muscle aldolase from human or other sources may be used as a linker in the manner described below. Also preferably, the antibody portion of the fused genes comprises sequences encoding one of the chains of an antibody Fab fragment (*i.e.*, kappa or Fd) and the fused gene is co-expressed in a host cell with the other Fab gene, or the antibody portion comprises sequences encoding a single chain antibody.

Alternatively, since fusion proteins of the present invention may be of low (approximately 55 kDa) molecular weight while maintaining full enzymatic activity, such fusions may be constructed without a linker and still possess cytotoxic activity. Such low-molecular weight fusions are not as susceptible to kinetic and steric hinderance as are the larger fusions, such as fusions involving IgG molecules. Therefore, cleavage of the gelonin away from the fusion may not be necessary to facilitate activity of the gelonin.

Immunotoxins including immunoconjugates and fusion proteins according to the invention (immunofusions), are suited for treatment of diseases where the elimination of a particular cell type is a goal, such as autoimmune disease, cancer, and graft-versus-host disease. The immunotoxins are also suited for use in causing immunosuppression and in treatment of infections by viruses such as the Human Immunodeficiency Virus.

Specifically illustrating polynucleotide sequences according to the present invention are the inserts in the plasmid pING3731 in *E. coli* MC1061 (designated strain G274) and in the plasmid pING3803 in *E. coli* E104 (designated strain G275), both deposited with the American Type Culture Collection (ATCC), 12301 Parklawn Drive, Rockville, Maryland, on October 2, 1991, and assigned ATCC Accession Nos. 68721 and 68722, respectively. Additional polynucleotide sequences illustrating the invention are the inserts in the plasmid pING3746 in *E*. *coli* E104 (designated strain G277) and in the plasmid pING3737 in *E. coli* E104 (designated strain G276), which were both deposited with the ATCC on June 9, 1992, and were respectively assigned Accession Nos. 69008 and 69009. Still other polynucleotide sequences illustrating the invention are the inserts in the plasmid pING3747 in *E. coli* E104 (designated strain G278), in the plasmid pING3754 in *E. coli* E104 (designated strain G279), in the plasmid pING3758 in *E. coli* E104 (designated strain G280) and in the plasmid pING3759 in *E. coli* E104 (designated strain G281), which plasmids were all deposited with the ATCC on October 27, 1992 and were assigned ATCC Accession Nos. 69101, 69102, 69103 and 69104, respectively.

As noted above, according to the invention gelonin may be fused to humanized or human-engineered antibodies, such as he3. Thus, the present invention also provides novel proteins comprising an humanized antibody variable domain which is specifically reactive with an human CD5 cell differentiation marker. Specifically, the present invention provides proteins comprising the he3 light and heavy chain variable regions as shown in SEQ ID NOS: 95 or 96, respectively. DNA encoding certain he3 proteins is shown in SEQ ID NOS: 67 and 68.

In a preferred embodiment of the present invention, the protein comprising an humanized antibody variable region is an intact he3 immunoglobulin deposited as ATCC HB 11206.

Also in a preferred embodiment of the invention, the protein comprising an humanized antibody variable region is a Fab or F(ab')₂ or Fab fragment.

Proteins according to the present invention may be made by methods taught herein and in EP 0 571 613.

### BRIEF DESCRIPTION OF THE DRAWINGS

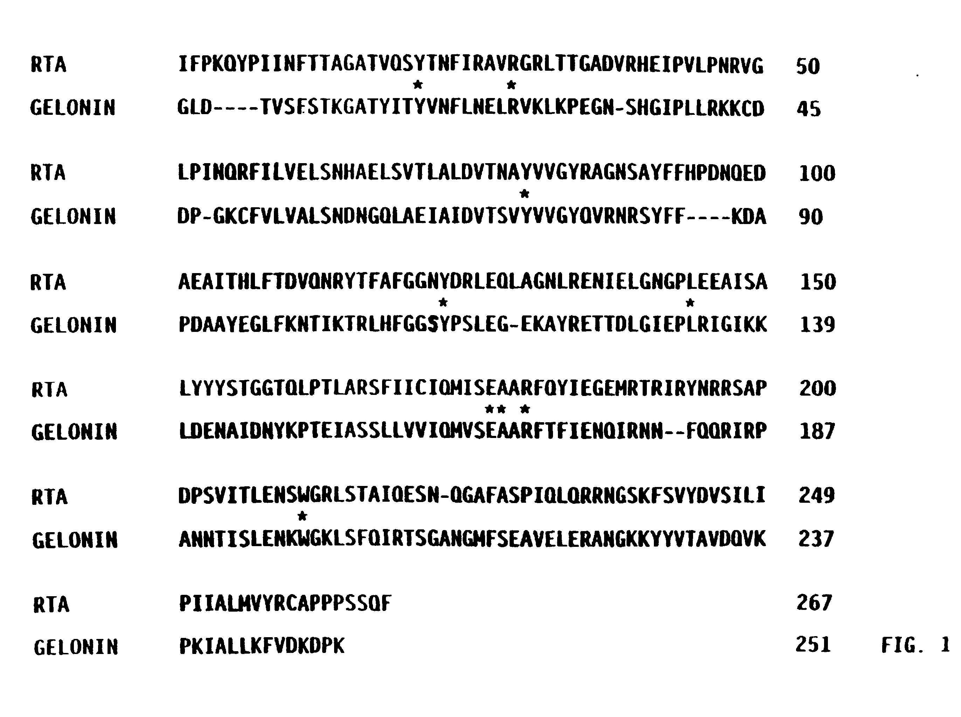

FIG. 1 is a computer-generated alignment of the amino acid sequence of the ricin A-chain (RTA) (SEQ ID NO: 1) with the amino acid sequence of the Type I ribosome-inactivating protein gelonin (SEQ ID NO: 2), wherein starred positions indicate amino acids invariant among the ricin A-chain and the Type I RIPs;

### DETAILED DESCRIPTION

Nucleotide sequences of genes encoding gelonin and expression vectors containing the genes are provided by the present invention. A plant RIP, gelonin, is produced by seeds of *Gelonium multiflorum,* a plant of the Euphorbiaceae family native to the tropical forests of eastern Asia.

Type I RIP analogs e.g. gelonin offer distinct advantages over the natural proteins for use as components of immunotoxins. Chemical treatment to introduce free sulfhydryl groups in the natural proteins lacking free cysteines typically involves the non-selective modification of amino acid side chains. This non-selectivity often results in antibodies conjugated to different sites on different RIP molecules (*i.e.*, a heterogeneous population of conjugates) and also in a decrease in RIP activity if antibodies are conjugated in or near important regions of the RIP (*e.g*., the active site or regions involved in translocation across cell membranes). In contrast, RIP analogs according to the present invention may be conjugated to a single antibody through a disulfide bond to a specific residue of the analog resulting in reduced batch to batch variation of the immunoconjugates and, in some cases, immunoconjugates with enhanced properties (*e.g*., greater cytotoxicity or solubility).

Type I plant RIPs, as well as bacterial RIPs such as shiga and shiga-like toxin A-chains, are homologous to the ricin A-chain and are useful in the present invention.

Type I RIPS may be defined and sites for substitution of a cysteine in a RIP may be identified by comparing the primary amino acid sequence of the RIP to the natural ricin A-chain amino acid sequence, the tertiary structure of which has been described in Katzin *et al., Proteins, 10*:251-259 (1991), which is incorporated by reference herein.

Amino acid sequence alignment defines Type I RIPs in that the ricin A-chain and the Type I plant RIPs have nine invariant amino acids in common. Based on the ricin sequence the invariant amino acids are tyrosine₂₁, arginine₂₉, tyrosine₈₀, tyrosine₁₂₃, leucine₁₄₄, glutamic acid₁₇₇, alanine₁₇₈, arginine₁₈₀, and tryptophan₂₁₁. The ricin A-chain may be used as a model for the three-dimensional structure of Type I RIPs. A protein lacking a cysteine available for conjugation while having ribosome-inactivating activity and having the nine invariant amino acids when its primary sequence is compared to the primary sequence of the ricin A-chain [according to the alignment algorithm of Myers *et al., CABIOS COMMUNICATIONS, 4(1)*:11-17 (1988), implemented by the PC/GENE program PALIGN (Intelligenetics, Inc., Mountain View, California) and utilizing the Dayhoff Mutation Data Matrix (MDM-78) as described in Schwartz *et al.*, pp. 353-358 in Atlas *of Protein Sequence and Structure*, 5 Supp. 3, National Biomedical Research Foundation, Washington, D.C. (1978)] is defined as a Type I RIP herein and is expected to be useful in the present invention. "Corresponding" refers herein to amino acid positions which align when two amino acid sequences are compared by the strategy of Myers *et al., supra*.

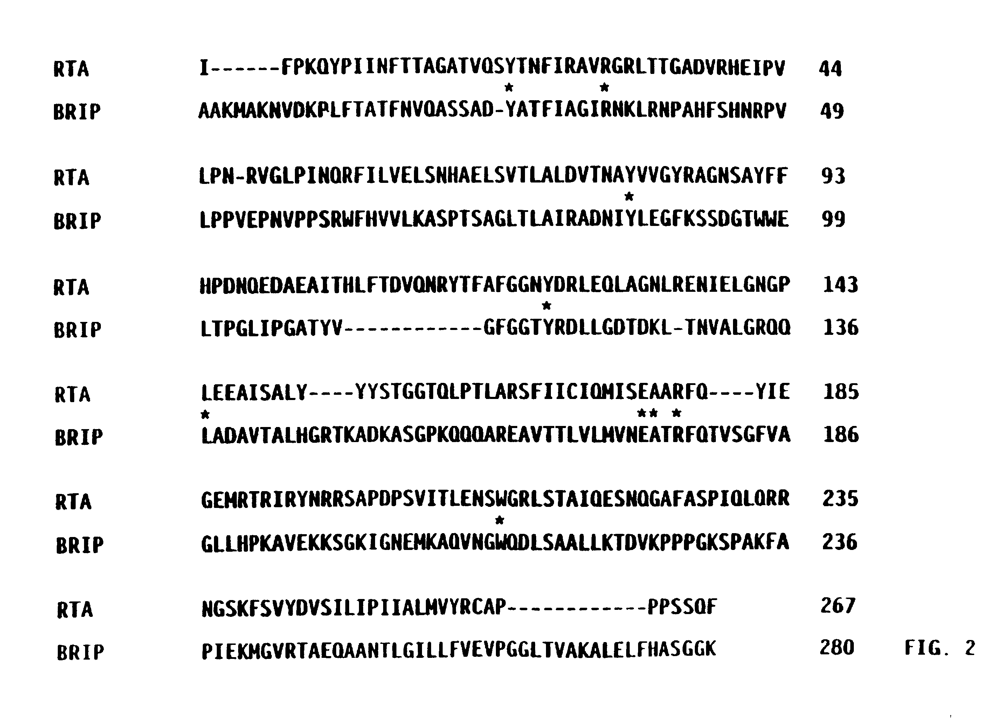

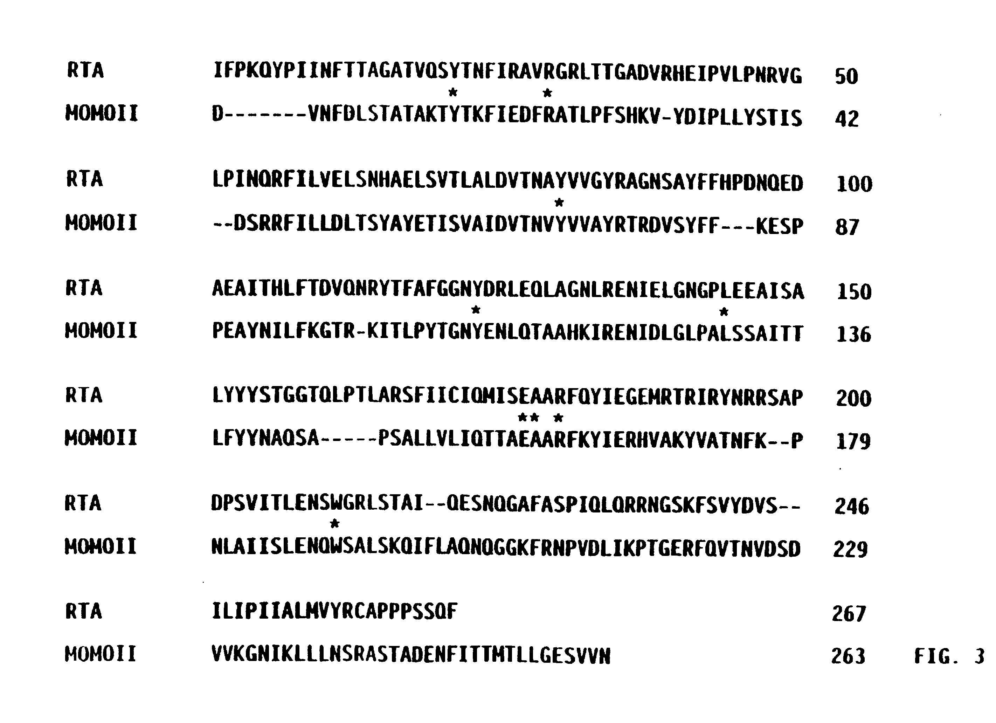

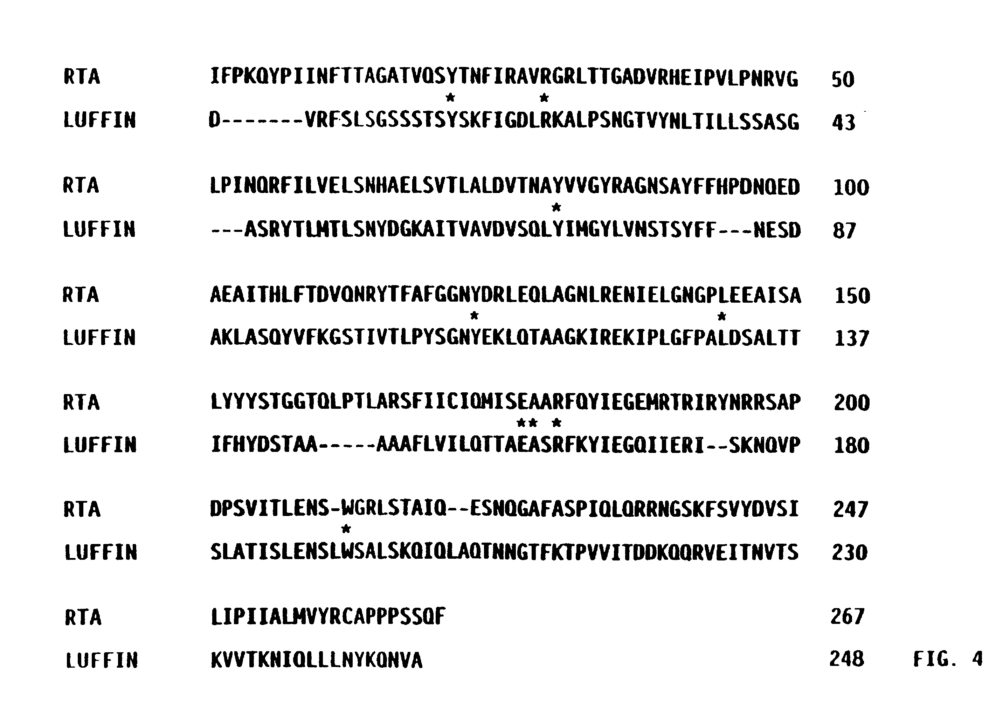

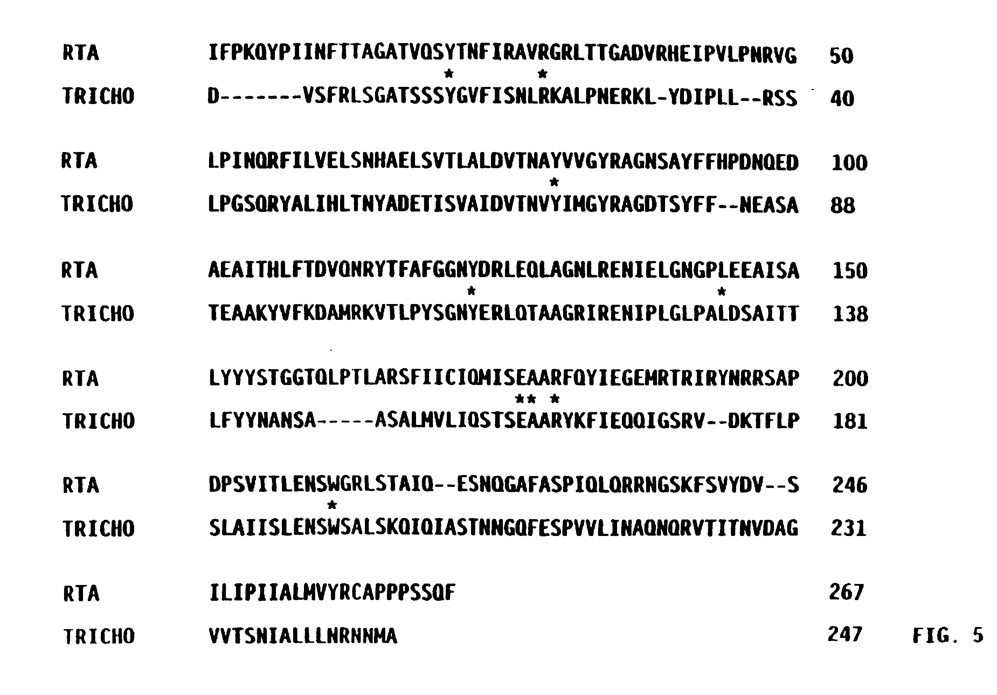

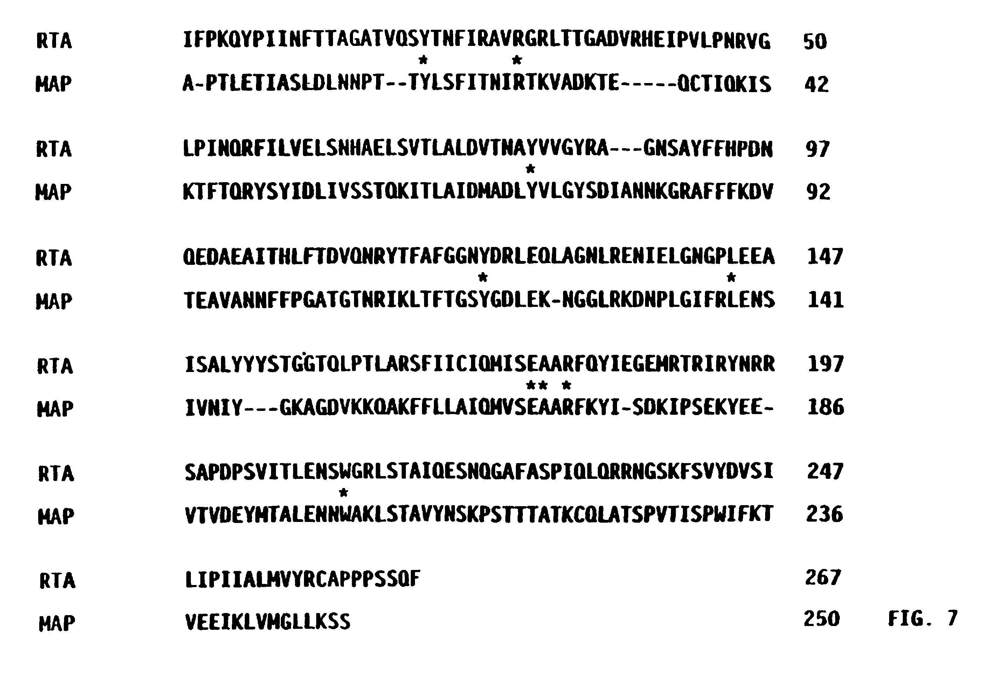

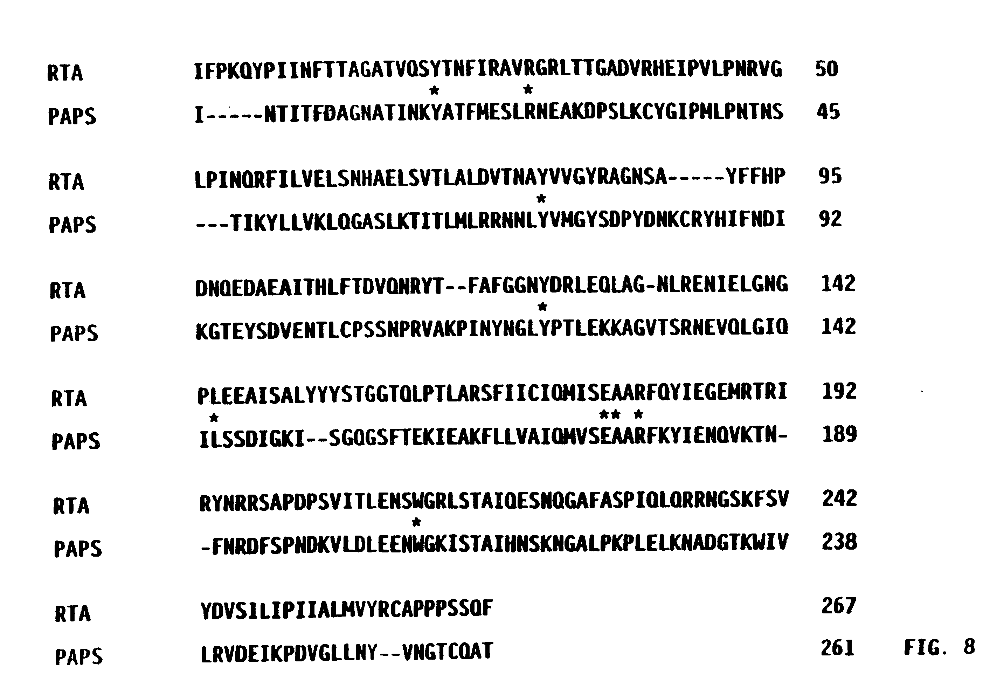

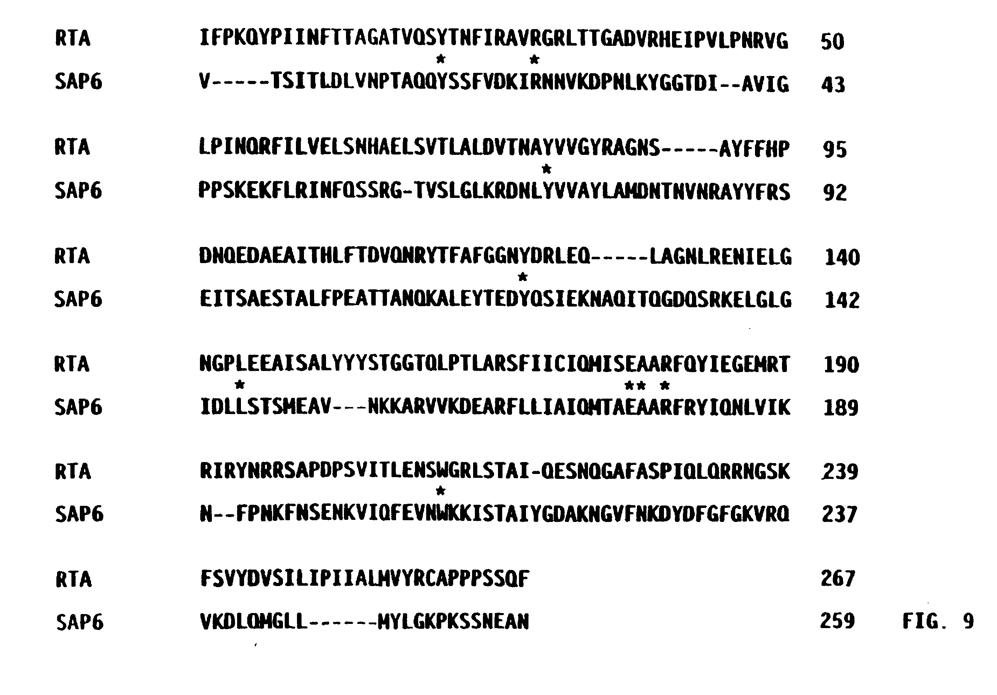

The primary amino acid sequences of the Type I RIPs:gelonin, BRIP, momordin II, luffin [see Islam *et al., Agricultural Biological Chem., 54(5)*:1343-1345 (199)], αtrichosanthin [see Chow *et al., J. Biol. Chem., 265*:8670-8674 (1990)], momordin I [see Ho *et al., BBA, 1088*:311-314 (1991)], *Mirabilis* anti-viral protein [see Habuka *et al.,* *J. Biol. Chem., 264(12)*:6629-6637 (1989)], pokeweed antiviral protein isolated from seeds [see Kung *et al., Agric. Biol. Chem., 54(12)*:3301-3318 (1990)] and saporin [see Benatti *et al., Eur. J. Biochem., 183*:465-470 (1989)] are individually aligned with the primary sequence of the ricin A-chain [see Hailing *et al., Nucleic Acids Res.*, *13*:8019-8033 (1985)] in FIGS 1-9, respectively, according to the algorithm of Myers *et al., supra, as* specified above.

For purposes of the present invention, immunotoxins comprise a class of compounds of which toxin-antibody fusions and immunoconjugates are examples. Immunotoxins are particularly suited for use in treatment of human autoimmune diseases and in the treatment of diseases in which depletion of a particular cell type is a goal, such as cancer. For example, treatment of autoimmune diseases with immunotoxins is described in International Publication No. WO89/06968 published August 10, 1989, which is incorporated by reference herein.

In any treatment regimen, the immunotoxins may be administered to a patient either singly or in a cocktail containing two or more immunotoxins, other therapeutic agents, compositions, or the like, including, but not limited to, immunosuppressive agents, tolerance-inducing agents, potentiators and side-effect relieving agents. Particularly preferred are immunosuppressive agents useful in suppressing allergic reactions of a host. Preferred immunosuppressive agents include prednisone, prednisolone, DECADRON (Merck, Sharp & Dohme, West Point, Pennsylvania), cyclophosphamide, cyclosporine, 6-mercaptopurine, methotrexate, azathioprine and i.v. gamma globulin or their combination. Preferred potentiators include monensin, ammonium chloride, perhexiline, verapamil, amantadine and chloroquine. All of these agents are administered in generally-accepted efficacious dose ranges such as those disclosed in the *Physician's Desk Reference,* 41st Ed., Publisher Edward R. Barnhart, New Jersey (1987). Patent Cooperation Treaty (PCT) patent application WO 89/069767 published on August 10, 1989, discloses administration of an immunotoxin as an immunosuppressive agent and is incorporated by reference herein.

Immunotoxins that are fusion proteins of the present invention may be formulated into either an injectable or topical preparation. Parenteral formulations are known and are suitable for use in the invention, preferably for intramuscular or intravenous administration. The formulations containing therapeutically-effective amounts of immunotoxins are either sterile liquid solutions, liquid suspensions, or lyophilized versions, and optionally contain stabilizers or excipients. Lyophilized compositions are reconstituted with suitable diluents, *e.g*., water for injection, saline, 0.3% glycine and the like, at a level of about from 0.01 mg/kg of host body weight to 10 mg/kg where the biological activity is less than or equal to 20 ng/ml when measured in a reticulocyte lysate assay. Typically, the pharmaceutical compositions containing immunotoxins of the present invention are administered in a therapeutically effective dose in a range of from about 0.01 mg/kg to about 5 mg/kg of the patient. A preferred, therapeutically effective dose of the pharmaceutical composition containing immunotoxins of the invention is in a range of from about 0.01 mg/kg to about 0.5 mg/kg body weight of the patient administered over several days to two weeks by daily intravenous infusion, each given over a one hour period, in a sequential patient dose-escalation regimen.

Immunotoxin that are fusion proteins according to the invention may be formulated into topical preparations for local therapy by including a therapeutically effective concentration of immunotoxin in a dermatological vehicle. The amount of immunotoxin to be administered, and the immunotoxin concentration in the topical formulations, depend upon the vehicle selected, the clinical condition of the patient, the systemic toxicity and the stability of the immunotoxin in the formulation. Thus, a physician knows to employ the appropriate preparation containing the appropriate concentration of immunotoxin in the formulation, as well as the appropriate amount of formulation to administer depending upon clinical experience with the patient in question or with similar patients. The concentration of immunotoxin for topical formulations is in the range of greater than from about 0.1 mg/ml to about 25 mg/ml. Typically, the concentration of immunotoxin for topical formulations is in the range of greater than from about 1 mg/ml to about 20 mg/ml. Solid dispersions of immunotoxins according to the invention, as well as solubilized preparations, may be used. Thus, the precise concentration to be used in the vehicle is subject to modest experimental manipulation in order to optimize the therapeutic response. For example, greater than about 10 mg immunotoxin/100 grams of vehicle may be useful with 1% w/w hydrogel vehicles in the treatment of skin inflammation. Suitable vehicles, in addition to gels, are oil-in-water or water-in-oil emulsions using mineral oils, petroleum and the like.

Immunotoxins that are fusion proteins according to the invention may be optionally administered topically by the use of a transdermal therapeutic system [Barry, *Dermatological Formulations*, p. 181 (1983) and literature cited therein]. While such topical delivery systems may be designed for transdermal administration of low molecular weight drugs, they are capable of percutaneous delivery. Further, such systems may be readily adapted to administration of immunotoxin or derivatives thereof and associated therapeutic proteins by appropriate selection of the rate-controlling microporous membrane.

Topical preparations of immunotoxins that are fusion proteins of the invention, either for systemic or local delivery may be employed and may contain excipients as described above for parenteral administration and other excipients used in a topical preparation such as cosolvents, surfactants, oils, humectants, emollients, preservatives, stabilizers and antioxidants. Pharmacologically-acceptable buffers may be used, *e.g.*, Tris or phosphate buffers. The topical formulations may also optionally include one or more agents variously termed enhancers, surfactants, accelerants, adsorption promoters or penetration enhancers, such as an agent for enhancing percutaneous penetration of the immunotoxin or other agents. Such agents should desirably possess some or all of the following features as would be known to the ordinarily skilled artisan: pharmacological inertness, non-promotive of body fluid or electrolyte loss, compatible with immunotoxin (non-inactivating), and capable of formulation into creams, gels or other topical delivery systems as desired.

Immunotoxins that are fusion proteins according to the present invention may also be administered by aerosol to achieve localized delivery to the lungs. This is accomplished by preparing an aqueous aerosol, liposomal preparation or solid particles containing immunotoxin. ordinarily, an aqueous aerosol is made by formulating an aqueous solution or suspension of immunotoxin together with conventional pharmaceutically acceptable carriers and stabilizers. The carriers and stabilizers vary depending upon the requirements for the particular immunotoxin, but typically include: nonionic surfactants (Tweens, Pluronics, or polyethylene glycol); innocuous proteins like serum albumin, sorbitan esters, oleic acid, lecithin; amino acids such as glycine; and buffers, salts, sugars or sugar alcohols. The formulations may also include mucolytic agents as well as bronchodilating agents. The formulations are sterile. Aerosols generally are prepared from isotonic solutions. The particles optionally include normal lung surfactants.

Alternatively, immunotoxins that are fusion proteins of the invention may be administered orally by delivery systems such as proteinoid encapsulation as described by Steiner, *et al.*, U.S. Patent No. 4,925,673, incorporated by reference herein. Typically, a therapeutically-effective oral dose of an immunotoxin according to the invention is in the range from about 0.05 mg/kg body weight to about 50 mg/kg body weight per day. A preferred effective dose is in the range from about 0.05 mg/kg body weight to about 5 mg/kg body weight per day.

Immunotoxins according to the present invention may be administered systemically, rather than topically, by injection intramuscularly, subcutaneously, intrathecally or intraperitoneally or into vascular spaces, particularly into the joints, *e.g.*, intraarticular injection at a dosage of greater than about 1 µg/cc joint fluid/day. The dose will be dependent upon the properties of the specific immunotoxin employed, *e.g.,* its activity and biological half-life, the concentration of immunotoxin in the formulation, the site and rate of dosage, the clinical tolerance of the patient involved, the disease afflicting the patient and the like, as is well within the skill of the physician.

The immunotoxins that are fusion proteins of the present invention may be administered in solution. The pH of the solution should be in the range of pH 5 to 9.5, preferably pH 6.5 to 7.5. The immunotoxin or derivatives thereof should be in a solution having a suitable pharmaceutically-acceptable buffer such as phosphate, Tris(hydroxymethyl)aminomethane-HCl or citrate and the like. Buffer concentrations should be in the range of 1 to 100 mM. The immunotoxin solution may also contain a salt, such as sodium chloride or potassium chloride in a concentration of 50 to 150 mM. An effective amount of a stabilizing agent such as an albumin, a globulin, a gelatin, a protamine or a salt of protamine may also be included, and may be added to a solution containing immunotoxin or to the composition from which the solution is prepared.

Systemic administration of immunotoxin may be made daily and is generally by intramuscular injection, although intravascular infusion is acceptable. Administration may also be intranasal or by other nonparenteral routes. Immunotoxins that are fusion proteins of the present invention may also be administered via microspheres, liposomes or other microparticulate delivery systems placed in certain tissues including blood. Topical preparations are applied daily directly to the skin or mucosa and are then preferably occluded, i.e., protected by overlaying a bandage, polyolefin film or other barrier impermeable to the topical preparation.

The following Examples are illustrative of practice of the invention but are not to be construed as limiting the invention. The present application is broadly organized as follows. The first portion of the application broadly teaches the preparation, expression and properties of an exemplary RIP, gelonin. A second portion of the application teaches the preparation of human-engineered antibodies. A third portion of the application teaches the construction and properties of immunoconjugates, comprising an RIP and an antibody or fragment thereof comprising an antigen-binding portion. A forth portion of the application relates to the preparation and properties of immunofusion proteins comprising an RIP and an antibody or fragment thereof comprising an antigen-binding portion. A fifth portion of the application teaches the preparation and properties of the RIP Barley ribosome-inactivating protein and a final aspect of the invention provides the preparation and properties of the RIP momordin.

Specifically, Example 1 relates to the preparation of the RIP gelonin. Construction of expression vector, comprising the gelonin gene, including expression and purification of gelonin, is taught in Example 2. The assembly of gelonin genes with cysteine residues available for conjugation is taught in Example 3 and Example 4 provides results of a reticulocyte lysate assay performed on gelonin.

Example 5 teaches the construction of human-engineered antibodies for use in immunotoxins of the invention and Example 6 demonstrates transfection of he3 genes, expression of those genes, and purification of the products thereof.

Example 7 next teaches the preparation of gelonin immunoconjugates. The procedures and results of whole cell kill assays are next presented in Example 8. Various properties of gelonin immunoconjugates are taught in Example 9 and Examples 10 and 11 teach the pharmacokinetics of two types of immunoconjugates. Examples 12 and 13 teach the immunogenicity of immunoconjugates of the invention and the *in vivo* efficacy of those immunoconjugates, respectively.

The construction of genes encoding gelonin immunofusions is taught in Examples 14, 15, 16, 17 and 18. Example 19 teaches alternative cathepsin cleavable linkers for use in the immunofusions of the invention. The expression and purification of various genes encoding immunoconjugates are presented in Example 20 and their activity properties are presented in Example 21.

The construction of genes encoding the RIP, BRIP, and its expression and properties are taught in Examples 22, 23, and 24.

Finally, construction of genes encoding momordin and properties of momordin on expression are taught in Example 25.

### Example 1

### Preparation Of Gelonin

The cloning of the gelonin gene according to the present invention obviates the requirement of purifying the RIP gene product from its relatively scarce natural source, *G. multiflorum* seeds. Cloning also allows development of gelonin analogs which may be conjugated to antibodies without prior chemical derivatization and also allows development of gelonin gene fusion products.

### A. Preparation Of RNA From G. Multiflorum Seeds

Total RNA was prepared from *Gelonium* seeds (Dr. Michael Rosenblum, M.D. Anderson Cancer Center, Houston, Texas) by a modification of the procedure for preparation of plant RNA described in Ausubel *et al., eds., Current Protocols in Molecular Biology,* Wiley & Sons, 1989. Briefly, 4.0 grams of seeds were ground to a fine powder in a pre-cooled (-70°C) mortar and pestle with liquid N₂. The powder was added to 25 ml Grinding buffer (0.18M Tris, 0.09M LiCl, 4.5mM EDTA, 1% SDS, pH 8.2) along with 8.5 ml of phenol equilibrated with TLE (0.2M Tris, 0.1M LiCl, 5mM EDTA pH8.2). The mixture was homogenized using a Polytron PT-1035 (#5 setting). 8.5 ml of chloroform was added, mixed and incubated at 50°C for 20 minutes. The mixture was centrifuged at 3000 g for 20 minutes in a rotor precooled to 4°C and the aqueous phase was transferred to a new tube. 8.5 ml of phenol was added followed by 8.5 ml of chloroform and the mixture was recentrifuged. This extraction was repeated 3 times. The RNA in the aqueous phase was then precipitated by adding 1/3 volume 8M LiCl, and incubated at 4°C for 16 hours. Next, the RNA was pelleted by centrifugation for 20 minutes at 4°C. The pellet was washed with 5 ml of 2M LiCl, recentrifuged and resuspended in 2 ml of water. The RNA was precipitated by addition of NaOAc to 0.3M and 2 volumes of ethanol. The RNA was stored in 70% ethanol at -70°C.

### B. cDNA Preparation

cDNA was prepared from total *Gelonium* RNA by two methods. The first method involved making a cDNA library in the bacterial expression plasmid pcDNAII using the Librarian II cDNA Library Construction System kit (Invitrogen). Approximately 5 µg of total RNA was converted to first strand cDNA with a 1:1 mixture of random primers and oligo-dT. Second strand synthesis with DNA polymerase I was performed as described by the system manufacturer. Double stranded cDNA was ligated to *Bst*Xl linkers and size fractionated. Pieces larger than about 500 bp were ligated into the expression vector provided in the kit. Individual vectors were introduced into *E. coli* either by transformation into high-efficiency competent cells or by electroporation into electrocompetent cells. Electroporation was performed with a BTX100 unit (BTX, San Diego, CA) in 0.56µ Flatpack cells as recommended by BTX based on the method of Dower *et al*., *Nucleic Acids Res., 16*:6127-6145 (1988), at a voltage amplitude of 850 V and a pulse length of 5 mS. The resulting library consisted of approximately 150,000 colonies.

The second method involved generating cDNA using the RNA-PCR kit sold by Perkin-Elmer-Cetus. About 100 ng of total Gelonium RNA was used as template for cDNA synthesis.

### C. Determination Of The Gelonin Protein Sequence

The partial sequence of the native gelonin protein was determined by direct amino acid sequence analysis using automated Edman degradation as recommended by the manufacturer using an Applied Biosystems model 470A protein sequencer. Proteolytic peptide fragments of gelonin (isolated from the same batch of seeds as the total RNA) were sequenced.

### D. Cloning Of The Gelonin Gene

Three overlapping gelonin cDNA fragments were cloned and a composite gelonin gene was assembled from the three fragments.

### 1. Cloning Of The Fragment Encoding The Middle Amino Acids Of Gelonin In Vector pING3823

Degenerate DNA primers based on the gelonin partial amino acid sequences were used to PCR-amplify segments of the cDNA generated with Perkin-Elmer-Cetus kit. Six primers were designed based on regions of the gelonin amino acid sequence where degeneracy of the primers could be minimized. Appropriate pairs of primers were tested for amplification of a gelonin gene fragment. Products of the expected DNA size were identified as ethidium bromide-stained DNA bands on agarose gels that DNA was treated with T4 DNA polymerase and then purified from an agarose gel. Only the primer pair consisting of primers designated gelo-7 and gelo-5 yielded a relatively pure product of the expected size. The sequences of degenerate primers gelo-7 and gelo-5 are set out below using IUPAC nucleotide symbols. Primer gelo-7 corresponds to amino acids 87-97 of gelonin while primer gelo-5 corresponds to amino acids 226-236. The blunt-ended DNA fragment (corresponding to amino acids 87 to 236 of gelonin) generated with primers gelo-7 and gelo-5 was cloned into pUC18 (BRL, Gaithersburg, Maryland). The DNA sequence of the insert was determined, and the deduced amino acid sequence based on the resulting DNA sequence matched the experimentally determined gelonin amino acid sequence. The clone containing this gelonin segment was denoted pING3726.

The insert of clone pING3726 was labeled with ³²P and used as a probe to screen the 150,000-member Gelonium cDNA library. Only one clone hybridized to the library plated in duplicate. This clone was purified from the library and its DNA sequence was determined. The clone contains a fragment encoding 185 of the 270 amino acids of gelonin (residues 25-209) and is denoted pING3823.

### 2. Cloning Of The Fragment Encoding The N-Terminal Amino Acids Of Gelonin

Based on the sequence determined for the gelonin gene segment in pING3726, exact oligonucleotide primers were designed as PCR amplification primers to be used in conjunction with a degenerate primer to amplify a 5' gelonin gene fragment and with a nonspecific primer to amplify a 3' gelonin gene fragment. cDNA generated using the Perkin-Elmer-Cetus RNA-PCR kit was amplified.

To amplify the 5'-end of the gelonin gene, PCR amplification with a degenerate primer gelo-1 and an exact primer gelo-10 was performed. The sequences of the primers are set out below. Primer gelo-1 corresponds to amino acids 1-11 of the gelonin gene while primer gelo-10 corresponds to amino acids 126-133. The product from the reaction was reamplified with gelo-1 (SEQ ID NO: 16) and gelo-11 (an exact primer comprising sequences encoding amino acids 119-125 of gelonin) to confer specificity to the reaction product. The sequence of primer gelo-11 is listed below. Hybridization with an internal probe confirmed that the desired specific gelonin DNA fragment was amplified. That fragment was cloned into pUC18 and the vector generated was designated pING3727. The fragment was sequenced, revealing that the region of the fragment (the first 27 nucleotides) corresponding to part of the degenerate primer gelo-1 could not be translated to yield the amino acid sequence upon which primer gelo-1 was originally based. This was not unexpected considering the degeneracy of the primer. The fragment was reamplified from the Gelonium cDNA with exact primers gelo-11 (SEQ ID NO: 18) and gelo-5' (which extends upstream of the 5' end of the gelonin gene in addition to encoding the first 16 amino acids of gelonin). The sequence of primer gelo-5' is set out below. The resulting DNA fragment encodes the first 125 amino acids of gelonin. While the majority of the sequence is identical to the natural gelonin gene, the first 32 nucleotides of the DNA fragment may be different. For the purposes of this application this N-terminal fragment is referred to as fragment GEL1-125.

### 3. Cloning Of The Fragment Encoding The C-Terminal Amino Acids Of Gelonin

To amplify the 3'-end of the gelonin gene as well as 3' untranslated sequences, PCR amplification with exact primers gelo-9 and XE-dT was performed. The sequence of each of the primers is set out below. Primer gelo-9 corresponds to amino acids 107-113 of gelonin. Primer XE-dT consists of a 3' oligo-dT portion and a 5' portion containing the restriction sites *Hind*III and *Xho*I, and will prime any poly A-containing cDNA. The reaction product was reamplified with exact primers gelo-8 and XE. The sequences of primers gelo-8 and XE are set out below. Primer gelo-8 consists of sequences encoding amino acids 115-120 of gelonin while the primer XE corresponds to the 5' portion of the XE-dT primer which contains *Hind*III and *Xho*I restriction sites. Hybridization with internal probes confirmed that the desired gelonin gene fragment was amplified. That fragment was then cloned into *pUC*18 by two different methods. First, it was cloned as a blunt-ended fragment into the *Sma*I site of *pUC*18 (the resulting vector was designated pING3728) and, second, it was cloned as an *Eco*RI to *Hind*III fragment into pUC18 (this vector was designated pING3729). Both vector inserts were sequenced. The insert of pING3728 encodes amino acids 114-270 of gelonin, while the insert of pING3729 encodes amino acids 184-270 of gelonin plus other 3' sequences.

### 4. Assembly Of The Overlapping Gelonin DNA Fragments Into A Composite Gelonin Gene

To reassemble the C-terminal two-thirds of the gelonin gene, vector pING3729 was cut with *Ssp*I (one *Ssp*I site is located within the vector and the second is located about 80 bp downstream of the termination codon of the insert in the vector) and an *Xho*I linker (8 bp, New England Biolabs) was ligated to the resulting free ends. The DNA was then cut with *Xho*I and *Eco*RI, and the 350 bp fragment generated, encoding amino acids 185-270 of gelonin, was isolated. This 350 bp fragment was ligated adjacent to a *Nco*I to *Eco*RI fragment from pING3823 encoding amino acids 37-185 of gelonin in a intermediate vector denoted pING3730, thus reassembling the terminal 87% of the gelonin gene (amino acids 37-270).

Next, fragment GEL1-125 was cut with *Sma*I and *Nco*I, resulting in a fragment encoding amino acids 1-36 of gelonin which was ligated along with the *Nco*I to *Xho*I fragment of pING3730 into the vector pIC100. [pIC100 is identical to pING1500 described in Better, *et al*., *Science, 240*:1041-1043 (1988), incorporated by reference herein], except that it lacks 37 bp upstream of the *pe1*B leader sequence. The 37 bp were eliminated by digestion of pING1500 with *Sph*I and *Eco*RI, treatment with T4 polymerase, and religation of the vector. This manipulation regenerated an *Eco*RI site in the vector while eliminating other undesirable restriction sites.] Before ligation, the vector pIC100 had previously been digested with *Sst*I, treated with T4 polymerase, and cut with *Xho*I. The ligation generated a new vector containing a complete gelonin gene which was designated plasmid pING3731 and deposited with The American Type Culture Collection, 12301 Parklawn Drive, Rockville, Maryland 20852 on October 2, 1991 as Accession No. 68721. The complete DNA sequence of the gelonin gene is set out in SEQ ID NO: 11.

### Example 2

### A. Construction Of Expression Vectors Containing The Gelonin Gene

A first *E. coli* expression vector was constructed containing the gelonin gene linked to the *Erwinia carotovora pel*B leader sequence, and to the *Salmonella typhimurium ara*B promoter. A basic vector containing the araB promoter is described in co-owned U.S. Patent No. 5,028,530 issued July 2, 1991 which is incorporated by reference herein. The vector containing the *ara*B promoter was cut with *Eco*RI and *Xho*I. Two DNA fragments were then ligated in tandem immediately downstream of the promoter. The fragment ligated adjacent to the promoter was a 131 bp fragment derived from *Sst*I digestion, T4 polymerase treatment and digestion with *Eco*RI of the pIC100 vector which includes the leader sequence of the *E. carotovora pel*B gene. The translated leader sequence is a signal for secretion of the respective protein through the cytoplasmic membrane. The fragment ligated downstream of the leader sequence was a *Sma*I to *Xho*I fragment from pING3731 which contains the complete gelonin gene. Thus, the expression vector contains the gelonin gene linked to the *pel*B leader sequence and the *ara*B promoter. This plasmid is designated pING3733.

A second expression vector may be constructed that is identical to the first except that the gelonin gene sequences encoding the nineteen C-terminal amino acids of gelonin are not included. The cDNA sequence of the gelonin gene predicted a 19 residue C-terminal segment that was not detected in any peptide fragments generated for determination of the gelonin amino acid sequence. These 19 amino acids may represent a peptide segment that is cleaved from the mature toxin post-translationally, *i.e.* that is not present in the native protein. A similar C-terminal amino acid segment was identified in the plant toxin α-trichosanthin [Chow *et al., J. Biol. Chem., 265*:8670-8674 (1990)]. Therefore, the expression product without the C-terminal fragment is of interest.

For construction of a gelonin expression vector without the 19 C-terminal amino acids of gelonin, PCR was used to amplify and alter the 3'-end of the gene. pING3728 was amplified with primers gelo-14 and gelo-9 (SEQ ID NO: 20). The sequence of primer gelo-14 is set out below. Primer gelo-14, which corresponds to gelonin amino acids 245-256, introduces a termination codon (underlined in the primer sequence) in the gelonin gene sequence which stops transcription of the gene before the sequences encoding the terminal 19 amino acids of the gelonin and also introduces a *Xho*I site immediately downstream of the termination codon. The PCR product was cut with *Xho*I and *Eco*RI, and the resulting 208 bp fragment encoding amino acids 185-251 of gelonin was purified from an agarose gel. This fragment was ligated adjacent to the *Nco*I to *Eco*RI fragment from pING3823 encoding amino acids 37-185 of gelonin to generate plasmid pING3732. A final expression vector, pING3734, containing a gelonin gene with an altered 3'-end was generated by substituting an *Nco*I to *Xho*I fragment encoding amino acids 37-251 of gelonin from pING3732 into pING3733.

### B. Identification Of The Native Gelonin 5'-End

Inverse PCR was used to identify a cDNA clone encoding the 5'-end of the mature gelonin gene. 5 µg of total *G. multiflorum* RNA was converted to cDNA using the Superscript Plasmid System (BRL, Gaithersburg, Maryland) with Gelo-11 (SEQ ID NO: 18) as a primer. Gelonin cDNA was self-ligated to generate covalent circular DNA and the ligated DNA was amplified by PCR with oligonucleotides Gelo-9 (SEQ ID NO: 20) and Gelo-16. The sequence of primer Gelo-16 is set out below. The PCR product was size-fractionated on an agarose gel and DNAs larger than 300 bp were cloned into *Sma*I cut pUC18. Several clones were sequenced with the primer Gelo-18, the sequence of which is set out below. A clone identified as having the largest gelonin-specific insert was designated pING3826. The DNA sequence of pING3826 included the first 32 nucleotides of the natural, mature gelonin gene not necessarily present in gelonin expression plasmids pING3733 and pING3734. The complete DNA sequence of the natural gelonin gene is set out in SEQ ID NO: 11.

### C. Construction Of Expression Vectors Containing A Gelonin Gene With A Natural 5' End

Derivatives of expression vectors pING3733 and pING3734 (described above) containing a gelonin gene with the natural 5' sequence were generated as follows. The 5'-end of gelonin was amplified from pING3826 with the PCR primers Gelo-16 (SEQ ID NO: 24) and Gelo-17, the sequence of which is set out below. The 285 bp PCR product was treated with T4 polymerase and cut with *Nco*I. The resulting 100 bp 5'-end DNA fragment was isolated from an agarose gel and ligated adjacent to the 120 bp *pelB* leader fragment from p1C100 (cut with *Sst*I, treated with T4 polymerase and cut with *Pst*I) into either pING3733 or pING3734 digested with *Pst*I and *Nco*I. The resulting plasmids pING3824 and pING3825 contain the entire native gelonin gene and the native gelonin gene minus the nineteen amino acid carboxyl extension, respectively, linked to the *pelB* leader and under the transcriptional control of the *ara*B promoter. The gene construct without the nineteen amino acid carboxyl extension in both pING3734 and pING3825 encodes a protein product referred to in this application as "recombinant gelonin".

### D. Purification Of Recombinant Gelonin

Recombinant gelonin was purified by the following procedure: *E. coli* fermentation broth was concentrated and buffer-exchanged to 10 mM sodium phosphate at pH 7.0 by using an S10Y10 cartridge over a DC10 unit (Amicon) the concentrated and buffer-exchanged material was applied to a CM52 column (100 g, 5X10 cm). The column was washed with 1 L of starting buffer and eluted with a 0 to 300 mM NaCl gradient in starting buffer (750 ml total volume). The pure gelonin containing fractions were pooled (elution was from 100-250 mM NaCl), concentrated over an Amicon YM10 membrane, equilibrated with 10 mM sodium phosphate buffer, pH 7.0, and stored frozen at -20°C. A further purification step was attempted using Blue Toyopearl chromatography. However, this procedure did not result in an increased purity of material and resulted in an approximate 50% loss of the starting material.

### Example 3

### Assembly Of Gelonin Genes With Cysteine Residues Available For Conjugation

The wild-type gelonin protein has two cysteine residues at positions 44 and 50 which are linked by an endogenous disulfide bond. The protein contains no free cysteine residue directly available for conjugation to antibodies or other proteins. Analogs of gelonin which contain a free cysteine residue available for conjugation were generated by three different approaches. In one approach, various residues along the primary sequence of the gelonin were replaced with a cysteine residue, creating a series of analogs which contain an odd number of cysteine residues. In another approach, one of the two endogenous cysteines was replaced by alanine, creating a molecule which lacks an intrachain disulfide bond but contains a single, unpaired cysteine. In yet another approach both endogenous cysteines were replaced by alanines and a third non-cysteine residue was replaced by a cysteine, creating an analog with a single, unpaired cysteine.

Fifteen analogs of gelonin were constructed. Ten non-cysteine residues of gelonin were targeted for substitution with a cysteine residue. Comparison of the amino acid sequence of gelonin to the natural amino acid sequence and tertiary structure of the ricin A-chain (see FIG. 1) suggested that these positions would be at the surface of the molecule and available for conjugation. Each of the ten gelonin analogs include a cysteine substituted in place of one of the following residues: lysine₁₀, asparagine₆₀, isoleucine₁₀₃, aspartic acid₁₄₆, arginine₁₈₄, serine₂₁₅, asparagine₂₃₉, lysine₂₄₄, aspartic acid₂₄₇, and lysine₂₄₈, and the analogs have respectively been designated Gel_{C10}, Gel_{C60}, Gel_{C103}, Gel_{C146}, Gel_{C184}, Gel_{C215}, Gel_{C239}, Gel_{C244}, Gel_{C247}, and Gel_{C248}.

Two analogs of gelonin were constructed in which one of the native gelonin cysteines that participates in an endogenous disulfide bond was replaced with a non-cysteine residue. Specifically, the cysteine at position 50 was replaced with an alanine residue, creating a gelonin analog (designated Gel_{A50(C44)}, shown in SEQ ID NO: 99) which has a cysteine available for disulfide bonding at position 44. The Gel_{A50(C44)} analog has been referred to previously as Gel_{C44} (*see*, *e.g*., co-owned, co-pending U.S. Patent Application Serial No. 07/988,430, incorporated by reference herein). Conversely, the cysteine at position 44 was replaced with an alanine residue, resulting in an analog (designated Gel_{A44(C50)}, shown in SEQ ID NO: 100) which has a cysteine available for disulfide bonding at position 50. The Gel_{A44(C50)} analog has been referred to previously as Gel_{C50} *(see, e.g.,* co-owned, co-pending U.S. Patent Application Serial No. 07/988,430, incorporated by reference herein). The combined series of the foregoing twelve analogs thus spans the entire length of the mature gelonin protein.

Another gelonin analog (Gel_{A44A50} SEQ ID NO: 101) was constructed in which both native gelonin cysteines were replaced with alanines. The Gel_{A44A50} analog has been referred to previously as Gel_{C44AC50A} (*see, e.g.*, co-owned, co-pending U.S. Patent Application Serial No. 07/988,430, incorporated by reference herein). Two additional analogs were constructed which have alanine residues substituted in place of both native cysteines and have either a cysteine residue substituted in place of the native lysine at position 10 (Gel_{C10A44A50}, shown in SEQ ID NO: 110) or a cysteine residue substituted in place of the native aspartate at position 247 (Gel_{C247A44A50}, shown in SEQ ID NO: 111).

The variants of recombinant gelonin were constructed by restriction fragment manipulation or by overlap extension PCR with synthetic oligonucleotides. The sequences of the primers used for PCR are set out below. In each mutagenic primer sequence, the nucleotides corresponding to the changed amino acid, either a cysteine or an alanine residue, are underlined.
Gelo-9 (SEQ ID NO: 20)
Gelo-11 (SEQ ID NO: 18)
Gelo-16 (SEQ ID NO: 25)
Gelo-17 (SEQ ID NO: 27)
Gelo-18 (SEQ ID NO: 26)

(1) Specifically, a cysteine was introduced at amino acid 247 of gelonin (which is normally occupied by an aspartic acid which corresponds to the cysteine at position 259 in the ricin A-chain) by PCR with mutagenic primers GeloC-3-2 and GeloC-4 in conjunction with primers *HIND*III-2 (a primer located in the vector portion of pING3734 or pING3825), Gelo-9 and Gelo-8. Template DNA (pING3734) was amplified with GeloC-3-2 and *HIND*III-2 and in a concurrent reaction with GeloC-4 and Gelo-9. The products of these reactions were mixed and amplified with the outside primers Gelo-8 and *HIND*III-2. The reaction product was cut with *Eco*RI and *Xho*I, purified, and was inserted into plasmid pING3825 in a three-piece ligation. The DNA sequence of the Gel_{C247} variant (SEQ ID NO: 102) was then verified. The plasmid containing the sequence encoding Gel_{C247} was designated pING3737 and was deposited with the American Type Culture Collection, 12301 Parklawn Drive, Rockville, MD 20852 on June 9, 1992 as ATCC Accession No. 69009.
(2-3) In the same manner, a cysteine residue was introduced in place of the amino acid at position 248 (a lysine) of gelonin with the mutagenic oligonucleotides GeloC-1 and GeloC-2 to generate analog Gel_{C248} (SEQ ID NO: 103) in plasmid pING3741, and a cysteine residue was introduced at amino acid position 239 (normally occupied by a lysine) with primers GeloC-9 and GeloC-10 to generate analog Gel₂₃₉ (SEQ ID NO: 104) in plasmid pING3744.
(4) Also in the same manner, a cysteine residue was introduced at amino acid 244 (a lysine) of gelonin with mutagenic primers GeloC-5 and GeloC-6 to generate analog Gel_{C244} (SEQ ID NO: 105) in a plasmid designated pING3736. This variant was prepared by PCR using plasmid pING3734 as template DNA rather than pING3825. It therefore encodes the same N-terminal gelonin amino acid sequence as plasmids pING3737, pING3741, and pING3744, but includes the PCR primer-derived 5'-end 32 nucleotides instead of the native gelonin 5'-end nucleotides.
(5) A cysteine residue was introduced in place of the amino acid (normally occupied by a lysine) at position 10 of gelonin by a similar procedure. A cysteine was introduced with mutagenic primers GeloC-13 and GeloC-14 by amplifying pING3824 with araB2 (a vector primer) and GeloC-14, and in a separate reaction, with GeloC-13 and Gelo-11. These reaction products were mixed and amplified with the outside primers *ara*B2 and Gelo-11. The PCR product was cut with *Pst*I and *Nco*I, purified, and cloned back into pING3825 to generate analog Gel_{C10} (SEQ ID NO: 106) in the plasmid designated pING3746 and deposited with the American Type Culture Collection, 12301 Parklawn Drive, Rockville, MD 20852 on June 9, 1992 as ATCC Accession No. 69008.
(6) The asparagine at position 60 of gelonin was replaced with a cysteine residue using two mutagenic oligos, GeloC-15 and GeloC-16, in conjunction with oligos *ara*B2 and Gelo-11 in the same manner as for the Gel_{C10} variant. The plasmid encoding the Gel_{C60} (SEQ ID NO: 107) analog was designated pING3749.
(7) A cysteine was introduced at amino acid 103 (an isoleucine) by PCR with mutagenic primers GeloC-20 and GeloC-21 in conjunction with primers araB2 and *HIND*III-2. Template DNA (pING3733) was amplified with GeloC-21 and *ara*B2 and separately with GeloC-20 and *HIND*III-2. The products of these reactions were mixed and amplified with the outside primers *ara*B2 and *HIND*III-2. The reaction product was cut with *Nco*I and *Bcl*I, purified, and inserted into pING3825 digested with *Nco*I and *Bcl*I. The oligonucleotides used to place a cysteine at residue 103 also introduced an *Afl*III restriction site which was verified in the cloned gene. The plasmid containing the Gel_{C103} (SEQ ID NO: 108) analog was designated pING3760.
(8) A cysteine was introduced at position 146 (an aspartic acid) by a similar strategy. Template DNA (pING3733) was amplified with mutagenic primer GeloC-22 and Gelo-14 and separately with mutagenic primer GeloC-23 and Gelo-19. The products of these reactions were mixed, and amplified with Gelo-19 and Gelo-14. The reaction product was cut with *Bgl*II and *Eco*RI, and can be inserted into pING3825 in a three-piece ligation. The oligonucleotides used to place a cysteine at residue 146 also introduced a *Nde*I restriction site which can be verified in the cloned gene.
(9) To introduce a cysteine at position 184 (normally occupied by an arginine) of gelonin, template DNA (pING3733) was amplified with mutagenic primer GeloC-25 and *ara*B-2 and separately with mutagenic primer GeloC-24 and *HIND*III-2. The products of these reactions were mixed, and amplified with *ara*B2 and Gelo-14. The reaction product was cut with *Nco*I and *Bcl*I, and inserted into pING3825 previously digested with *Nco*I and *Bcl*I. The oligonucleotides used to place a cysteine at residue 184 also introduced an NsiI restriction site which was verified in the cloned gene. The plasmid containing the sequence encoding the Gel_{C184} (SEQ ID NO: 109) variant was designated pING3761.
(10) A cysteine may be introduced at position 215 (a serine) by a similar strategy. Template DNA (pING3733) was amplified with mutagenic primer GeloC-27 and araB2 and separately with mutagenic primer GeloC-26 and *HIND*III-2. The products of these reactions were mixed, and amplified with *ara*B2 and *HIND*III-2. The reaction product was cut with *Eco*RI and *Bcl*I, and may be inserted into pING3825 in a three-piece ligation.
(11) Another gelonin variant with a free cysteine residue was generated by replacing one of the two naturally occurring gelonin cysteine residues, the cysteine a position 50, with an alanine. Plasmid pING3824 was amplified with primers GeloC-17 and Gelo-11, and concurrently in a separate reaction with primers GeloC-19 and *ara*B2. The reaction products were mixed and amplified with *ara*B2 and Gelo-11. This product was cut with *Nco*I and *Bgl*II, and cloned back into the vector portion of pING3825 to generate pING3747 (ATCC 69101). This analog was designated Gel_{A50(C44)} and it contains a cysteine available for disulfide bonding at amino acid position 44. Non-cysteine residues, other than alanine, which do not disrupt the activity of gelonin, also may be inserted at position 50 in natural gelonin in order to generate a gelonin analog with a single cysteine at position 44.
(12) A gelonin variant in which the natural cysteine at position 44 was changed to alanine was constructed by amplifying pING3733 using the mutagenic oligonucleotides GeloC-28 and GeloC-29 in conjunction with primers *ara*B2 and *HIND*III-2. The amplified DNA was cut with *Nco*I and *Bgl*II and cloned into a gelonin vector, generating pING3756. That variant generated was designated Gel_{A44(C50)}. Non-cysteine residues, other than alanine, which do not disrupt gelonin activity, also may be inserted at position 44 in order to generate a gelonin analog with a single cysteine at position 50.
(13) A gelonin variant in which both the cysteine at position 44 and the cysteine at position 50 of gelonin were changed to alanine residues was constructed by overlap PCR of pING3824 using the mutagenic oligonucleotides GeloC-17 and GeloC-18 in conjunction with primers *ara*B2 and Gelo-11. This analog, like the native gelonin protein, has no cysteine residues available for conjugation. The plasmid encoding the analog was designated pING3750. The analog generated was designated Gel_{A44A50} (SEQ ID NO: 101). Non-cysteine residues, other than alanine, which do not disrupt gelonin activity, also may be substituted at both positions 44 and 50 in order to generate a gelonin analog with no cysteine residues.
(14) The triple mutant Gelonin_{C247A44A50} (SEQ ID NO: 111) was constructed from the plasmids pING3824, pING3750 and pING3737. This variant contains an introduced cysteine at position 247 while both of the naturally occurring cysteine residues at positions 44 and 50 have been replaced with alanine. The analog is desirable because, in this analog, disulfide linkage to an antibody is only assured at a single cysteine residue. Plasmid pING3824 was cut with *Nco*I and *Xho*I and the vector fragment was purified in an agarose gel. pING3750 was cut with *Nco*I and *Eco*RI and pING3737 was cut with *Eco*RI and *Xho*I. The *Nco*I-*Eco*RI fragment encodes the alanines at positions 44 and 50 while the *Eco*RI-*Xho*I fragment encodes the cysteine at position 247. Each of these fragments was purified and ligated to the *Nco*I to *Xho*I vector fragment. The resulting plasmid is named pING3752.
(15) The triple mutant Gelonin_{C10A44A50} (SEQ ID NO: 110) was also constructed by assembly from previously assembled plasmids. In this case, pING3746 was cut with *Pst*I and *Nco*I, while pING3750 was cut with *Nco*I and *Xho*I. Each of the insert fragments were purified by electrophoresis in an agarose gel, and the fragments were ligated into a *Pst*I and *Xho*I digested vector fragment. The resulting vector was designated pING3753. The Gel_{C10A44A50} analog has been referred to previously as Gel_{C10C44AC50A} *(see, e.g.*, co-owned, co-pending U.S. Patent Application Serial No. 07/988,430, incorporated by reference herein).

Each of the gelonin variants constructed was transformed into *E. coli* strain E104. Upon induction of bacterial cultures with arabinose, gelonin polypeptide could be detected in the culture supernatants with gelonin-specific antibodies. There were no significant differences detected in the expression levels of gelonin from plasmids pING3734 and pING3825, or in the levels from any of the gelonin variants. Each protein was produced in *E. coli* at levels of approximately 1 g/l.

### Example 4

### Reticulocyte Lysate Assay

The ability of gelonin and recombinant gelonin analogs to inhibit protein synthesis *in vitro* was tested using a reticulocyte lysate assay (RLA) described in Press *et al., Immunol. Letters, 14*:37-41 (1986). The assay measures the inhibition of protein synthesis in a cell-free system using endogenous globin mRNA from a rabbit red blood cell lysate. Decreased incorporation of tritiated leucine (³H-Leu) was measured as a function of toxin concentration. Serial log dilutions of standard toxin (the 30 kD form of ricin A-chain, abbreviated as RTA 30), native gelonin, recombinant gelonin (rGelonin or rGel) and gelonin analogs were tested over a range of 1 µg/ml to 1 pg/ml. Samples were tested in triplicate, prepared on ice, incubated for 30 minutes at 37°C, and then counted on an Inotec Trace 96 cascade ionization counter. By comparison with an uninhibited sample, the picomolar concentration of toxin (pM) which corresponds to 50% inhibition of protein synthesis (IC₅₀) was calculated. As is shown in Table 1 below, recombinant gelonin and most of its analogs exhibit activity in the RLA comparable to that of native gelonin. For some of the analogs (such as Gel_{C239}), RLA activity was diminished.

**Table 1**

| Toxin | IC₅₀(pM) |
|---|---|
| RTA 30 | 2.5 |
| Gelonin | 15 |
| rGelonin | 11 |
| Gel_{C10} | 60 |
| Gel_{A50(C44)} | 20 |
| Gel_{A44(C50)} | 47 |
| Gel_{C60} | 26 |
| Gel_{C239} | 955 |
| Gel_{C244} | 32 |
| Gel_{C247} | 12 |
| Gel_{C248} | 47 |
| Gel_{A44A50} | 16 |
| Gel_{C10A4450A} | 7 |
| Gel_{C247A44A50} | 20 |

### Example 5

### Human-Engineered Antibodies For Construction Of Immunotoxins

Antibodies for use in constructing immunotoxins according to the present invention may be humanized antibodies, such as he3 and fragments thereof which display increased content of human amino acids and a high affinity for human CD5 cell differentiation marker. he3 is a humanized form of a mouse H65 antibody (H65 is a preferred monoclonal antibody for use in preparing humanized antibodies according to the present invention and is produced by hybridoma cell line XMMLY-H65 (H65) deposited with the American Type Culture Collection in Rockville, Maryland (A.T.C.C.) and given the Accession No. HB9286).

Humanized antibodies for use in the present invention are prepared as disclosed herein using the humanized forms of the murine H65 antibody in which both low and moderate risk changes described below were made in both variable regions. Such humanized antibodies should have less immunogenicity and have therapeutic utility in the treatment of autoimmune diseases in humans. For example, because of their increased affinity over existing therapeutic monoclonal antibodies such as H65, he3 antibodies of the invention may be administered in lower doses than H65 anti-CD5 antibodies in order to obtain the same therapeutic effect.

Humanized antibodies, such as he3, are useful in reducing the immunogenicity of foreign antibodies and also results in increased potency when used as a portion of an immunoconjugate.

Construction of humanized antibody variable domains according to the present invention and for use as components of immunotoxins may be based on a method which includes the steps of: (1) identification of the amino acid residues of an antibody variable domain which may be modified without diminishing the native affinity of the domain for antigen while reducing its immunogenicity with respect to a heterologous species; and (2) the preparation of antibody variable domains having modifications at the identified residues which are useful for administration to heterologous species. The methods of the invention are based on a model of the antibody variable domain described herein which predicts the involvement of each amino acid in the structure of the domain.

Unlike other methods for humanization of antibodies, which advocate replacement of the entire classical antibody framework regions with those from a human antibody, the methods described herein introduce human residues into the variable domain of an antibody only in positions which are not critical for antigen-binding activity and which are likely to be exposed to immunogenicity-stimulating factors. The present methods are designed to retain sufficient natural internal structure of the variable domain so that the antigen-binding capacity of the modified domain is not diminished in comparison to the natural domain.

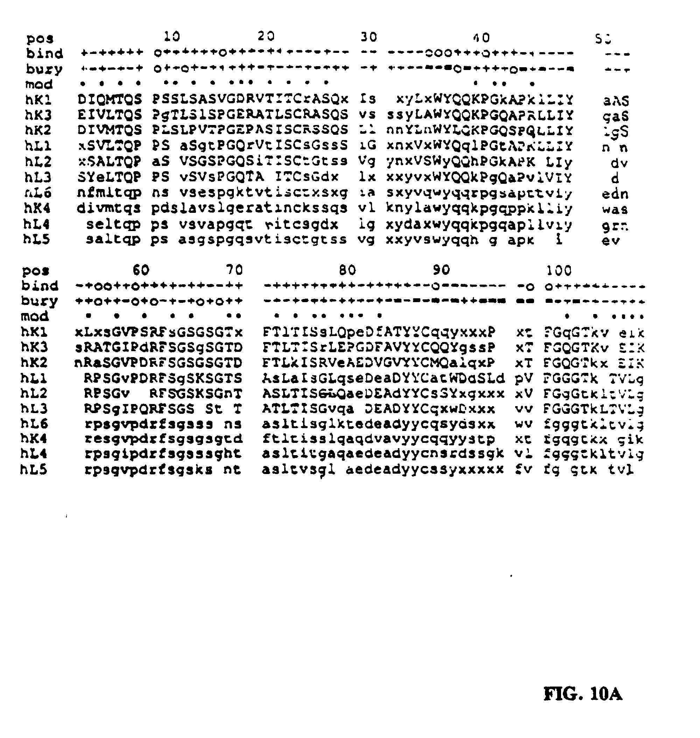

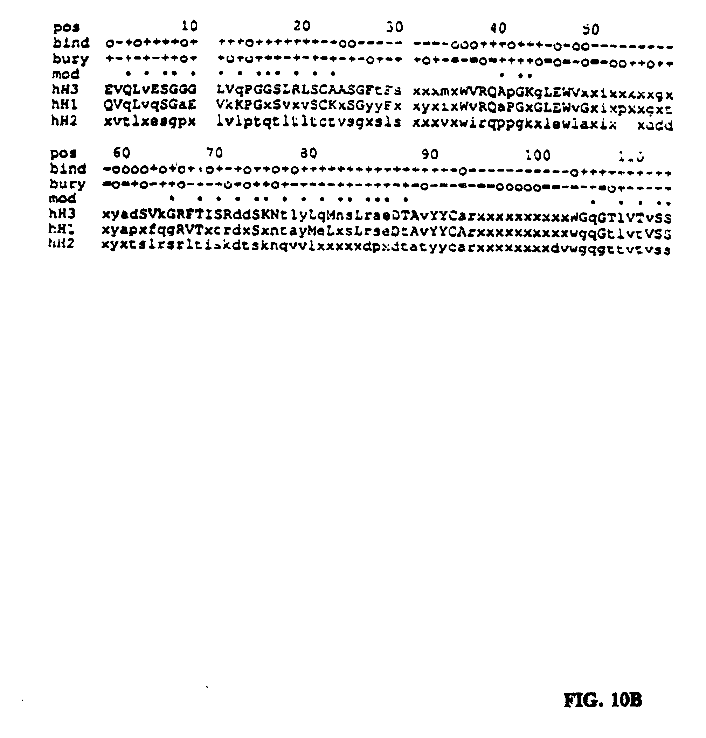

The human consensus sequences in which moderate risk residues are converted from mouse residues to human residues are represented in Figures 10A and 10B as lines labelled hK1 (*i.e.*, subgroup 1 of the human kappa chain) and hH3 (*i.e.*, subgroup 3 of the human heavy chain). Symbols in the figures for conservation and for risk in "bind" and "bury" lines are follows:
First Symbol in Pair (Ligand Binding)
   - +: Little or not direct influence on antigen-binding loops, low risk if substituted
   - °: Indirectly involved in antigen-binding loop structure, moderate risk if changed
   - -: Directly involved in antigen-binding loop conformation or antigen contact, great risk if modified
Second Symbol in Pair (Immunogenicity/Struture)
   - +: Highly accessible to solvent, high immunogenicity, low risk if substituted
   - °: Partially buried, moderate immunogenicity, moderate risk if altered
   - -: Completely buried in subunit's hydrophobic core, low immunogenicity, high risk if changed
   - =: Completely buried in interface between subunits, low immunogenicity, high risk if modified
Significance of Pairs
   - ++: Low risk
   Highly accessible to solvent and high immunogenicity, but little or no effect on specific antigen binding
   - °+, +°, °°: Moderate Risk
   Slight immunogenicity or indirect involvment with antigen binding
   - any - or =: High risk
   Buried within the subunit core/interface or strongly involved in antigen binding, but little immunogenic potential

In the line labelled "mod", a dot (.) represents a residue which may be mutated from "mouse" to "human" at moderate risk. There are 29 such moderate risk positions.

The mouse residue matches the human consensus residue more than 50% of the time at 131 positions (102 positions match 90%-100% and 29 positions match 50% to 90%). These positions were not changed.

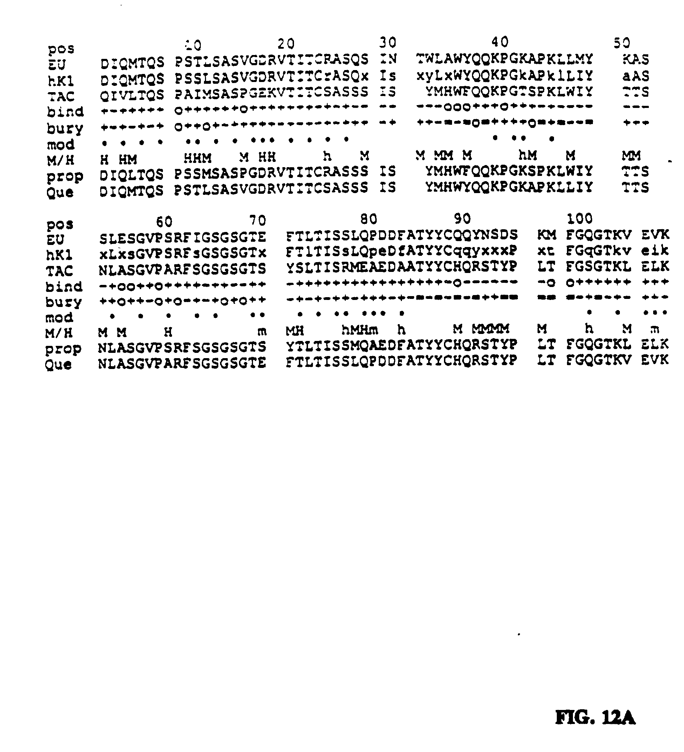

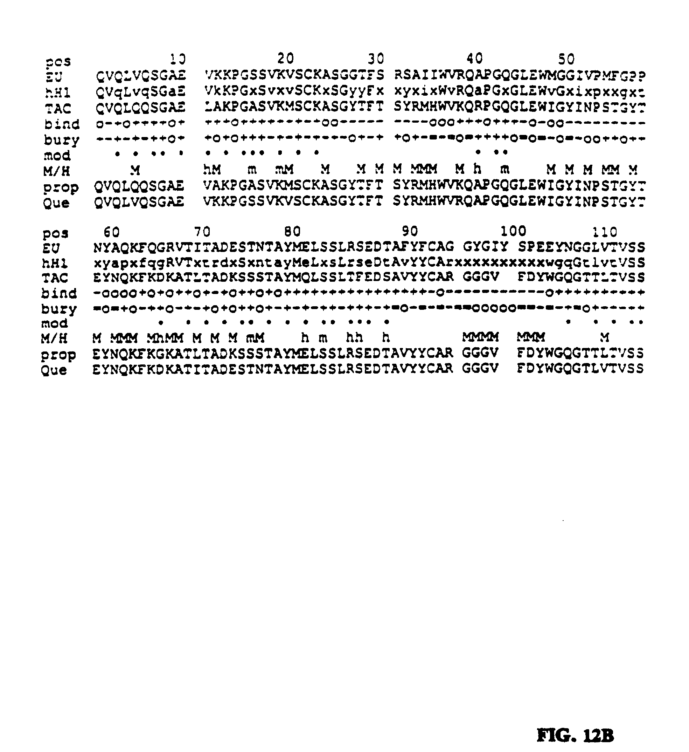

The lines labelled M/H in Figures 12A and 12B indicate the 91 positions which differed significantly between the mouse and human sequences (*i.e.*, where the human sequences have the mouse residue less than 50% of the time). Moderate risk positions, designated m in the M/H line, were kept "mouse"; whereas those designated H or h were changed to human. The 25 low risk positions which were already human-like or which were previously humanized (as described *supra* in Example 2) are designated " ^ " in the M/H line. Finally, the 54 high risk positions in which the mouse and human residues did not match are designated M and are kept "mouse".

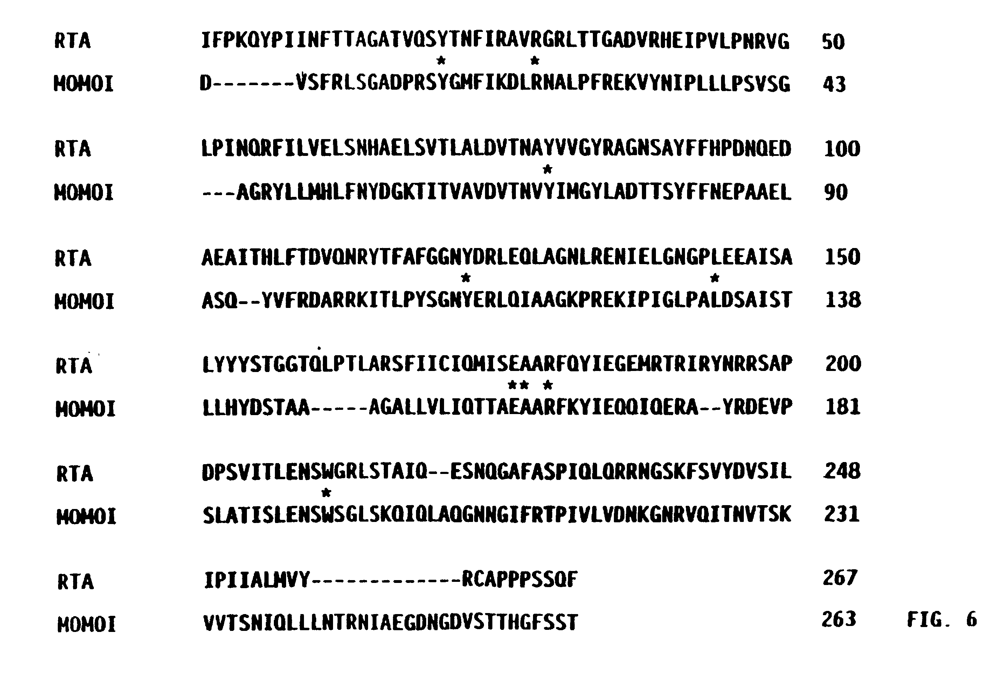

Fifteen differences occur at moderate risk positions at which the mouse and human sequences differ. At ten of those positions (designated "H" on the M/H lines of Figure 6) the mouse residue aligns with a human consensus amino acid which is highly conserved. Therefore, the mouse residue at that position is identified as one to be changed to the conserved human residue.

At moderate risk positions (designated "m") in which the mouse and the human sequences differ, the mouse residue aligns with a human consensus amino acid which is moderately conserved. However, since the mouse residue is found at that position in other actual sequences of human antibodies [See Kabat, *et al*., sequences of Proteins of Immunoglobulin Interest,Fourth Edition, U.S. Department of Health and Human Services, Public Health Service, National Institutes of Health (1987)] the positions are identified as ones to be kept "mouse." Although there are no such positions in this particular sequence, such positions may occur in other antibodies.

At four moderate risk positions (designated "h"), the mouse residue aligns with a human consensus amino acid which is moderately conserved but the mouse residue is not found at that position in an actual human antibody sequence in Kabat, *et al. Sequences of Proteins of Immunoglobulin Interest, supra.* Therefore, that position is identified as ones to be changed to "human."

At one moderate risk position (designated "m") in which the mouse and human sequences differ, the mouse residue aligns with a human consensus amino acid which is poorly conserved. Therefore, that position is identified as one to be kept "mouse."

### A. Assembly Of Moderate Risk Heavy Chain Expression Vectors

The humanized H65 heavy chain containing the moderate risk residues was assembled by the following strategy. The moderate-risk expression vector was assembled from intermediate vectors. The six oligonucleotide sequences (oligos), disclosed in Figure 12 and labelled HUH-G11, HUH-G12, HUH-G3, HUH-G4, HUH-G5, and HUH-G6 (the sequences of HUH-G11 and HUH-G12 are set out in SEQ ID Nos. 131 and 132 and HUH-G3, HUH-G4, HUH-G5, and HUH-G6 are set out in SEQ ID NOS: 137-140) were assembled by PCR. Oligonucleotides containing the synthetic humanized antibody gene were mixed in pairs (HUH-G11 + HUH-G12, HUH-G3 + HUH-G4, and HUH-G5 + HUH-G6) in a 100 µl reaction with 1 µg of each DNA and filled in as described above. A portion of each reaction product was mixed in pairs (HUH-G11, 12 + HUH-G3, 4; HUH-G3, 4 + HUH-G5, 6), 2.5 U Taq was added and samples were reincubated as described above. The V-J region was assembled by mixing equal amounts of the HUH-G11, 12, 3, 4 reaction product with the HUH-G3, 4, 5, 6 product, followed by PCR with 0.5 ug of primers H65G-2S and H65-G2 as described above. The reaction product was cut with *Sal*I and *Bst*EII and cloned into the expression vector, similar to that described for heavy chain in Robinson *et al., Hum. Antibod. Hybridomas 2*:84 (1991), generating pING4617. That plasmid was sequenced with Sequenase (USB, Cleveland), revealing that two residues were altered (a G-A at position 288 and a A-T at position 312, numbered from the beginning of the leader sequence). The correct variable region was restored by substitution of this region from pING4612, generating the expected V-region sequence in pING4619.

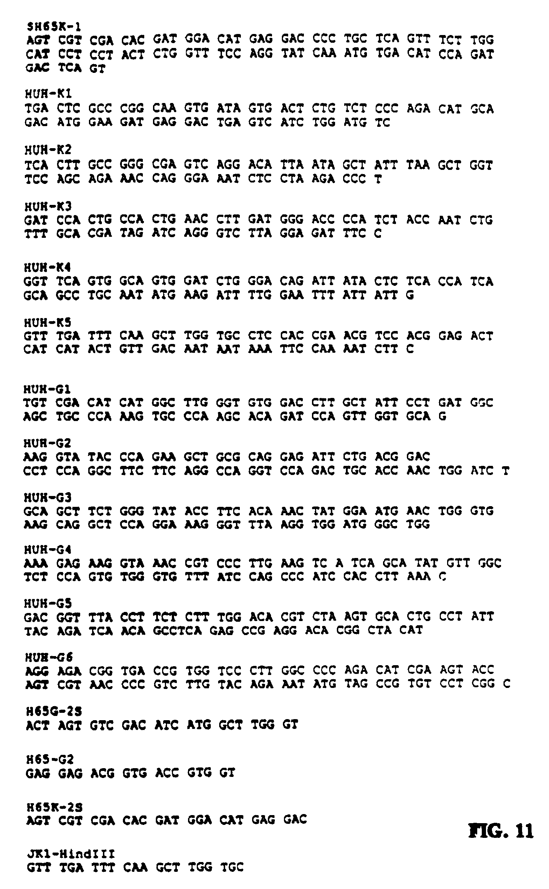

An intermediate vector containing the other moderate-risk changes was constructed by PCR assembly of the oligos HUH-G13, HUH-G14, HUH-G15, and HUH-G16 (Fig. 11 and SEQ ID Nos: 133-136). Oligos HUH-G13 + HUH-G14 and HUH-G15 + HUH-G16 were mixed and filled in with Vent polymerase (New England Biotabs) in a reaction containing 10 mM KCl, 20 mM TRIS pH 8.8, 10 mM (NH₄)₂SO₂, 2mM MgSO₄, 0.1% Triton X-100, 100 ng/ml BSA, 200 uM of each dNTP, and 2 units of Vent polymerase in a total volume of 100 µl. The reaction mix was incubated at 94°C for 1 minute, followed by 2 minutes at 50°C and 20 minutes at 72°C. The reaction products (40 µl) were mixed and amplified with the oligonucleotides H65-G13 and H65-G2 with Vent polymerase in the same reaction buffer and amplified for 25 cycles with denaturation at 94°C for 1 minute, annealing at 50°C for 2 minutes and polymerization at 72°C for 3 minutes. The reaction product was treated with T4 polymerase and then digested with *Acc*I. The 274 base pair (bp) fragment was purified on an agarose gel and ligated along with the 141 bp *Sal*I to *Acc*I fragment from pING4619 into pUC18 cut with *Sal*I and *Sma*I to generate pING4620. pING4620 contains the entire signal sequence, V-region, and J-region of the moderate-risk H65 heavy chain.

The final expression vector for the moderate-risk H65 heavy chain, pING4621, was assembled by cloning the *Sal*l to *Bst*EII fragment from pING4620 into the same expression vector described above.

### B. Assembly Of Moderate-Risk Light Chain Expression Vectors

The moderate-risk humanized V- and J-segments of the light chain were assembled from six oligonucleotides, $H65K-1 (SEQ ID NO: 117), HUH-K7 (SEQ ID NO: 119), HUH-K6 (SEQ ID NO: 118), HUH-K8 (SEQ ID NO: 120), HUH-K4 (SEQ ID NO: 121 and HUH-K5 (SEQ ID NO: 122). The oligonucleotides were amplified with PCR primers H65K-2S and JK1-*Hind*III. Oligonucleotides containing the synthetic humanized antibody gene were mixed in pairs ($H65-K1 + HUH-K7, HUH-K6 + HUH-K4 + HUH-K5) and incubated with Vent polymerase as described for the moderate-risk heavy chain. A portion of each reaction product (40 ul) was mixed in pairs ($H65H-K1/HUH-K7 + HUH-K6, 8; HUH-K6, 8 + HUH-K4, 5) and filled in as above. The light chain gene was then assembled by amplifying the full length gene with the PCR primers H65K-2S and JK1-*Hind*III with Vent polymerase for 25 cycles as outlined above. The assembled V/J region was cut with *Sal*I and *Hind*III, purified by electrophoresis on an agarose gel, and assembled into a light chain antibody expression vector, pING4630.

### Example 6

### Transfection Of he3 Genes And Purification Of Expression Products

### A. Stable Transfection Of Mouse Lymphoid Cells For The Production Of he3 Antibody

The cell line Sp2/0 (American Type Culture Collection Accession No. CRL1581) was grown in Dulbecco's Modified Eagle Medium plus 4.5 g/l glucose (DMEM, Gibco) plus 10% fetal bovine serum. Media were supplemented with glutamine/penicillin/streptomycin (Irvine Scientific, Irvine, California).

The electroporation method of Potter, H., *et al.*, *Proc. Natl. Acad. Sci*., *USA,* 81:7161 (1984) was used. After transfection, cells were allowed to recover in complete DMEM for 24-48 hours, and then seeded at 10,000 to 50,000 cells per well in 96-well culture plates in the presence of selective medium. Histidinol (Sigma) selection was at 1.71 µg/ml, and mycophenolic acid (Calbiochem) was at 6 µg/ml plus 0.25 mg/ml xanthine (Sigma). The electroporation technique gave a transfection frequency of 1-10 x 10⁻⁵ for the Sp2/0 cells.

The he3 light chain expression plasmid pING4630 was linearized by digestion with *Pvu*I restriction endonuclease and transfected into Sp2/0 cells, giving mycophenolic acid - resistant clones which were screened for light chain synthesis.

Four of the top-producing subclones, secreting 4.9-7.5 µg/ml were combined into two pools (2 clones/pool) and each pool was transfected with plasmid pING42621, containing the moderate-risk heavy chain. After selection with histidinol, the clones producing the most light plus heavy chain, Sp2/0-4630 and 4621 Clones C1705 and C1718, secreted antibody at approximately 15 and 22 µg/ul respectively in the presence of 10⁻⁷ M dexamethasone in an overgrown culture in a T25 flask. Clone C1718 was deposited with the American Type Culture Collection, 1230 Parklawn Drive, Rockville, Maryland, 20852 on December 1, 1992 as ATCC HB 11206. The best producer is a subclone of Clone C1718 which is produced by limiting dilution subcloning of Clone C1718.

### B. Purification Of he3 Antibody Secreted In Tissue Culture

Sp2/0-4630 + 4621 Clone C1705cells were grown in culture medium HB101 (Hana Biologies)+ 1% Fetal Bovine Serum, supplemented with 10 mM HEPES, 1x Glutamine-Pen-Strep (Irvine Scientific #9316). The spent medium was centrifuged at about 5,000 x g for 20 minutes. The antibody level was measured by ELISA. Approximately 200 ml of cell culture supernatant was loaded onto a 2 ml Protein A-column (Sigma Chemicals), equilibrated with PBS (buffer 0.15 M NaCl, 5 mM sodium phosphate, 1 mM potassium phosphate, buffer pH 7.2). The he3 antibody was eluted with a step pH gradient (pH 5.5, 4.5 and 2.5). A fraction containing he3 antibody (9% yield) but not bovine antibody, was neutralized with 1 M Tris pH 8.5, and then concentrated 10-fold by Centricon 30 (Amicon) diluted 10-fold with PBS, reconcentrated 10-fold by Centricon 30, diluted 10-fold with PBS, and finally reconcentrated 10-fold. The antibody was stored in 0.25 ml aliquots at -20° C.

### C. Affinity Measurements Of he3 IgG For CD5

The affinity of he3 IgG for CD5 was determined using Molt-4M cells, which express CD5 on their surface, and I¹²⁵-labeled chimeric H65 IgG in a competitive binding assay. Culture supernatants from Clone C1705 and C1718 and purified IgG from C1705 were used as the sources of he3 IgG.

For this assay, 20 µg of chimeric H65 IgG (cH65 IgG) was iodinated by exposure to 100 µl lactoperoxidase-glucose oxidase immobilized beads (Enzymobeads, BioRad), 100 µl of PBS, 1.0 mCi I¹²⁵ (Amersham, IMS30), 50 µl of 55 mM b-D-glucose for 45 minutes at 23°C. The reaction was quenched by the addition of 20 µl of 105 mM sodium metabisulfite and 120 mM potassium iodine followed by centrifugation for 1 minute to pellet the beads. ¹²⁵I-cH65 IgG was purified by gel filtration using 7 mls of sephadex G25, using PBS (137 mM NaCl, 1.47 mM KH₂PO₄, 8.1 mM Na₂HPO₄, 2.68 mM KCl at pH 7.2-7.4) plus 0.1% BSA. ¹²⁵I-cH65 IgG recovery and specific activity were determined by TCA precipitation.

Competitive binding was performed as follows: 100 µl of Molt-4M cells were washed two times in ice-cold DHB binding buffer (Dubellco's modified Eagle's medium (Gibco, 320-1965PJ), 1.0% BSA and 10 mM Hepes at pH 7.2. -7.4). Cells were resuspended in the same buffer, plated into 96 v-bottomed wells (Costar) at 3 x 10⁵ cells per well and pelleted at 4°C by centrifugation for 5 min at 1,000 rpm using a Beckman JS 4.2 rotor; 50 µl of 2X-concentrated 0.1 nM ¹²⁵I-cH65 IgG in DHB was then added to each well and competed with 50 µl of 2X - concentrated cH65 IgG or humanized antibody in DHB at final antibody concentrations from 100 nM to 0.0017 nM. Humanized antibody was obtained from culture supernatants of Sp2/0 clone C1718 which expresses he3 IgG. The concentration of the antibody in the supernatants was established by ELISA using a chimeric antibody as a standard. The concentration of the antibody in the purified preparation was determined by binding was allowed to proceed at 4°C for 5 hrs and was terminated by washing cells three times with 200 µl of DHB binding buffer by centrifugation for 5 min at 1,000 rpm. All buffers and operations were at 4°C. Radioactivity was determined by solubilizing cells in 100 µl of 1.0 M NaOH and counting in a Cobra II auto gamma counter (Packard). Data from binding experiments were analyzed by the weighted nonlinear least squares curve fitting program, MacLigand, a Macintosh version of the computer program "Ligand" from Munson, *Analyt. Biochem.*, 107:220 (1980). Objective statistical criteria (F, test, extra sum squares principle) were used to evaluate goodness of fit and for discriminating between models. Nonspecific binding was treated as a parameter subject to error and was fitted simultaneously with other parameters.

Figure 11, provides data showing relative binding of he3 and CH65 to CD5 on molt-4M cells in a competition binding assay. These results demonstrate that the moderate-risk changes made in he3 IgG result in an antibody with a higher affinity than the chimeric mouse-human form of this antibody (cH65) for its target, CD5.

### Example 7

### Preparation of Gelonin Immunoconjugates

Gelonin analogs of the invention were variously conjugated to murine (ATCC HB9286) and chimeric H65 (cH65) antibody, cH65 antibody domains (including cFab, cFab' and cF(ab')₂ fragments), and humanized antibodies and antibody domains, all of which are specifically reactive with the human T cell determinant CD5. H65 antibody was prepared and purified by methods described in U.S. Patent Application Serial No. 07/306,433, *supra* and International Publication No. WO 89/06968, *supra.* Chimeric H65 antibody was prepared according to methods similar to those described in Robinson *et al*., *Human Antibodies and Hybridomas, 2*:84-93 (1991), incorporated by reference herein. Chimeric H65 Fab, Fab', and F(ab')₂ proteins were prepared as described in Better, *et al*., *Proc. Nat. Acad. Sci. (USA), 90:* 457-461 (1993), incorporated by reference herein. Finally, he3 humanized antibodies were prepared according to the procedures described in U.S. Patent Application Serial No. 07/808,464, incorporated by reference herein.

### A. Conjugation To H65 Antibodies

To expose a reactive sulfhydryl, the unpaired cysteine residues of the gelonin analogs were first reduced by incubation with 0.1 to 2 mM DTT (30-60 minutes at room temperature), and then were desalted by size-exclusion chromatography.

Specifically, the Gel_{C248} analog (3.8 mg/ml) was treated with 2 mM DTT for 60 minutes in 0.1 M Naphosphate, 0.25 M NaCl, pH 7.5 buffer. The Gel_{C244} variant (7.6 mg/ml) was treated with 2 mM DTT for 30 minutes in 0.1 M Naphosphate, 0.25 M NaCl, pH 7.5 buffer. The Gel_{C247} analog (4 mg/ml) was treated with 2 mM DTT for 30 minutes in 0.1 M Naphosphate, 0.5 M NaCl, pH 7.5 buffer with 0.5 mM EDTA. The Gel_{C239} variant (3.2 mg/ml) was treated with 2 mM DTT for 30 minutes in 0.1 m Naphosphate, 0.5 M NaCl, pH 7.5 buffer with 0.5 mM EDTA. The Gel_{A50(C44)} analog (4.2 mg/ml) was treated with 0.1 mM DTT for 30 minutes in 0.1 M Naphosphate, 0.1 M NaCl, pH 7.5 buffer with 0.5 mM EDTA. Lastly, the Gel_{C10} variant (3.1 mg/ml) was treated with 1 mM DTT for 20 minutes in 0.1 M Naphosphate, 0.1 M NaCl, pH 7.5 buffer with 1 mM EDTA.

The presence of a free sulfhydryl was verified by reaction with DTNB and the average value obtained was 1.4 ± 0.65 SH/molecule. No free thiols were detected in the absence of reduction.

H65 antibody and chimeric H65 antibody were chemically modified with the hindered linker 5-methyl-2-iminothiolane (M2IT) at lysine residues to introduce a reactive sulfhydryl group as described in Goff *et al., Bioconjugate Chem*., *1*:381-386 (1990) and co-owned Carroll *et al*., U.S. Patent No. 5,093,475, incorporated by reference herein.

Specifically, for conjugation with Gel_{C248} and Gel_{C244}, murine H65 antibody at 4 mg/mL was derivitized with 18x M2IT and 2.5 mM DTNB in 25 mM TEOA, 150 mM NaCl, pH 8 buffer for 1 hour at 23°C. The reaction gave 1.9 linkers per antibody as determined by DTNB assay.

For conjugation with Gel_{C247} and Gel_{C239}, H65 antibody at 4.7 mg/mL was derivitized with 20x M2IT and 2.5 mM DTNB in 25 mM TEOA 150 mM NaCl, pH 8 buffer for 50 minutes at 23°C. The reaction gave 1.6 linkers per antibody as determined by DTNB assay.

Before reaction with Gel_{A50(C44)}, H65 antibody at 5.8 mg/mL was derivitized with 20x m2IT and 2.5 mM DTNB in 25 mM TEOA, 150 mM NaCl, pH 8 buffer for 30 minutes at 23°C. The reaction gave 1.5 linkers per antibody as determined by DTNB assay.

For conjugation with Gel_{C10}, H65 antibody at 2.2 mg/mL was derivitized with 10x m2IT and 2.5 mM DTNB in 25 mM TEOA, 150 mM NaCl, pH 8 buffer for 1 hour at 23°C. The reaction gave 1.4 linkers per antibody as determined by DTNB assay.

Chimeric H65 antibody was prepared for conjugation in a similar manner to murine H65 antibody.

Two methods were initially compared for their effectiveness in preparing immunoconjugates with recombinant gelonin. First, the native disulfide bond in recombinant gelonin was reduced by the addition of 2mM DTT at room temperature for 30 minutes. The reduced gelonin was recovered by size-exclusion chromatography on a column of Sephadex GF-05LS and assayed for the presence of free sulfhydryls by the DTNB assay. 1.4 free SH groups were detected. This reduced gelonin was then reacted with H65-(M2IT)-S-S-TNB (1.8 TNB groups/H65). Under these experimental conditions, little or no conjugate was prepared between reduced gelonin and thiol-activated H65 antibody.

In contrast, when both the recombinant gelonin and the H65 antibody were first derivitized with the crosslinker M2IT (creating gelonin-(M2IT)-SH and H65-(M2IT)-S-S-TNB) and then mixed together, H65-(M2IT)-S-S-(M2IT)-gelonin conjugate was prepared in good yield (toxin/antibody ratio of 1.6). The starting materials for this conjugation (gelonin-(M2IT)-SH and H65-(M2IT)-S-S-TNB) contained linker/protein ratios of 1.2 and 1.4, respectively. Native gelonin was derivatized in a similar manner prior to conjugation to murine or chimeric H65 antibody.

The reduced gelonin analogs were mixed with H65-(M2IT)-S-S-TNB to allow conjugation. The following conjugation reactions were set up for each analog: 23 mg (in 7.2 ml) of H65-M2IT-TNB were mixed with a 5-fold molar excess of Gel_{C248} (23 mg in 6 ml) for 2 hours at room temperature, then for 18 hours overnight at 4°C; 23 mg (in 7.3 ml) of H65-m2IT-TNB were mixed with a 5-fold molar excess of Gel_{C244} (23 mg in 3 ml) for 3 hours at room temperature, then for 18 hours overnight at 4°C; 9 mg (in 2.8 mL) of H65-m2IT-TNB were mixed with a 5-fold molar excess of Gel_{C247} (9 mg in 2.25 mL) for 2 hours at room temperature, then for 5 nights at 4°C; 9 mg (in 2.8 mL) of H65-m2IT-TNB were mixed with a 5-fold molar excess of Gel_{C239} (9mg in 2.6 mL) for 2 hours at room temperature, then at 4°C for 3 days; 12 mg (in 1.9 mL) of H65-m2IT-TNB were mixed with a 5.6-fold molar excess of Gel_{A50(C44)} (13.44 mg in 3.2 mL) for 4.5 hours at room temperature, then 4°C overnight; and 11 mg of H65-m2IT-TNB were mixed with a 5-fold molar excess of Gel_{C10} (11 mg in 3.5 mL) for 4 hours at room temperature, then at 4°C overnight.

Following conjugation, unreacted M2IT linkers on the antibody were quenched with 1:1 mole cysteamine to linker for 15 minutes at room temperature. The quenched reaction solution was then loaded onto a gel filtration column [Sephadex G-150 (Pharmacia) in the case of Gel_{C248}, Gel_{C247}, Gel_{C244} and Gel_{C239} and an AcA-44 column (IBF Biotecnics, France) in the case of Gel_{A50(C44)} and Gel_{C10}]. The reactions were run over the gel filtration columns and eluted with 10 mM Tris, 0.15M NaCl pH 7. The first peak off each column was loaded onto Blue Toyopearl® resin (TosoHaas, Philadelphia, Pennsylvania) in 10 mM Tris, 30 mM NaCl, pH 7 and the product was eluted with 10 mM Tris, 0.5 M NaCl, pH 7.5.

Samples of the final conjugation products were run on 5% non-reduced SDS PAGE, Coomassie stained and scanned with a Shimadzu laser densitometer to quantitate the number of toxins per antibody (T/A ratio). The yield of final product for each analog conjugate was as follows: Gel_{C248}, 17 mg with a T/A ration of 1.6; Gel_{C247}, 1.1 mg with a T/A ratio of 1; Gel_{C244}, 4.5 mgs with a T/A ratio of 1.46; Gel_{C239}, 2.9 mg with a T/A ratio of 2.4; Gel_{A50(C44)}, 7.3 mg with a T/A ratio of 1.22; and Gel_{C10}, 6.2 mg with a T/A ratio of 1.37. Conjugation efficiency (i.e., conversion of free antibody to immunoconjugate) was significantly greater (~80%) for some analogs (Gel_{C10}, Gel_{A50(C44)}, Gel_{C239}, Gel_{C247}, and Gel_{C248}) than for others (~10%, Gel_{C244}).

### B. Gelonin Immunoconjugates With Chimeric And Humanized Antibodies

Analogs Gel_{C247} and Gel_{A50(C44)} were also conjugated to various chimeric [cH65Fab, cH65Fab' and cH65F(ab')₂] and "human engineered" [he1 Fab, he2-Fab, he3-Fab, he1Fab' and he1 F(ab')₂] antibody fragments. Chimeric H65 antibody fragments may be prepared according to the methods described in International Publication No. WO 89/00999, *supra*. The DNA sequences encoding the variable regions of H65 antibody fragments that were human engineered (referring to the replacement of selected murine-encoded amino acids to make the H65 antibody sequences less immunogenic to humans) according to the methods described above in Example 5, are set out in SEQ ID NO: 69 (the kappa chain of he1 and he2), SEQ ID NO: 70 (the gamma chain of he1), SEQ ID NO: 71 (the gamma chain of he2 and he3) and SEQ ID NO: 72 (the kappa chain of he3)

The chimeric H65 antibody fragments were conjugated to the Gel_{C247} analog in the same manner as described below for conjugation of human engineered Fab and Fab' fragments to Gel_{C247} and Gel_{A50(C44)}.

### (i) he1 Fab-Gel_{C247}

The he1 Fab was dialyzed into 25 mM TEOA buffer, 250 mM NaCl, pH 8 and then concentrated to 6.8 mg/mL prior to derivitization with the M2IT crosslinker. For the linker reaction, M2IT was used at 20-fold molar excess, in the presence of 2.5 mM DTNB. The reaction was allowed to proceed for 30 minutes at room temperature, then desalted on GF05 (gel filtration resin) and equilibrated in 0.1 M Na phosphate, 0.2M NaCl, pH 7.5. A linker number of 1.8 linkers per Fab was calculated based on the DTNB assay. The he1 Fab-M2IT-TNB was concentrated to 3.7 mg/mL prior to conjugation with Gel_{C247}.

Gel_{C247} at 12.8 mg/mL in 10 mM Na phosphate, 0.3M NaCl, was treated with 1 mM DTT, 0.5 mM EDTA for 20 minutes at room temperature to expose a reactive sulfhydryl for conjugation and then was desalted on GF05 and equilibrated in 0.1 M Na phosphate, 0.2 M NaCl, pH 7.5. Free thiol content was determined to be 0.74 moles of free SH per mole of Gel_{C247} using the DTNB assay. The gelonin was concentrated to 8.3 mg/mL prior to conjugation with activated antibody.

The conjugation reaction between the free thiol on Gel_{C247} and the derivitized he1 Fab-M2IT-TNB, conditions were as follows. A 5-fold excess of the gelonin analog was added to activated he1 Fab-M2IT-TNB (both proteins were in 0.1M Na phosphate, 0.2M NaCl, pH7.5) and the reaction mixture was incubated for 3.5 hours at room temperature and then overnight at 4°C. Following conjugation, untreated M2IT linkers were quenched with 1:1 mole cysteamine to linker for 15 minutes at room temperature. The quenched reaction solution was loaded onto a gel filtration column (G-75) equilibrated with 10 mM Tris, 150 mM NaCl, pH 7. The first peak off this column was diluted to 30 mM NaCl with 10 mM Tris, pH7 and loaded on Blue Toyopearl®. The product was eluted with 10 mM Tris, 0.5 M NaCl, pH 7.5.

### (ii) he1 Fab'-Gel_{C247}

Similarly, the H65 he1 Fab' fragment was dialyzed into 25 mM TEOA buffer, 400 mM MaCl, pH 8 at 2.9 mg/mL prior to derivitization with the M2IT crosslinker. For the linker reaction, M2IT was used at 20-fold molar excess, in the presence of 2.5 mM DTNB. The reaction was allowed to proceed for 1 hour at room temperature then it was desalted on GF05 (gel filtration resin) and equilibrated in 0.1 M Na phosphate, 0.2 M NaCl, pH 7.5. A linker number of 1.6 linkers per Fab' was calculated based on the DTNB assay. The he1 Fab'-M2IT-TNB was concentrated to 3.7 mg/mL prior to conjugation with Gel_{C247}

The Gel_{C247} at 77 mg/mL was diluted with 10 mM Na phosphate, 0.1 M NaCl to a concentration of 5 mg/mL, treated with 1 mM DTT, 0.5 mM EDTA for 30 minutes at room temperature to expose a free thiol for conjugation and then was desalted on GF05 and equilibrated in 0.1 M Na phosphate, 0.2 M NaCl, pH 7.5. Free thiol content was determined to be 1.48 moles of free SH per mole of Gel_{C247} using the DTNB assay. The Gel_{C247} was concentrated to 10 mg/mL prior to conjugation with activated he1 Fab'-M2IT-TNB.

For the reaction between the free thiol on Gel_{C247} and the derivitized he1 Fab'-M2IT-TNB, conditions were as follows. A 5.7-fold molar excess of gelonin was added to activated he1 Fab'-M2IT-TNB and the final salt concentration was adjusted to 0.25 M. The reaction mix was incubated for 1.5 hours at room temperature and then over the weekend at 4°C. Following conjugation, unreacted M2IT linkers were quenched with 1:1 mole cysteamine to linker for 15 minutes at room temperature. The quenched reaction solution was loaded onto a gel filtration column (AcA54) equilibrated with 10 mM Tris, 250 mM NaCl, pH 7.5. The first peak off this column was diluted to 20 mM NaCl with 10 mM Tris, pH 7 and loaded on Blue Toyopearl® which was equilibrated in 10 mM Tris, 20 mM NaCl, pH 7. The column was then washed with 10 mM Tris, 30 mM Nacl, pH 7.5. The product was eluted with 10 mM Tris, 1 M NaCl, pH 7.5.

### (iii) he2-Fab Gel_{A50(C44)}

The he2-Fab was dialyzed overnight into 25 mM TEOA, 0.25 M NaCl, pH 8 buffer and then concentrated to 13.3 mg/mL prior to derivitization with the M2IT crosslinker. For the linker reaction, M2IT was used in a 20-fold molar excess in the presence of 2.5 mM DTNB. The reaction was allowed to proceed for 20 minutes at room temperature and was then desalted on a GF05-LS (gel filtration) column, equilibrated in 0.1 M Na phosphate, 0.2 M NaCl with 0.02% Na azide. A linker number of 1.7 linkers per Fab-M2IT-TNB was calculated based on the DTNB assay. After derivitization and gel filtration, the he2-Fab concentration was 5.2 mg/mL.

Gel_{A50(C44)} at 8.33 mg/mL in 10 mM Na phosphate, pH 7.2 was treated with 5 mM DTT and 0.5 mM EDTA for 30 minutes at room temperature to expose a reactive thiol for conjugation and then was desalted on GF05-LS resin equilibrated in 0.1 M Na phosphate, 0.1 M NaCl with 0.5 mM EDTA plus 0.02% Na azide, pH 7.5. Free thiol content was determined to be 0.83 moles of free SH per mole of Gel_{A50(C44)} using the DTNB assay. The gelonin was concentrated to 11.4 mg/mL prior to conjugation with activated he2-Fab.

The conjugation reaction conditions between the free thiol on Gel_{A50(C44)} and the derivitized he2-Fab-M2IT-TNB were as follows. A 3-fold excess of the gelonin analog was added to activated he2-Fab-M2IT-TNB (both proteins were in 0.1 M Na phosphate, 0.1 M NaCl, pH 7.5 but the gelonin solution contained 0.5 mM EDTA as well). The reaction mixture was concentrated to half its original volume, then the mixture was incubated for 4 hours at room temperature followed by 72 hours at 4°C. Following the incubation period the efficiency of conjugation was estimated at 70-75% by examination of SDS PAGE.

Following conjugation the excess M2IT linkers were quenched by incubation with 1:1 mole cysteamine to linker for 15 minutes at room temperature. The quenched reaction as loaded onto a gel filtration column (G-75) equilibrated in 10 mM Tris, 0.15 M NaCl, pH 7. The first peak off this column was diluted to 30 mM NaCl with 10 mM Tris, pH 7 and loaded onto a Blue Toyopearl® (TosoHaas) column. The product was eluted with 10 mM Tris, 1 M NaCl, pH 7.5.

### (iv) he3-Fab Gel_{A50(C44)}

Similarly, the he3-Fab was dialyzed overnight into 25 mM TEOA, 0.25 M NaCl, pH 8 buffer and then concentrated to 5 ng/mL prior to derivitization with the M2IT crosslinker. For the linker reaction, M2IT was used in a 10-fold molar excess in the presence of 2.5 mM DTNB. The reaction was allowed to proceed for 45 minutes at room temperature and was then desalted on a GF05-LS (gel filtration) column, equilibrated in 0.1 M Na phosphate, 0.2 M NaCl with 0.02% Na azide. A linker number of 1 M2IT per Fab-M2IT-TNB was calculated based on the DTNB assay. After derivitization and gel filtration, the he3-Fab concentration was 5.3 mg/mL.

Gel_{A50(C44)} at 7.8 mg/mL in 0.1 M Na phosphate, 0.1 M NaCl, pH 7.5 was treated with 1.5 mM DTT and 1 mM EDTA for 30 minutes at room temperature to expose a reactive thiol for conjugation and then was desalted on GF05-LS resin equilibrated in 0.1 M Na phosphate, 0.1 M NaCl plus 0.02% Na azide, pH 7.5. Free thiol content was determined to be 0.66 moles of free SH per mole of Gel_{A50(C44)} using the DTNB assay. The gelonin was concentrated to 5.2 mg/mL prior to conjugation with activated he3-Fab.

The conjugation reaction conditions between the free thiol on Gel_{A50(C44)} and the derivitized he3-Fab-M2IT-TNB were as follows. A 5-fold excess of the gelonin analog was added to activated he3-Fab-M2IT-TNB (both proteins were in 0.1 M Na phosphate 0.1 M NaCl, pH 7.5). The reaction mixture was incubated for 2 hours at room temperature followed by 72 hours at 4°C. Following the incubated period the efficiency of conjugation was estimated at 70-75% by examination of SDS PAGE.

Following conjugation, the excess M2IT linkers were quenched by incubation with 1:1 mole cysteamine to linker for 15 minutes at room temperature. The quenched reaction was loaded onto a GammaBind G (immobilized protein G affinity resin, obtained from Genex, Gaithersburg, Maryland) equilibrated in 10 mM Na phosphate, 0.15 M NaCl, pH 7. It was eluted with 0.5 M NaOAc, pH 3 and neutralized with Tris. It was dialyzed into 10 mM Tris, 0.15 M NaCl, pH 7 overnight, then diluted to 30 mM NaCl with 10 mM Tris, pH 7 and loaded onto a blue Toyopearl® (TosoHaas) column. The product was eluted with 10 mM Tris, 1 M NaCl, pH 7.5

### Example 8

### Whole Cell Kill Assays

Immunoconjugates prepared with gelonin and gelonin analogs were tested for cytotoxicity against an acute lymphoblastoid leukemia T cell line (HSB2 cells) and against human peripheral blood mononuclear cells (PBMCs). Immunoconjugates of ricin A-chain with H65 antibody (H65-RTA) and antibody fragments were also tested. The ricin A-chain (RTA) as well as the H65-RTA immunoconjugates were prepared and purified according to methods described in U.S. Patent Application Serial No. 07/306,433, *supra* and in International Publication No. WO 89/06968, *supra.*

Briefly, HSB2 cells were incubated with immunotoxin and the inhibition of protein synthesis in the presence of immunotoxin was measured relative to untreated control cells. The standard immunoconjugates H65-RTA (H65 derivitized with SPDP linked to RTA), H65-Gelonin and H65-rGelonin, H65 fragment immunoconjugate, and gelonin immunoconjugate samples were diluted with RPMI without leucine at half-log concentrations ranging from 2000 to 0.632 ng/ml. All dilutions were added in triplicate to wells of microtiter plates containing 1 x 10⁵ HSB2 cells per well. HSB2 plates were incubated for 20 hours at 37°C and then pulsed with ³H-Leu for 4 hours before harvesting. Samples were counted on the Inotec Trace 96 cascade ionization counter. By comparison with an untreated sample, the picomolar concentration (pM) of immunotoxin which resulted in a 50% inhibition of protein synthesis (IC₅₀) was calculated. In order to normalize for conjugates containing differing amounts of toxin or toxin analog, the cytotoxicity data were converted to picomolar toxin (pM T) by multiplying the conjugate IC₅₀ (in pM) by the toxin/antibody ratio which is unique to each conjugate preparation.

The PMBC assays were performed as described by Fishwild *et al*., *Clin. and Exp. Immunol*., *86*:506-513 (1991) and involved the incubation of immunoconjugates with PBMCs for a total of 90 hours. During the final 16 hours of incubation, ³H-thymidine was added; upon completion, immunoconjugate-induced inhibition of DNA synthesis was quantified. The activities of the H65 and chimeric H65 antibody conjugates against HSB2 cells and PBMC cells are listed in Table 2 below.

**Table 2**

| IC₅₀ (pM T) | | |
|---|---|---|
| Conjugate | HSB2 Cells | PBMCs |
| H65-RTA | 143 | 459 |
| H65-(M2IT)-S-S-(M2IT)-Gelonin | 1770 | 81 |
| H65-(M2IT)-S-S-(M2IT)-rGelonin | 276 | 75 |
| H65-(M2IT)-S-S-Gel_{C10} | 140 | 28 |
| H65-(M2IT)-S-S-Gel_{A50(C44)} | 99 | 51 |
| H65-(M2IT)-S-S-Gel_{C239} | 2328 | 180 |
| H65-(M2IT)-S-S-Gel_{C244} | >5000 | >2700 |
| H65-(M2IT)-S-S-Gel_{C247} | 41 | 35 |
| H65-(M2IT)-S-S-Gel_{C248} | 440 | 203 |
| cH65-RTA₃₀ | 60 | 400 |
| cH65-(M2IT)-S-S-(M2IT)-Gelonin | 1770 | 140 |
| cH65-(M2IT)-S-S-(M2IT)-rGelonin | 153 | 120 |
| cH65-(M2IT)-S-S-Gel_{C239} | >7000 | 290 |
| cH65-(M2IT)-S-S-Gel_{C247} | 34 | 60 |
| cH65-(M2IT)-S-S-Gel_{C248} | 238 | 860 |
| H65-(M2IT)-S-S-Gel_{A44(C50)} | 338 | ND* |
| H65-(M2IT)-S-S-Gel_{C247A44A50} | 71 | ND* |

| | | |
|---|---|---|
| * -- Not determined. | | |

Against HSB2 cells, many of the gelonin analog immunoconjugates were significantly more potent than conjugates prepared with native gelonin or recombinant, unmodified gelonin, both in terms of a low IC₅₀ value, but also in terms of a greater extent of cell kill. Against human PBMCs, the gelonin analog conjugates were at least as active as native and recombinant gelonin conjugates. Importantly, however, some of the conjugates (for example, Gel_{C10}, Gel_{A50(C44)} and Gel_{C247}) exhibited an enhanced potency against PBMCs compared to native and recombinant gelonin conjugates, and also exhibited an enhanced level of cell kill.

The activities of the H65 antibody fragment conjugates against HSB2 cells and PBMC cells are listed in Tables 3 and 4 below, wherein extent of kill in Table 3 refers to the percentage of protein synthesis inhibited in HSB2 cells at the highest immunotoxin concentration tested (1 µg/ml).

**Table 3**

| IC₅₀ (pM T) | | |
|---|---|---|
| Conjugate | HSB2 Cells | PBMCs |
| cH65Fab'-RTA 30 | 530 | 1800 |
| cH65Fab'-rGelonin | 135 | 160 |
| cH65Fab'-Gel_{C247} | 48 | 64 |
| cH65F(ab')₂-RTA 30 | 33 | 57 |
| cH65F(ab')₂-rGelonin | 55 | 34 |
| cH65F(ab')₂-Gel_{C247} | 23 | 20 |
| cH65F(ab')₂-Gel_{C248} | 181 | 95 |

**Table 4**

| IC₅₀ (pM T) | | |
|---|---|---|
| Conjugate | HSB2 Cells | Extent of Kill |
| he1 Fab'-Gel_{C247} | 57.7 | 93% |
| he1 Fab-Gel_{C247} | 180.0 | 94% |
| he2-Fab-Gel_{A50(C44)} | 363.0 | 91% |
| he3-Fab-Gel_{A50(C44)} | 191.0 | 93% |
| cH65Fab'-Gel_{C247} | 47.5 | 93% |
| cH65F(ab')₂-rGelonin | 45.4 | 85% |
| cH65F(ab')₂-Gel_{C247} | 77.5 | 83% |
| cH65F(ab')₂-Gel_{C247} | 23.2 | 85% |

The data in Table 3 show that monovalent (Fab or Fab') fragments conjugated to various forms of gelonin are more potent than RTA conjugates. Table 4 shows that the human-engineered gelonin-Fab conjugates exhibit a very high degree of extent of kill.

### Example 9

### Properties Of Gelonin Immunoconjugates

### A. Solubility

Recombinant gelonin and the gelonin analogs exhibited enhanced solubility in comparison to both native gelonin and RTA30. In addition, recombinant gelonin and gelonin analog immunoconjugates exhibited enhanced solubility relative to immunoconjugates prepared with native gelonin and RTA30. This enhanced solubility was particularly noteworthy for recombinant gelonin and analog conjugates prepared with chimeric Fab fragments.

### B. Disulfide Bond Stability Assay

The stability of the disulfide bond linking a RIP to a targeting molecule (such as an antibody) is known to influence the lifespan of immunoconjugates *in vivo* [*See* Thorpe *et al., Cancer Res., 47*:5924-5931 (1987), incorporated by reference herein]. For example, conjugates in which the disulfide bond is easily broken by reduction *in vitro* are less stable and less efficacious in animal models [*See* Thorpe *et al., Cancer Res., 48*:6396-6403 (1988), incorporated by reference herein].

Immunoconjugates prepared with native gelonin, recombinant gelonin and gelonin analogs were therefore examined in an *in vitro* disulfide bond stability assay similar to that described in Wawrzynczak *et al.*, *Cancer Res., 50*:7519-7526 (1990), incorporated by reference herein. Conjugates were incubated with increasing concentrations of glutathione for 1 hour at 37°C and, after terminating the reaction with iodoacetamide, the amount of RIP released was quantitated by size-exclusion HPLC on a TosoHaas TSK-G2000SW column.

By comparison with the amount of RIP released by high concentrations of 2-mercaptoethanol (to determine 100% release), the concentration of glutathione required to release 50% of the RIP (the RC₅₀) was calculated. The results of assays for H65 antibody conjugates are set out in Table 5 below.

**Table 5**

| Conjugate | RC₅₀ (mM) |
|---|---|
| H65-RTA 30 | 3.2 |
| H65-(M2IT)-S-S-(M2IT)-gelonin | 11.1 |
| H65-(M2IT)-S-S-(M2IT)-rGelonin | 3.0 |
| H65-(M2IT)-S-S-Gel_{C10} | 2.5 |
| H65-(M2IT)-S-S-Gel_{A50(C44)} | 0.6 |
| H65-(M2IT)-S-S-Gel_{C239} | 774.0 |
| H65-(M2IT)-S-S-Gel_{C244} | 1.2 |
| H65-(M2IT)-S-S-Gel_{C247} | 0.1 |
| H65-(M2IT)-S-S-Gel_{C248} | 0.4 |
| cH65-RTA 30 | 2.50 |
| cH65-(M2IT)-S-S-(M2IT)-rGelonin | 2.39 |
| cH65-(M2IT)-S-S-Gel_{C247} | 0.11 |
| cH65-(M2IT)-S-S-Gel_{C248} | 0.32 |
| H65-(M2IT)-S-S-Gel_{A44(C50)} | 9.2 |
| H65-(M2IT)-S-S-Gel_{C247A44A50} | 0.3 |

The foregoing results indicate that the stability of the bonds between the different gelonin proteins and H65 antibody varied greatly. With the exception of Gel_{C10} and Gel_{C239}, most of the gelonin analogs resulted in conjugates with linkages that were somewhat less stable in the *in vitro* assay than the dual-linker chemical conjugate. The stability of the Gel_{C239} analog, however, was particularly enhanced.

The results of the assay for H65 antibody fragment conjugates are set out in Table 6 below.

**Table 6**

| Conjugate | RC₅₀ (mM) |
|---|---|
| he1 Fab'-Gel_{C247} | 0.07 |
| cFab'-Gelonin | 1.27 |
| cFab'-Gel_{C247} | 0.08 |
| cF(ab')₂-RTA 30 | 1.74 |
| cF(ab')₂-rGelonin | 2.30 |
| cF(ab')₂-Gel_{C247} | 0.09 |
| cF(ab')₂-Gel_{C248} | 0.32 |
| he2-Fab-Gel_{A50(C44)} | 0.46 |
| he3-Fab-Gel_{A50(C44)} | 0.58 |

From the RC₅₀ results presented in Tables 5 and 6, it appears that the particular RIP analog component of each immunotoxin dictates the stability of the immunotoxin disulfide bond *in vitro*.

### Example 10

### Pharmacokinetics Of Conjugates To H65 Antibody

The pharmacokinetics of gelonin analogs Gel_{C247}, Gel_{A50(C44)}, and Gel_{C10} linked to whole H65 antibody was investigated in rats. An IV bolus of 0.1 mg/kg of ¹²⁵I-labelled immunoconjugate H65-(M2IT)-S-S-Gel_{C247}, H65-(M2IT)-S-S-Gel_{A50(C44)} or H65-(M2IT)-S-S-Gel_{C10} was administered to male Sprague-Dawley rats weighing 134-148 grams. Serum samples were collected from the rats at 3, 15, 30 and 45 minutes, and at 1.5, 2, 4, 6, 8, 18, 24, 48, 72, and 96 hours. Radioactivity (cpm/ml) of each sample was measured, and SDS-PAGE was performed to determine the fraction of radioactivity associated with whole immunoconjugate. Immunoconjugate-associated serum radioactivity was analyzed using the computer program PCNONLIN (SCI Software, Lexington, Kentucky). Table 7 below lists the pharmacokinetic parameters of the immunoconjugates. In that table, the standard error for each value is indicated and a one way analysis of variance is presented, IC is the immunoconjugate (specified by the abbreviation for the gelonin variant that is part of the immunoconjugate), n is the number of animals in the study, Vc is the central volume of distribution, Cl is the clearance, MRT is the total body mean residence time, Alpha is the α half-life and Beta is the β half-life of the immunoconjugate.

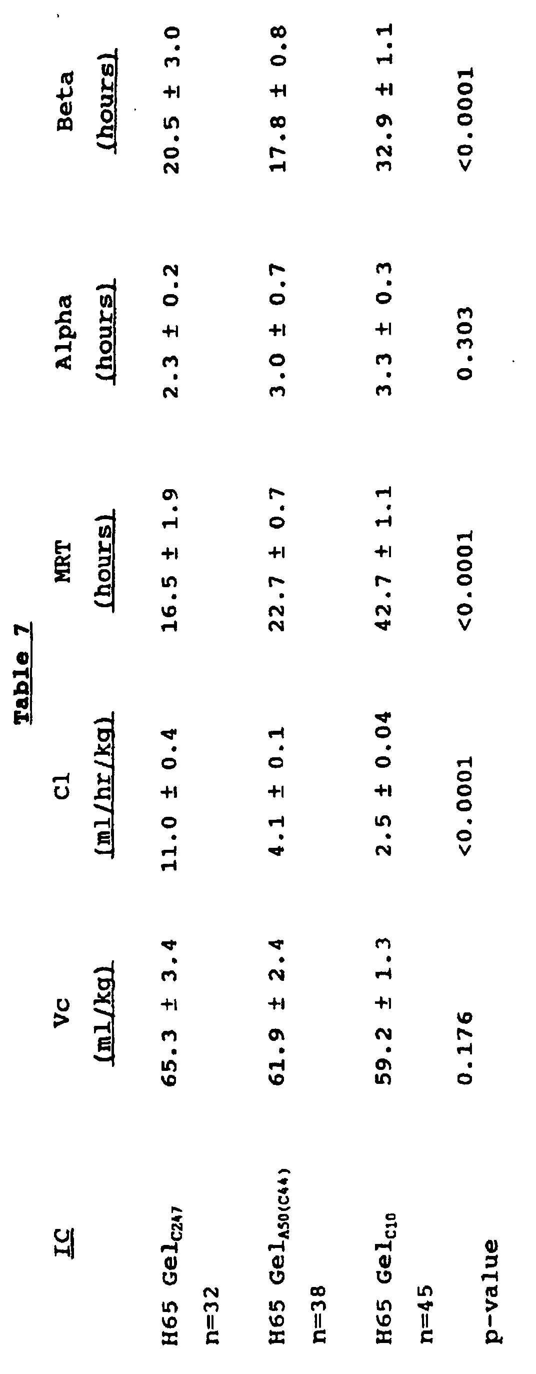

The Gel_{C247} immunoconjugate was found to have α and β half lives of 2.3 and 20 hours, with a total mean residence time of 17 hours. The 72 and 96 hour time points were excluded from analysis because of the poor resolution of immunoconjugate associated radioactivity on the SDS-PAGE gel for these serum samples.

Because *in vitro* studies suggested that the Gel_{C10} immunoconjugate had greater disulfide bond stability, it was anticipated that its half lives in vivo would be longer relative to the cys₂₄₇ form of the immunoconjugate. The β half life of the immunoconjugate was about 33 hours compared to 20 hours for the Gel_{C247} conjugate. The total mean residence time was also much greater for the Gel_{C10} immunoconjugate (42 hours versus 42 hours for the Gel₂₄₇ conjugate). In addition, the clearance of the Gel_{C10} immunoconjugate was 2.5 ml/hr/kg, about four times less than that of the Gel_{C247} immunoconjugate (11 ml/hr/kg). As also predicted from the *in vitro* disulfide stability data, the clearance of the Gel_{A50(C44)} immunoconjugate was intermediate between those of the Gel_{C10} and Gel_{C247} immunoconjugates.

Based on these studies, the Gel_{C10} analog conjugated to H65 antibody has greater *in vivo* stability than the Gel_{A50(C44)} and Gel_{C247} analogs conjugated to H65 antibody (as determined by the longer mean residence time and clearance rates), although the properties of the Gel_{A50(C44)} immunoconjugate more closely resembled those of the Gel_{C10} immunoconjugate than the Gel_{C247} immunoconjugate.

### Example 11

### Pharmacokinetics Of Conjugates To H65 Antibody Fragments

The pharmacokinetics of Gel_{C247} and Gel_{A50(C44)} analogs linked to human engineered H65 Fab fragments were also investigated in rats. An IV bolus of 0.1 mg/kg of ¹²⁵l-labelled he1 H65 Fab-Gel_{C247}, he2 H65 Fab-Gel_{A50(C44)} or he3 H65 Fab-Gel_{A50(C44)} was administered to male Sprague-Dawley rats weighing 150-180 grams. Serum samples were collected at 3, 5, 15, 20, 30, and 40 minutes, and 1, 1.5, 3, 6, 8, 18, 24, 32, 48, and 72 hours, and were analyzed by ELISA using rabbit anti-Gelonin antibody as the capture antibody and biotin-labelled goat anti-human kappa light chain antibody as the secondary antibody. Results of the analysis are presented in Table 8 below. In the table, the standard error for each value is shown, and IC is the immunoconjugate, n is the number of animals in the study, Vc is the central volume of distribution, Vss is the steady state volume of distribution, C1 is the clearance, MRT is the total body mean residence time, Alpha is the α half-life and Beta is the β half-life of the indicated conjugate.

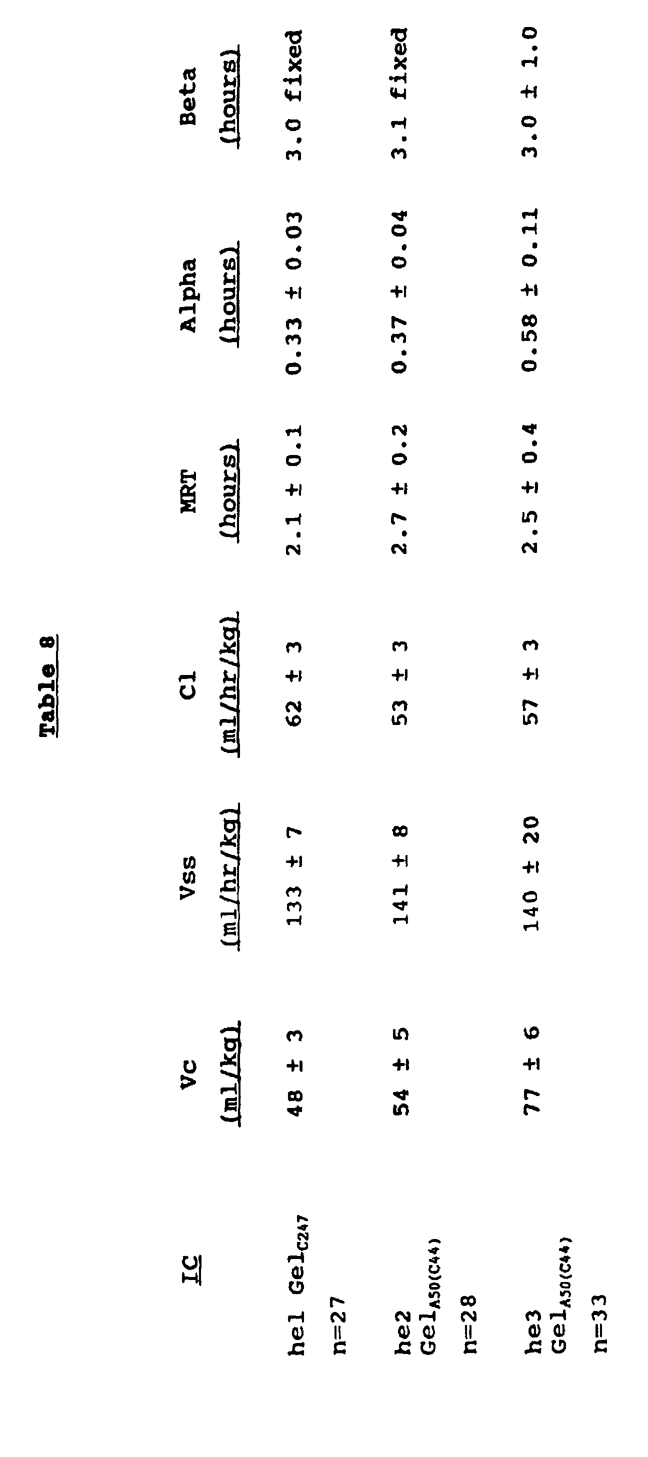

Comparing the three immunoconjugates, the pharmacokinetics of he1 H65 Fab-Gel_{C247}, he2 H65 Fab-Gel_{A50(C44)} and he3-Fab-Gel_{A50(C44)} were very similar, having similar alpha and beta half-lives, mean residence times, and clearance, particularly when comparing parameters obtained from the ELISA assayed curves. This is in contrast to their whole antibody immunoconjugate counterparts, where the clearance of Gel_{C247} immunoconjugate (11 ml/kg/hr) was three-fold greater than that of Gel_{A50(C44)} immunoconjugate (4 ml/kg/hr). This suggests that cleavage of the disulfide bond linking the Fab fragment and gelonin is not as important for the serum clearance of Fab immunoconjugates as for whole antibody immunoconjugates.

### Example 12

### Immunogenicity Of Immunoconjugates

Outbred Swiss/Webster mice were injected repeatedly (0.2 mg/kg each injection) with murine H65 antibody conjugates prepared with RTA, RTA30 and recombinant gelonin. The cycle was such that each animal was injected on days 1 and 2, and then the injections were repeated 28 and 29 days later. The animals received 5 such cycles of injections. One week and three weeks following each series of injections, blood was collected and the amount of anti-RIP antibodies present was determined by ELISA; peak titers for each cycle are shown in Table 9. RTA and RTA30 generated strong responses which began immediately following the first cycle of injections and remained high throughout the experiment. In contrast, no immune response was detected for the gelonin conjugate, even after 5 cycles of injections. When the conjugates were mixed with Complete Freund Adjuvant and injected i.p. into mice, anti-RTA and RTA-30 antibodies were readily detected after several weeks. These data indicate that anti-gelonin antibodies, if generated, would have been detected by the ELISA assay, and suggest that recombinant gelonin may be much less immunogenic in animals than is RTA.

**Table 9**

| Cycle | H65-RTA | H65-RTA30 | H65-rGel |
|---|---|---|---|
| Prebleed | 100 | 100 | 100 |
| Cycle 1 | 168 | 117 | 100 |
| Cycle 2 | 4208 | 1008 | 100 |
| Cycle 3 | 7468 | 3586 | 100 |
| Cycle 4 | 5707 | 3936 | 100 |
| Cycle 5 | 4042 | 2505 | 100 |

### Example 13

### In vivo Efficacy Of Immunoconjugates

A human peripheral blood lymphocyte (PBL)-reconstituted, severe combined immunodeficient mouse model was utilized to evaluate the *in vivo* efficacy of various immunoconjugates comprising the gelonin analogs Gel_{C247} and Gel_{A50(C44)}. Immunoconjugates were tested for the capacity to deplete human blood cells expressing the CD5 antigen.

### A. Human PBL Donors And Cell Isolation

Human peripheral blood cells were obtained from lymphapheresis samples (HemaCare Corporation, Sherman Oaks, CA) or venous blood samples (Stanford University Blood Bank, Palo Alto, CA) collected from healthy donors. Blood cells were enriched for PBLs using Ficoll-Hypaque density gradient centrifugation (Ficoll-Paque®; Pharmacia, Piscataway, New Jersey) and subsequently washed 4 times with PBS. Residual erythrocytes were lysed with RBC lysing buffer (16 µM ammonium chloride, 1 mM potassium bicarbonate, 12.5 µM EDTA) during the second wash. Cell viability in the final suspension was >95% as assessed by trypan blue dye exclusion.

### B. Animals And Human PBL Transfer

CB.17 *scid*/*scid* (SCID) mice were purchased from Taconic (Germantown, New York) or were bred under sterile conditions in a specific pathogen-free animal facility (original breeding pairs were obtained from Hana Biologics, Alameda, California). Animals were housed in filter-top cages and were not administered prophylactic antibiotic treatment. Cages, bedding, food and water were autoclaved before use. All manipulations with animals were performed in a laminar flow hood.

Untreated SCID mice were bled for determination of mouse Ig levels. Human PBL-injected mice were bled at various intervals for quantitation of human Ig and sIL-2R. Blood collection was from the retro-orbital sinus into heparinized tubes. Blood samples were centrifuged at 300 x g for 10 min, and plasma was collected and stored at -70°C. Mouse and human Ig were quantified using standard sandwich ELISAs. Briefly, flat-bottom microtiter plates (MaxiSorp Immuno-Plates, Nunc, Roskilde, Denmark) were coated overnight at 4°C with goat anti-mouse IgG+IgA+IgM (Zymed Laboratories, Inc., South San Francisco, California) or goat anti-human Igs (Tago, Inc., Burlingame, California) in bicarbonate buffer, pH 9.6. Plates were blocked for 2 hours at room temperature with 1% BSA in Tris-buffered saline, pH 7.5 (TBS), and then incubated at 37°C for 1 hour with standards or samples serially-diluted in TBS/1% BSA/0.05% Tween 20. Standards used were a monoclonal mouse IgG2a (IND1 anti-melanoma; XOMA Corporation, Berkeley, California) and polyclonal human Ig (Sigma Chemical Co., St. Louis, Missouri). Subsequently, plates were washed with TBS/Tween 20 and incubated at 37°C for 1 hour with alkaline phosphatase-conjugated goat anti-mouse IgG+IgA+IgM or goat anti-human Igs (Caltag Laboratories, South San Francisco, California). Detection was by measurement of absorbance at 405 nm following incubation with 1 mg/ml p-nitro-phenylphosphate (Sigma) in 10% diethanolamine buffer, pH 9.8. Plasma from a normal BALB/c mouse was used as a positive control in the mouse Ig ELISA. Plasma samples from naive SCID mice or normal BALB/c mice did not have detectable levels of human Ig. Human sIL-2R was quantified using an ELISA kit (Immunotech S.A., Marseille, France) as per the manufacturer's instructions.

Five-to-seven week old mice with low plasma levels of mouse Ig (<10µg/ml) were preconditioned with an i.p. injection of cyclophosphamide (Sigma) at 200 mg/kg. Two days later, they were injected i.p. with 25-40 x 10⁶ freshly-isolated human PBL suspended in 0.8 ml PBS.

### C. Immunoconjugate Treatment

SCID mice were bled at approximately 2 weeks after human PBL transplantation. Mice with undetectable (<10 pM) or low plasma levels of human SIL-2R were eliminated from the study. The cut-off for exclusion of mice with detectable, but low, levels of human sIL-2R was empirically determined for each study and was generally 20 pM. The remaining mice were divided into groups and were administered vehicle or immunoconjugate as an i.v. bolus (0.2 mg/kg) daily for 5 consecutive days. Animals were sacrificed 1 day after cessation of treatment for quantitation of human T cells in tissues and human sIL-2R in plasma.

### D. Collection Of Tissues And Analysis Of PBL Depletion

Blood was collected from the retro-orbital sinus into heparinized tubes. Mice were then killed by cervical dislocation and spleens were removed aseptically. Single cell suspensions of splenocytes were prepared in HBSS by pressing the spleens between the frosted ends of sterile glass microscope slides. Collected cells were washed twice with PBS. Erythrocytes were eliminated from blood and splenocyte suspensions using RBC lysing buffer. Subsequently, cells were resuspended in PBS for enumeration. Recovered cells were then assayed for Ag expression using flow cytometry.

Two to five hundred thousand cells in 100 µl of PBS/1% BSA/0.1% sodium azide were incubated on ice for 30 min. with saturating amounts of various FITC- or phycoerythrin (PE)-conjugated Abs (Becton-Dickinson, Mountain View, CA) Abs used for staining included: HLe-1-FITC (IgG1 anti-CD45), Leu 2-FITC (IgG1 anti-CD8), Leu 3 PE (IgG1 anti-CD4), and Leu M3-PE (IgG2a anti-CD14). Cells were then washed in cold buffer and fixed in 0.37% formaldehyde in PBS. Samples were analyzed on a FACscan (Becton-Dickinson) using log amplifiers. Regions to quantify positive cells were set based on staining of cells obtained from naive SCID mice. The absolute numbers of human Ag-positive cells recovered from SCID tissues were determined by multiplying the percent positive cells by the total number of cells recovered from each tissue sample. The total number of leukocytes in blood was calculated using a theoretical blood volume of 1.4 ml/mouse. The detection limit for accurate quantitation of human cells in SCID mouse tissues was 0.05%. All statistical comparison between treatment groups were made using the Mann-Whitney U test. Treatment groups were determined to be significantly different from buffer control groups when the p value was <0.05. Results are presented in Table 10 below, wherein + indicates a significant difference from controls, - indicates an insignificant difference and NT means the conjugate was not tested. CD5 Plus (XOMA Corporation, Berkeley, California) is mouse H65 antibody chemically linked to RTA and is a positive control. OX19 Fab-Gel_{C247} is a negative control immunoconjugate. The OX19 antibody (European Collection of Animal Cell Cultures #84112012) is a mouse anti-rat CD5 antibody that does not cross react with human CD5.

**Table 10**

| Test Article | Human T Cell Depletion | |
|---|---|---|
| | Spleen | Blood |
| CD5 Plus | + | + |
| cH65 F(ab')₂ | - | - |
| cH65 Fab' | - | - |
| H65-rGEL | + | + |
| cH65 F(ab')₂-rGel | + | + |
| cH65 Fab'-rGel | + | + |
| cH65 F(ab')₂-Gel_{c247} | + | NT |
| cH65 Fab'-Gel_{c247} | + | + |
| he1H65 Fab'-Gel_{c247} | + | NT |
| cH65 Fab'-Gel_{A50(C44)} | + | + |
| OX19 Fab-Gel_{c247} | - | - |

All the gelonin immunoconjugates were capable of depleting human cells in the SCID mouse model.

### Example 14

### Construction Of Gelonin Immunofusions With Chimeric Antibodies

Several genetic constructs were assembled which included a natural sequence gelonin gene fused to an H65 truncated heavy chain gene (Fd or Fd'), or an H65 light chain gene (kappa). In this Example, H65 Fd, Fd', and H65 light chain refer to chimeric constructs. The H65 Fd sequence consists of the nucleotides encoding the murine H65 heavy chain variable (V), joining (J) and human IgG₁, constant (C) domain 1 regions, including the cysteine bound to light chain IgG₁ and has the carboxyl terminal sequence SCDKTHT (SEQ ID NO: 130). The H65 Fd' sequence has the H65 Fd sequence with the addition of the residues CPP from the hinge region of human IgG₁ heavy chain, including a cysteine residue which is bound to the other human IgG₁ heavy chain and its F(ab')₂ fragment. *See* Better, *et al*., *Proc. Nat. Acad. Sci. (USA), 90*: 457-461 (1993), incorporated by reference herein.

The H65 light chain sequence consists of the nucleotides encoding the murine H65 light chain variable (V), joining (J), and human kappa (Cₖ) regions. The DNA sequences of the V and J regions of the H65 Fd and kappa fragment genes linked to the *pel*B leader can be obtained from GenBank (Los Alamos National Laboratories, Los Alamos, New Mexico) under Accession Nos. M90468 and M90467, respectively. Several of the gene fusions included a gelonin gene linked at the 5' end of an H65 Fab fragment gene while the others included a gelonin gene linked at the 3' end of an H65 Fab fragment gene. A DNA linker encoding a peptide segment of the *E. coli* shiga-like toxin (SLT) (SEQ ID NO: 56), which contains two cysteine residues participating in a disulfide bond and forming a loop that includes a protease sensitive amino acid sequence) or of rabbit muscle aldolase [(RMA) as in SEQ ID NO: 57, which contains several potential cathepsin cleavage sites] was inserted between the gelonin gene and the antibody gene in the constructs. Alternatively, a direct fusion was made between a gelonin gene and an H65 Fab fragment gene without a peptide linker segment. Table 11 below sets out a descriptive name of each gene fusion and indicates the expression plasmid containing the gene fusion and the section of the application in which each is designated. Each plasmid also includes the Fab fragment gene (shown in parentheses in Table 11) with which each particular gene fusion was co-expressed. The inclusion of a cysteine from a hinge region (Fd') allows potential formation of either monovalent Fab' or bivalent F(ab')₂ forms of the expression product of the gene fusion.

**Table 11**

| Section | Plasmid | Description |
|---|---|---|
| B(i) | pING3754 | Gelonin::SLT::Fd' (kappa) |
| B(ii) | pING3757 | Gelonin::SLT::kappa (Fd') |
| B(iii) | pING3759 | Gelonin::RMA::Fd' (kappa) |
| B(iv) | pING3758 | Gelonin::RMA::kappa (Fd') |
| A(i) | pING4406 | Fd::SLT::Gelonin (kappa) |
| A(ii) | pING4407 | kappa::SLT::Gelonin (Fd) |
| A(iii) | pING4408 | Fd::RMA::Gelonin (kappa) |
| A(iv) | pING4410 | kappa::RMA::Gelonin (Fd) |
| C(i) | pING3334 | Gelonin::Fd (kappa) |

### A. Fusions Of Gelonin At The Carboxyl-Terminus Of Antibody Genes

### (i) Fd::SLT::Gelonin (kappa)

A gelonin gene fusion to the 3'-end of the H65 Fd chain with the 23 amino acid SLT linker sequence was assembled in a three piece ligation from plasmids pVK1, pING3731 (ATCC 68721) and pING4000. Plasmid pVK1 contains the Fd gene linked in-frame to the SLT linker sequence and some H65 Fd' and kappa gene modules as in pING3217, shown in Better, *et al., Proc. Nat. Acad. Sci. (USA)*: 457-461 (1993), except that the kappa and Fd' regions are reversed. Plasmid pING3731 contains the gelonin gene, and pING4000 contains the H65 kappa and Fd' genes each linked to the pe1B leader sequence under the control of the araB promoter as a dicistronic message.

Plasmid pVK1 was designed to link the 3'-end of a human IgG Fd constant region in-frame to a protease-sensitive segment of the SLT gene bounded by two cysteine residues which form an intra-chain disulfide bond. The SLT gene segment (20 amino acids from SLT bounded by cysteine residues, plus three amino acids introduced to facilitate cloning) was assembled from two oligonucleotides, SLT Linker 1 and SLT Linker 2. The two oligonucleotides were annealed and ligated into a vector (pING3185) containing *Pst*I and *Xho*I cohesive ends, destroying the *Pst*I site and maintaining the *Xho*I site. Plasmid pING3185 contained an engineered *Pst*I site at the 3'-end of the Fd gene, and contained an *Xho*I site downstream of the Fd gene. The product of this ligation, pVK1, contained the H65 Fd gene (fused to the pe1B leader) in frame with the SLT linker segment, and contained two restriction sites, *Fsp*I and *Sca*I, at the 3'-end of the SLT linker.

Plasmid pVK1 was digested with *Sau*I and *Sca*I, and the 217 bp fragment containing a portion of the Fd constant domain and the entire SLT gene segment was purified by electrophoresis on an agarose gel. pING3731 was digested with *Sma*I and *Xho*I and the 760 bp gelonin gene was similarly purified. Plasmid pING4000 was digested with *Sau*I and *Xho*I and the vector segment containing the entire kappa gene and a portion of the Fd gene was also purified. Ligation of these three DNA fragments resulted in pING4406 containing the Fd::SLT::Gelonin (kappa) gene fusion vector.

### (ii) kappa::SLT::Gelonin (Fd)

A gelonin gene fusion to the 3'-end of the H65 kappa chain with the 25 amino acid SLT linker sequence (20 amino acids from SLT bounded by cysteine residues, plus 5 amino acids introduced to facilitate cloning) was assembled from the DNA segments in pING3731 (ATCC 68721) and pING3713.

Plasmid pING3713 is an Fab expression vector where the H65 Fd and kappa genes are linked in a dicistronic transcription unit containing the SLT linker segment cloned in-frame at the 3'-end of the kappa gene. The plasmid was constructed as follows. In a source plasmid containing the H65 Fd and kappa genes, an *Eag*I site was positioned at the 3'-end of the kappa gene by site directed mutagenesis without altering the encoded amino acid sequence. The SLT gene segment from pVK1 was amplified with primers SLT-*Eag*I-5' and SalI for in frame linkage to the *Eag*I site at the 3'-end of the kappa gene. The 140 bp PCR product was digested with *Eag*I and *Xho*I, and the 75 bp fragment containing the SLT gene segment was cloned adjacent to the Fd and kappa genes in the source plasmid to generate pING3713.

For construction of gene fusion to gelonin, pING3713 was cut with *Sca*I and *Xho*I, and the vector fragment containing the Fd gene and kappa::SLT fusion was purified. pING3731 was digested with *Sma*I and *Xho*I and the DNA fragment containing the gelonin gene was also purified. The product of the ligation of these two fragments, pING4407, contains the Fd and kappa::SLT::gelonin genes.

### (iii) Fd::RMA::Gelonin (kappa)

A gelonin gene fusion to the 3'-end of the H65 Fd chain with the 21 amino acid RMA linker sequence (20 amino acids from RMA, plus 1 amino acid introduced to facilitate cloning) was assembled in a three piece ligation from plasmids pSH4, pING3731 (ATCC 68721) and pING4000.

Plasmid pSH4 contains an Fd gene linked in frame to the RMA linker sequence. The RMA gene segment was linked to the 3'-end of Fd by overlap extension PCR as follows. The 3'-end (constant region) of the Fd gene was amplified by PCR from a source plasmid with the primers KBA-γ2 and RMAG-1. Any Fd constant region may be used because constant regions of all human IgG₁ antibodies are identical in this region. The product of this reaction was mixed with primer RMA-76, which annealed to the amplified product of the first reaction, and the mixture was amplified with primers KBA-γ2 and RMAK-2. The PCR product contained a portion of the Fd constant region linked in-frame to the RMA gene segment. The product also contained a *Sca*I restriction site useful for in-frame fusion to a protein such as gelonin, and an *Xho*I site for subsequent cloning.

This PCR product was cut with *Sau*l and *Xho*I and ligated adjacent to the remainder of the Fd gene to generate pSH4.

For assembly of the gene fusion vector containing the Fd::RMA::Gelonin, kappa genes, pSH4 was cut with *Sau*I and *Sca*I and the Fd::RMA segment was purified. Plasmid pING3731 was cut with *Sma*I and *Xho*I and the 760 bp DNA fragment containing the gelonin gene was purified, and pING4000 was cut with *Sau*I and *Xho*I and the vector was purified. The product of the ligation of these fragments, pING4408, contained the Fd::RMA::Gelonin and kappa genes.

### (iv) kappa::RMA::Gelonin (Fd)

A gelonin gene fusion to the 3'-end of the H65 kappa chain with the 21 amino acid RMA linker sequence was assembled in a three piece ligation from plasmids pSH6, pING4408 (see the foregoing paragraph) and pING3713.

Plasmid pSH6 contains a kappa gene linked in-frame to the RMA linker sequence. The RMA gene segment was linked to the 3'-end of kappa by overlap extension PCR as follows. The 3'-end (constant region) of the kappa gene was amplified by PCR from a source plasmid with the primers KBA-K2 and RMAK-1. The product of this reaction was mixed with primer RMA-76 (SEQ ID NO: 81), which annealed to the amplified product of the first reaction, and the mixture was amplified with primers KBA-K2 and RMAK-2. The PCR product contained a portion of the kappa constant region linked in-frame to the RMA gene segment. The product also contained a *Sca*I restriction site useful for in-frame fusion to a protein such as gelonin, and an *Xho*I site for subsequent cloning. This PCR product was cut with *Sst*I and *Xho*I and ligated adjacent to the remainder of the kappa gene to generate pSH6.

For assembly of the gene fusion vector containing the kappa::RMA::Gelonin and Fd genes, pSH6 was cut with *Hind*III and *Pst*I and the DNA fragment containing the kappa constant region and a portion of the RMA linker (the *Pst*I RMA linker segment contains a *Pst*I site) segment was purified. Plasmid pING4408 was cut with *Pst*I and SalI and the DNA fragment containing a segment of the RMA linker, the gelonin gene and a portion of the tetracycline resistance gene in the vector segment was purified. pING3713 was cut with SalI and *Hind*III and the vector was purified. The product of the ligation of these three fragments, pING4410, contained the kappa::RMA::Gelonin and Fd genes.

### B. Fusions Of Gelonin At The Amino-Terminus Of Antibody Genes

### (i) Gelonin::SLT::Fd' (kappa)

A gelonin gene fusion to the 5'-end of the H65 Fd' chain with a 25 amino acid SLT linker sequence (20 amino acids from SLT bounded by cysteine residues, plus five amino acids introduced to facilitate cloning) was assembled in a three piece ligation from plasmids pING3748, pING3217, and a PCR fragment encoding the H65 heavy chain variable region (V_{H}) gene segment which is the variable region of the Fd' gene in pING3217. Plasmid pING3748 contains the gelonin gene linked in-frame to the SLT linker sequence, and pING3217 contains the H65 Fd' and kappa genes in a dicistronic transcription unit.

Plasmid pING3825 (see Example 2) was amplified with PCR primers gelo3'-*Eag* and gelo-9 to introduce an *Eag*I restriction site at the 3'-end of the gelonin gene by PCR mutagenesis. The PCR product was cut with *Bcl*I and *Eag*I and the 56 bp DNA fragment was purified. Plasmid pING3713 was cut with *Eag*I and *Xho*I, and the 77 bp DNA fragment containing the SLT linker was purified. The 56 bp *Bcl*I to *Eag*I fragment and the 77 bp *Eag*I to *Xho*I fragment were ligated into pING3825 which had been digested with *Bcl*I and *Xho*I to generate pING3748 which contains the gelonin gene linked in-frame to the SLT linker sequence.

For assembly of the gene fusion vector containing the Gelonin::SLT::Fd' and kappa genes, the H65 V_{H} was amplified by PCR from pING3217 with primers H65-G1 and H65-G2, and the product was treated with T4 polymerase followed by digestion with *Nde*I. The 176 bp fragment containing the 5'-end of the H65 heavy chain V-region was purified. Concurrently, pING3217 was digested with *Nde*I and *Xho*I, and the 1307 bp DNA fragment containing a portion of the Fd' gene and all of the kappa gene was purified. The two fragments were ligated to pING3748 which had been digested with *Sca*I and *Xho*I in a three piece ligation yielding pING3754 (ATCC 69102), which contains the Gelonin::SLT::Fd' and kappa genes.

### (ii) Gelonin::SLT::kappa (Fd')

A gelonin gene fusion to the 5'-end of the H65 kappa chain with the 25 amino acid SLT linker sequence was assembled in a three piece ligation from plasmids pING3748 (see the foregoing section), pING4000, and a PCR fragment encoding the H65 light chain variable region (V_{L}) gene segment.

For assembly of the gene fusion vector containing the Gelonin::SLT::kappa and Fd' genes, an H65 V_{L} fragment was amplified by PCR from pING3217 with primers H65-K1 and JK1-*Hind*III, and the product was treated with T4 polymerase followed by digestion with *Hind*III. The 306 bp fragment containing the light chain V-region was purified. Concurrently, pING4000 was digested with *Hind*III and *Xho*I, and the 1179 bp DNA fragment containing the kappa constant region and all of the Fd' gene was purified. The two fragments were ligated to pING3748 which had been digested with *Sca*I and *Xho*I in a three piece ligation yielding pING3757, which contains the Gelonin::SLT::kappa and Fd genes.

### (iii) Gelonin::RMA::Fd' (kappa)

A gelonin gene fusion to the 5'-end of the H65 Fd' chain with the 24 amino acid RMA linker sequence (20 amino acids from RMA, plus 4 amino acids introduced to facilitate cloning) was assembled in a three piece ligation from plasmids pING3755, pING3217 and a PCR fragment encoding the H65 V_{H} gene segment. Plasmid pING3755 contains the gelonin gene linked in-frame to the RMA linker sequence, and pING3217 contains the H65 Fd' and kappa genes in a dicistronic transcription unit.

Plasmid pING3755 was assembled to contain the gelonin gene linked to the RMA linker gene segment. The RMA linker gene segment was amplified by PCR from pSH4 with primers RMA-*Eag*I and *HIND*III-2. The 198 bp PCR product was cut with *Eag*I and *Hind*III, and the resulting 153 bp DNA fragment was purified. This RMA gene segment was cloned adjacent to gelonin using an *Pst*I to *Eag*I fragment from pING3748 and the *Pst*I to *Hind*III vector fragment from pING3825. The product of this three piece ligation was pING3755.

For assembly of the gene fusion vector containing the Gelonin::RMA::Fd', kappa genes, the H65 V_{H} was amplified by PCR from pING3217 with primers H65-G1 (SEQ ID NO: 84) and H65-G2 (SEQ ID NO: 85), and the product was treated with T4 polymerase followed by digestion with *Nde*I. The 186 bp fragment containing the 5'-end of the heavy chain V-region was purified. Concurrently, pING3217 was digested with *Nde*I and *Xho*I, and the 1307 bp DNA fragment containing a portion of the Fd' gene and all of the kappa gene was purified. These two fragments were ligated to pING3755 which had been digested with *Sca*I and *Xho*I in a three piece ligation yielding pING3759 (ATCC 69104), which contains the Gelonin::RMA::Fd' and kappa genes.

### (iv) Gelonin::RMA::kappa (Fd')

A gelonin gene fusion to the 5'-end of the H65 kappa chain with the 24 amino acid RMA linker sequence was assembled in a three piece ligation from plasmids pING3755, pING4000, and a PCR fragment encoding the H65 V_{L} gene segment.

For assembly of the gene fusion vector containing the Gelonin::RMA::kappa and Fd' genes, an H65 V_{L} segment was amplified by PCR from pING3217 with primers H65K-1 (SEQ ID NO: 86) and JK1-*Hind*III, and the product was treated with T4 polymerase followed by digestion with *Hind*III. The 306 bp fragment containing the 5'-end of the light chain V-region was purified. Concurrently, pING4000 was digested with *Hind*III and *Xho*I, and the 1179 bp DNA fragment containing the kappa constant region and all of the Fd' gene was purified. These two fragments were ligated to pING3755 which had been digested with *Sca*I and *Xho*I in a three piece ligation yielding pING3758 (ATCC 69103), which contains the Gelonin::RMA::kappa and Fd' genes.

### C. Direct Fusions Of Gelonin At The Amino Terminus Of Antibody Genes

### (i) Gelonin::Fd' (Kappa)

A direct gelonin gene fusion was constructed from pING3754. pING3754 was digested with *Bgl*II and *Xho*I and the vector segment was purified. Concurrently, pING3754 was digested with *Eag*I, treated with T4 polymerase, cut with *Bgl*II, and the gelonin gene segment was purified. pING3754 was also cut with *Fsp*I and *Xho*I, and the Fd and kappa gene segment was purified. These fragments were assembled in a three-piece ligation to generate pING3334, which contains a direct gene fusion of gelonin to Fd' in association with a kappa gene.

### Example 15

### Preparation of he3 Fab And Gelonin he3Fab Immunofusions

The sections below detail the construction of human-engineering he3Fab protein and immunofusions of gelonin to he3 Fd and kappa chains.

### A. he3-Fab Expression Plasmids

The he3 heavy chain V-region was PCR-amplified from plasmid pING4621 (pING4621 is fully described above in Example 5 above), with primers H65-G3, GAGATCCAGTTGGTGCAGTCTG (SEQ ID NO: 116) and H65G2 (SEQ ID NO: 85). Amplification was carried at using vent polymerase (New England Biolabs) for 25 cycles, including a 94°C denaturation for 1 minute, annealing at 50°C for 2 minutes, and polymerization for 3 minutes at 72°C. The PCR product was treated with polynucleotide kinase and digested with *Bst*EII and the V-region DNA was purified. The purified DNA fragment was then ligated into pIC100, which had been digested with *Sst*I, treated with T4 polymerase, and cut with *Bst*EII. The resulting fragment was then ligated with the *Bst*EII fragment from pING3218 (containing Fab' genes) to make pING4623 which contained the he3 Fd gene linked to the *pel*B leader sequence.

The he3 kappa V-region was next assembled as described above in Example 5 and in co-owned, co-pending U.S. Patent Application Serial No. 07/808,464, incorporated by reference herein, using six oligonucleotide primers,

The resulting PCR product was treated with T4 polymerase, digested with *Hind*III, and purified. The purified fragment was then cloned into pIC100, which had first been cut with *Sst*I, treated with T4 polymerase, and digested with *Xho*I, along with the 353 bp *Hind*III-*Xho*I fragment encoding the kappa constant region from pING3217. The resulting plasmid was pING4627 which contains the he3 kappa sequence linked in frame to the *pel*B leader.

Plasmid pING4628, containing the *pel*B-linked he3 kappa and Fd genes under transcriptional control of the *ara*B promoter, was assembled from pING4623 and pING4627 as follows.

An expression vector for unrelated kappa and Fd genes, pNRX-2, was first cut with *Sau*I and *Eco*RI, leaving a vector fragment which contains all the features relevant to plasmid replication, a tetracycline resistance marker, araB transcriptional control, and the 3' end of the Fd constant region. [Plasmid pNRX-2 comprises an *Eco*RI to *Xho*I DNA segment from pING 3104 (described in WO 90/02569, incorporated by reference herein). That segment contains the replication, resistance and transcription control features of pING3104 and is joined to an *Xho*I to *Sau*I DNA segment from pING1444 (described in WO 89/00999, incorporated by reference herein) which contains the 3' end of an Fd constant region.] Next pING4623 was cut with *Pst*I, treated with T4 polymerase, digested with *Sau*I and the *pel*B::Fd gene segment was then isolated. Plasmid pING4627 was cut with *Xho*I, treated with T4 polymerase, cut with *Eco*RI and ligated to the *pel*B::Fd gene segment and the pNRX-2 vector fragment to generate the he3-Fab expression vector pING4628. That plasmid contains two *Xho*I sites, one located between the kappa and Fd genes, and another 4 bp downstream of the termination codon for the Fd gene.

A vector, pING4633, which lacks the *Xho*I site between the kappa and Fd genes was constructed. To assemble pING4633, pING4623 was cut with *Eco*RI, treated with T4 polymerase, digested with *Sau*I. The *pel*B::kappa gene segment was then isolated and purified. The pNRX-2 vector fragment and the *pel*B::Fd gene segment were then ligated to the purified *pel*B::kappa gene segment to form pING4633.

Both pING4633 and pING4628 are bacterial expression vectors for he3-Fab and each comprises the he3 Fd and Kappa genes which are expressed as a dicistronic message upon induction of the host cell with L-arabinose. Moreover, pING4628 contains two *Xho*I restriction sites, one located 4bp past the Fd termination codon and one in the intergenic region between the 3' end of the Kappa gene and the 5' end of the Fd gene. Plasmid pING4633 lacks the *Xho*I site in the intergenic region.

### B. Purification Of he3Fab

Plasmids pING4628 and pING4633 were transformed into *E. coli* E104. Bacterial cultures were induced with arabinose and cell-free supernatant comprising the he3Fab was concentrated and filtered into 20 mm HEPES, pH 6.8. The sample was then loaded onto a CM Spheradex column (2.5 x 3 cm), equilibrated in 20 mM HEPEs, 1.5 mM NaCl, pH 6.8. The column was washed with the same buffer and eluted with 20 mm HEPES, 27 mM NaCl, pH 6.8. The eluate was split into 2 aliquots and each was loaded onto and eluted from a protein G (Bioprocessing) column (2.5 x 2.5 cm) separately. The protein G column was equilibrated in 20 mM HEPES, 75 MM NaCl, pH 6.8 and the sample was eluted with 100 mM glycine, 100 mM NaCl, pH 3.0. The two eluates were combined and diluted two times with 20 mM HEPES, 3 M ammonium sulfate, pH 6.8. The diluted eluates were loaded onto phenyl sepharose high substitution Fast Flow (Pharmacia) column (2.5 x. 3.3 cm), equilibrated n 20 mM HEPES, 1.5 M ammonium sulfate, pH 6.8. The column was then eluted with 20 mM HEPES, 0.6 M ammonium sulfate, pH 6.8.

### C. Gelonin::RMA::he3Kappa, he3Fd Fusions

Other genetic constructs were assembled which included a natural sequence gelonin gene fused to an he3-Fab via a linker.

A fusion comprising Gelonin::RMA::he3Kappa, Fd was assembled from DNA from plasmids pING3755, pING4633, and pING4628. Both pING4633 and pING4628 were assembled in a series of steps whereby the he3 heavy and light V-regions were individually linked in-frame to the *pel*B leader. The heavy and light V-regions were then placed together in a dicistronic expression vector under the control of the araB promoter in a bacterial expression vector.

Assembly of the Gelonin::RMA::he3Kappa, he3Fd fusions was accomplished by constructing three DNA fragments from plasmids pING3755, pING4633, and pING4628. The first such fragment was made by digesting pING3755 with *Sca*I and *Xho*I which excises the 4bp between those sites. The resulting vector fragment was purified. The second fragment for use in constructing the above fusions was obtained from plasmid pING4633, which was cut with *Ase*I (which cuts in V_{L}) and *Xho*I and the resulting 1404 bp fragment, containing the 3' end segment of the Kappa and Fd genes, was purified. The third fragment, comprising the 5' end of the Kappa variable region coding sequence, was prepared from the PCR amplified V_{L} gene contained in pING4628 using the oligonucleotide primers, Huk-7 and jkl-*Hind*III. The resulting 322 bp PCR-amplified V_{L} fragment was treated with T4 polymerase, digested with *Ase*I, and the 86 bp fragments containing the 5' end of V_{L} was purified. The three fragment produced above were ligated together to form pING3764. The DNA sequence of the PCR amplified V-region was verified by direct DNA sequencing of pING3764.

### D. Gelonin::SLT::he3Kappa, he3Fd Fusion

A Gelonin::SLT::he3Kappa, he3Fd fusion was constructed by ligating the pING4633 and pING4628 fragments described in section A immediately above with a fragment produced from pING3748 which contains Gelonin::SLT. The pING3748 fragment was produced using *Sca*I and *Xho*I as described immediately above for pING3755. The resulting vector was designated pING3763.

### E. Construction of Expression Vector Containing Gelonin::SLT::he3Fd, he3kappa Fusions

An expression vector containing the Gelonin::SLT::he3Fd, he3kappa fusion was constructed in two steps form DNA segments from plasmids pING3825, pING4628, pING4639, pING3217 [described in Better, *et al., Proc. Natl. Acad. Sci. (USA), 90*:457-461 (1993), incorporated by reference herein], and pING4627. pING3825 was digested with *Nco*I and *Xho*I, generating a 654 bp fragment containing the 3' end of the gelonin gene and a fragment containing the 5' end of the gelonin gene which were purified. Next, pING4639 was digested with *Nco*I and *Nde*I and the 903 bp fragment containing the 3' end of the Gelonin gene, the SLT linker, and the 5' end of V_{H} which resulted was purified. Finally, pING4628 was cut with *Nde*I and *Xho*I, resulting in a 523 bp fragment containing the 3' end of the Fd gene which was purified. The three fragments were then ligated to form plasmid pING3765 which contains a gene encoding a gelonin::SLT::he3Fd fusion.

Three vector fragments were used to assemble the final expression vector (containing the gelonin::SLT::he3Fd and he3 kappa segments). Plasmid pING3765 was digested with *Xho*I, treated with T4 polymerase, cut with *Nhe*I (which releases a 265 bp fragment encoding the tetracycline resistent marker), and the resulting vector fragment was purified. Plasmid pING4627, which contains the he3Kappa gene linked in-frame to the *pel*B leader was used for the construction of pING4628. Plasmid pING4627 was cut with *Pst*I, treated with T4 polymerase, and further digested with *Sst*I. The resulting 726 bp fragment, containing the Kappa gene (except 40 bp at the 3' end) was purified. Plasmid pING3217 was then cut with *Sst*I and *Nhe*I, resulting in a 318 bp fragment containing the 3' end of the Kappa gene and downstream portion, including a portion of the tetracycline resistance gene, which was purified. Ligation of the foregoing three fragments produced the final expression vector, pING3767.

### F. Construction Of Expression Vector Containing Gelonin::RMA::he3Fd Fusions

Gelonin::RMA:he3Fd, he3Kappa fusion expression vectors was constructed in two steps from plasmids pING3825, pING4628, pING3217, and pING4627. The cloning scheme used was identical to that used to generate pING3767 except that pING4638 was substituted for pING4639. Plasmid pING4638 differs from pING4639 as described below in Example 16. The intermediate vector encoding the Gelonin::RMA::Fd fusion was designated pING3766 and the final expression vector was designated pING3768.

### Example 16

### Gelonin-Single Chain Antibody Fusions

The natural sequence gelonin gene was also fused to a single chain form of the human engineered he3 H65 variable region. The gelonin gene was positioned at either the N-terminus or the C-terminus of the fusion gene and the SLT or RMA linker peptide was positioned between the gelonin and antibody domains to allow intracellular processing of the fusion protein with subsequent cytosolic release of gelonin.

### A. Construction of Gel::RMA::SCA(V_{L}-V_{H}), Gel::SLT::SCA (V_{L}-V_{H}), Gel::RMA::SCA(V_{H}-V_{L}), and Gel::SLT::SCA(V_{H}-V_{L})

A single chain antibody (SCA) form of the he3 H65 variable domain was assembled from previously constructed genes. This SCA segment consisted of the entire V and J region of the one chain (heavy or light) linked to the entire V and J segment of the other chain (heavy or light) via a 15 amino acid flexible peptide: [(Gly)₄ Ser]₃. This peptide is identical to that described in Huston *et al.*, *Proc. Natl. Acad. Sci. USA, 85*:5879-5883 (1988); Glockshuber *et al., Biochemistry, 29*:1362-1367 (1990); and Cheadle *et al., Molecular Immunol., 29*:21-30 (1992). The SCA was assembled in two orientations: V-Jₖₐₚₚₐ::[(Gly)₄Ser]₃::V-J_{Gamma} and V-J_{Gamma}::[(Gly)₄Ser]₃::V-Jₖₐₚₚₐ. Each SCA segment was assembled and subsequently fused to gelonin.

For assembly of the SCA segment V-Jₖₐₚₚₐ::[(Gly)₄Ser]₃::V-J_{Gamma}, primers HUK-7 and SCFV-1 were used to amplify a 352 bp DNA fragment containing the he3 V/J kappa sequences from pING4627 by PCR in a reaction containing 10 mM KCl, 20 mM TRIS pH 8.8, 10 mM (NH₄)₂SO₂, 2mM MgSO₄, 0.1% Triton X-100., 100 ng/ml BSA, 200 uM of each dNTP, and 2 Units of Vent polymerase (New England Biolabs, Beverley, Massachusetts) in a total volume of 100 µl. Concurrently, primers SCFV-2 and SCFV-3 were used to amplify a he3 heavy chain V/J gamma segment from pING4623, generating a 400 bp fragment. The products from these reactions were mixed and amplified with the outside primers HUK-7 and SCFV-3. The product of this reaction was treated with T4 polymerase and then cut with *Xho*I*.* The resulting 728 bp fragment was then purified by electrophoresis on an agarose gel. This fragment was ligated into the vectors pING3755 and pING3748 (see Example 10), each digested with *Sca*I and *Xho*I. The resulting vectors pING4637 and pING4412 contain the Gelonin::RMA::SCA V-Jₖₐₚₚₐ::[(Gly)₄Ser]₃::V-J_{Gamma} and Gelonin::SLT::SCA V-Jₖₐₚₚₐ::[(Gly)₄Ser]₃::V-J_{Gamma} fusion genes, respectively. The 728 bp fragment was also ligated into pIC100 previously digested with *Sst*I, treated with T4 polymerase and digested with *Xho*I, to generate pING4635. This plasmid contains the *pel*B leader sequence linked in-frame to the V-J_{Kappa}::[(Gly)₄Ser]₃::V-JJ_{gamma} gene. The *pel*B::SCA gene in pING4635 was excised as an *Eco*RI-*Xho*I restriction fragment and cloned into the bacterial expression vector to generate pING4640.

Similarly, the SCA V-J_{Gamma}::[(Gly)₄Ser]₃::V-Jₖₐₚₚₐ was assembled by amplification of pING4627 with primers SCFV-5 and SCFV-6 generating a 367 bp fragment containing he3 V/J kappa sequences, and pING4623 with primers H65-G3 and SCFV-4 generating a 385 bp fragment containing he3 gamma V/J sequences by PCR with Vent polymerase. The products from these reactions were mixed and amplified with H65-G3 and SCFV-6. The 737 bp product was treated with T4 polymerase and cut with *Xho*I. Ligation into pING3755 and pING3748 (digested with *Sca*I and *Xho*I) resulted in assembly of the Gelonin::RMA::SCA V-J_{Gamma}::[(Gly)₄Ser]₃::V-Jₖₐₚₚₐ gene fusion in pING4638 and Gelonin: :SLT::SCA V-J_{Gamma}::[(Gly)₄Ser]₃::V-Jₖₐₚₚₐ gene fusion in pING4639, respectively.

The vectors pING4637, pING4412, pING4638 and pING4639 were each transformed into *E. coli* strain E104 and induced with arabinose. Protein products of the predicted molecular weight were identified by Western blot with gelonin-specific antibodies.

### B. Construction of SCA(V_{L}-V_{H})::SLT::Gelonin Vectors

The expression vector containing SCA(V_{L}-V_{H})::SLT::Gelonin fusions was assembled using restriction fragments from previously-constructed plasmids pING4640 (containing SCA(V_{L}-V_{H})) pING4407 (containing Kappa::SLT::Gelonin, Fd), and pING3197. Plasmid pING4640 was first cut with *Bsp*HI, filled in with T4 polymerase in the presence of only dCTP, treated with mung bean nuclease (MBN) to remove the overhang and to generate a blunt end, and cut with *Ec*oRI. The resulting 849 bp fragment was purified. The SLT-containing fragment from pING4407 was excised by cutting with *Eag*I, blunted with T4 polymerase, cut with *Xho*I, and the approximately 850 bp fragment which resulted was purified. The two fragments were ligated together into pING3197, which had been treated with *Eco*RI and *Xho*I to generate pING4642. The DNA sequence at the *Bsp*HI-T4-MBN/*Eag*I junction revealed that two of the expected codons were missing but that the fusion protein was in frame.

### C. Construction of SCA(V_{H}-V_{L})::SLT::Gelonin Vectors

The expression vector containing the SCA(V_{H}-V_{L})::SLT::Gelonin fusions was assembled using DNA from plasmids pING4636, (the *E. coli* expression vector for SCA(V_{H}-V_{L})) and pING4407. Plasmid pING4636 was cut with *Bst*EII and *Xho*I and the resulting vector fragment was purified. Concurrently, pINg4636 was used as a template for PCR with primers SCFV-7, 5'TGATGCGGCCGACATCTCAAGCTTGGTGC (SEQ ID NO: 112) and H65-G13, TGATGCGGCCGACATCTCAAGCTTGGTGC3' (SEQ ID NO: 113). The amplified product was digested with *Eag*I and *Bst*EII and the resulting approximately 380 bp fragment was purified. Plasmid pING4407 was then cut with *Eag*I and *Xho*I, resulting in an approximately 850 bp fragment, which was purified. The three above fragments were ligated together to produce pING4643.

### D. Construction of SCA(V_{L}-V_{H})::RMA::Gelonin Vectors

Expression vectors containing SCA(V_{L}-V_{H})::RMA::Gelonin fusions were assembled using DNA from pING4640, pING4408 [Example 14A(iii)], and pING3825 (Example 2C). Plasmid pING4640 was cut with *Sal*I and *Bst*EII and the resulting approximately 700 bp vector fragment (containing the tetracycline resistance matter) was purified. Next, pING3825 was digested with *Nco*I and *Sal*I, resulting in an approximately 1344 bp fragment containing the 3' end of the gelonin gene and adjacent vector sequences. That fragment was purified. Plasmid pING4408 was then PCR amplified with oligonucleotide primers, RMA-G3 5'TCTAGGTCACCGTCTCCTCACCATCTGGACAGGCTGGA3' (SEQ ID NO: 114), and gelo-10. The resulting PCR product was cut with *Bst*EII and *Nco*I to generate an approximately 180 bp fragment containing the 3' end of V_{H}, RMA, and the 5' end of the Gelonin gene which was purified. The above three fragments were ligated to generate the final expression vector, pING4644.

### E. Construction of SCA(V_{H}-V_{L})::RMA::Gelonin Vectors

Expression vectors containing SCA(V_{H}-V_{L})::RMA::Gelonin were constructed using DNA from pING 4636, pING4410, and pING3825. Plasmid pING4636 was digested with *Sal*I and *Hind*III and the resulting vector fragment was purified. Next, pING3825 was cut with *Nco*I and *Sal*I and the 1344 bp fragment which resulted contained the 3' end of the gelonin gene and adjacent vector sequences encoding tetracycline resistance was purified. Finally, pING4410 was PCR amplified with primers RMA-G4, 5'TTCGAAGCTTGAGATGAAACCATCTGGACAGGCTGGA3' (SEQ ID NO: 115) and gelo-10. The PCR product was cut with *Hind*III and *Nco*I, resulting in a 180 bp fragment containing the 3'end of V_{L}, RMA, and the 5' end of Gelonin and was purified. The three above fragments were ligated together to generate the final expression vector, pING4645.

Gelonin::SCA fusions without a cleavable linker may be constructed by deletion of the SLT linker in pING4412 using the restriction enzymes *Eag*I and *Fsp*I. Digestion at these sites and religation of the plasmid results in an in-frame deletion of the SLT sequence.

### Example 17

### Multivalent Immunofusions

Multivalent forms of the immunofusions may be constructed.

### A. Construction of (Gel::RMA::kappa, Fd')₂ and (Gel::RMA::Fd', kappa)₂ Expression Vectors

Bacterial Fab expressionn vectors can result in the production of F(ab')₂ if the two cysteine residues from the human IgG1 hinge region are included into the carboxyl-terminus of the Fd protein [Better *et al., Proc. Nat*I. *Acad. Sci. USA, 90*:457-461 (1993)]. To express a gelonin fusion protein that could form a bi-valent structure like an F(ab')₂, the he3 Fd' (2C) hinge region (Better *et al., supra*) was cloned into the expression vector pING3764 (Example 15C) encoding the fusion protein Gel::RMA::kappa, Fd.

Plasmid pING3764 was cut with *Xho*I and *Bsu*36I and the approximately 7500 bp fragment containing the immunofusion gene and vector sequences was purified. Plasmid pING4629, which encodes he3 F(ab')₂, was also cut with *Bsu*36I and *Xho*I, and the approximately 200 bp DNA fragment containing the he3 Fd' (2C) gene segment was purified. These two DNA fragments were ligated to generate pING3775 encoding (Gel::RMA::kappa, Fd')₂. An expression vector encoding the fusion protein (Gel::RMA::Fd', kappa)₂ was also made.

### B. Construction of Vectors Containing Both Gel::RMA::Fd and Gel::RMA::K Fusions

In order to construct a plasmid comprising Gel::RMA::Fd and Gel::RMA::k fusions, plasmid pING3764 [described above in Example 15(b)] was digested with *Bsg*I and *Sau*I and a 5.7 kb vector fragment containing plasmid replication functions, Gel::RMA::k, and the 3' end of Fd was isolated and purified. Plasmid pING3768 [described above in Example 15(E)] was digested with *Sau*I and partially digested with PstI and a 1.5 kb fragment containing Gel::RMA::Fd was purified. Finally, pING4000 [described above in Example 14] was digested with *Bsg*I and *Pst*I, generating a 350 bp fragment containing the 3' end of the kappa gene. That fragment was purified and the 5.7 kb, 1.5 kb, and 350 kb fragments described above were ligated together to form pING3770, containing the gelonin::RMA::k and gelonin:RMA::Fd fusions.

### C. Construction of Vectors Containing Both Gel::SLT::Fd and Gel::SLT::k Fusions

Plasmid pING3772 contains the above-entitled fusions and was constructed as follows. Plasmid pING3763 [described above in Example 15(D)] was digested with *Bsg*I and *Sau*I and a 5.7 kb fragment containing the replication functions, the 5' end of Gel::SLT::k and the 3' end of Fd was generated and purified. Next, plasmid pING3767 [described in Example 15(D) above] was digested with *Sau*I and *Pst*I, generating a 1.5 kb fragment containing the 5' end of the gel::SLT::Fd fusion. That fragment was purified and pING4000 [described in Example 14 above] was digested with *Bsg*I and *Pst*I. The resulting 350 bp fragment was purified and the above-described 5.7 kb, 1.5 kb, and 350 bp fragments were ligated to form pING3772.

### D. Expression of Multivalent Fusions

Both pING3770 and pING3772 were transformed into *E. coli* (E104) cells by techniques known to those of ordinary skill in the art and induced with arabinose. Concentrated supernatants from the transformed cell cultures were analyzed by Western blot analysis with rabbit anti-gelonin antiserum. Transformants from both plasmids generated a reactive band on the gel at the size expected for a Fab molecule carrying two gelonins (approximately 105 kD). These results are consistent with the production of fusion proteins comprising monovalent Fab, with both Fd and kappa chains separately fused to gelonin.

*E. coli* strains containing plasmids pING3775, pING3770 and pING3772 were grown in fermenters and the fusion protein products were purified. The (Gel::RMA::kappa,Fd')₂ expressed from pING3775 was purified as described in Better *et al., supra*.

### Example 18

### Construction of Expression Vectors Encoding Immunofusions Without Linkers

Expression vectors encoding direct fusions of gelonin and dicistronic he3 Fab protein or single chain antibody were constructed as follows.

### A. V_{H}V_{L}::Gel

Plasmid pING4642 (Example 16B) which encodes the V_{L}V_{H}::SLT::Gel fusion protein was cut with *Fsp*I and *Nco*I, and the approximately 100 bp DNA fragment containing the 5'-end of the gelonin gene was purified. Plasmid pING4643 (Example 16C), which encodes the V_{H}V_{L}::SLT::Gel fusion protein, was cut with *Eag*I, treated with T4 polymerase and cut with *Pst*I. The approximately 850 bp DNA fragment encoding the V_{H}V_{L} gene segment was purified. The DNA fragments from pING4642 and pING4643 were ligated into the vector DNA fragment from pING4644 (Example 16D) [THIS PLASMID CONTRIBUTES THE REST OF THE GELONIN GENE THEN?] that had been cut with *Pst*I and *Nco*I to generate pING3781, which encodes the V_{H}V_{L}::Gel direct gene fusion.

### B. V_{L}V_{H}::Gel

Plasmid pING4640 which encodes the he3 SCA gene V_{L}V_{H} was cut with *Bsp*HI, treated with T4 polymerase in the presence of the nucleotide dCTP only, treated with mung bean nuclease to remove the remaining 5' overhang, and then cut with *Eco*RI. The approximately 800 bp DNA fragment containing the he3 V_{L}V_{H} gene was then purified on an agarose gel.

Plasmid pING3781 which encodes the direct fusion V_{H}V_{L}::Gel was digested with *Eag*I, treated with T4 polymerase, and then digested with *Xho*I. The approximately 800 bp DNA fragment encoding the gelonin gene was then purified on an agarose gel. The two DNA fragments from pING4640 and pING3781 were ligated into the vector DNA from pING3767 which had been digested *Eco*RI and *Xho*I and purified on an agaraose gel. The resultant plasmid, pING3348, encoded the V_{L}V_{H}::Gel fusion protein. The DNA sequence at the fusion junction was verified by direct DNA sequencing.

### C. Gel::V_{H}V_{L}

The plasmid pING3755 [Example 14B(iii)], which contains the gelonin gene with an engineered *Eag*I site at its 3'-end, was cut with *Eag*I, treated with T4 polymerase, and digested with *Nco*I. The approximately 650 bp DNA fragment containing the 3'-end of the gelonin gene was purified on an agarose gel. The plasmid pING4639 (Example 16A) encoding the fusion Gel::SLT::V_{H}V_{L} was cut with *Xho*I and then partially digested with *Fsp*I. The approximately 730 bp DNA fragment containing all of the he3 V_{H}V_{L} gene was then purified in an agarose gel (a single *Fsp*I restriction site occurs in the V_{H} gene segment, and the purified he3 V_{H}V_{L} gene was separated from the incomplete gene segment which was approximately 660 bp). The two DNA fragments from pING3755 and pING4639 were ligated into the vector pING3825 that had been digested with *Nco*I plus *Xho*I and purified on an agarose gel. The plasmid pING3350 was generated which encoded the Gel::V_{H}V_{L} fusion protein. The DNA sequence at the fusion junction was verified by direct DNA sequencing.

### D. Gel::V_{L}V_{H}

Plasmid pING3336 which encodes the he3 V_{L}V_{H} single chain antibody gene was cut with SstI and AseI, and the approximately 5500 bp DNA fragment containing the 3'-end of V_{L}V_{H} and downstream vector sequences was purified. (pING3336 is identical to pING4640 except that the V_{L}V_{H} gene encodes six histidine residues in frame at the carboxyl-terminus). Plasmid PING4627 (Example 15A) served as a substrate for PCR amplification of the V_{H} gene segment. Plasmid pING4627 was amplified with the two oligonucleotide primers HUK-7 (SEQ ID NO: 92) and JK1-HindIII (SEQ ID NO: 87), the resultant product was treated with T4 polymerase and cut with AseI, and the 86 bp DNA fragment containing the 5'-end of the V_{L} was purified. The DNa fragments from pING3336 and pING4627 were ligated to the approximately 2350 bp DNA fragment of pING3755 generated by digestion with EagI, treatment with T4 polymerase and subsequent digestion with SstI. The resultant vector containing the Gel::V_{L}V_{H} gene fusion was named pING4652. The DNA sequence of pING4652 was verified at ligation juctions.

### E. Gel::kappa, Fd

The direct gene fusion which encodes Gel::kappa, Fd was also assembled from DNA segments from three plasmids. Plasmid pING3764 (Example 15C) was digested with *Hind*III and *Xho*I, and the approximately 1200 bp DNA fragment encoding the 3'-end of the kappa gene and the Fd gene was purified. Plasmid pING4652, which encodes a direct gene fusion of gelonin to the he3 SCA gene V_{L}V_{H}, was cut with *Bgl*II and *Hind*III, and the approximately 850 bp DNA fragment encoding the 3'-end of the gelonin gene and the V_{L} region of kappa was purified. The DNA fragments from pING3764 and pING4652 were ligated into the vector fragment from pING3825 (Example 2C) that had been digested with *Bgl*II and *Xho*I to generate pING3784 encoding Gel::kappa, Fd.

### F. Gel::Fd. kappa

Plasmid pING3768 (Example 15F), which encodes the fusion protein Gel::RMA::Fd, kappa, was cut with NdeI and NheI, and the DNA segment containing the majority of the he3 Fd gene, the he3 kappa gene and a portion of the tetracycline resistance gene of the vector was purified. Plasmid pING3350, which is described in section C above, was cut with NdeI and PstI, and the DNA fragment containing the 5'-end of the he3 Fd gene linked to the gelonin gene was purified. The DNA fragments from pING3350 and pING3768 were ligated into the vector fragment from pING4633 (Example 16D) that had been cut with Nhel and Pstl to generate pING3789. Plasmid pING3789 encodes the fusion protein Gel::Fd, kappa.

### Example 19

### Alternative Cathepsin Cleavable Linkers

The segment of rabbit muscle aldolase chosen for the RMA linker described herein is known to contain peptide sequences susceptible to digestion with cathepsins. Other cathepsin-cleavable protein segments are effective targets for intracellular cleavage, and two particular amino acid sequences were included as cleavable linkers in additional immunofusions of the invention. These are the amino acid sequence KPAKFFRL (SEQ ID NO: 141 ("CCF") and KPAKFLRL (SEQ ID NO: 142) ("CCL"). Two oligonucleotides were synthesized that encode these peptide segments. Degeneracy was introduced at one nucleotide position in each synthetic primer to allow the appropriate amino acid to be encoded at the particular amino acid position in which CCF and CCL differ. The two oligonucleotides 5'-GGCCGCAAAGCCGGCTAAGTTCTT(A/C)CGTCTGAGT-3' (SEQ ID NO: 143) and 5'-ACTCAGACG(G/T)AAGAACTTAGCCGGCTTTGC-3' (SEQ ID NO: 144). The oligonucleotide linkers were then used to assemble a family of fusion gene expression vectors encoding: Gel::CCL::kappa, Fd; Gel::CCF::kappa, Fd; Gel::CCF::V_{L}V_{H}; and Gel::CCL::V_{H}V_{L}.

The CCL and CCF linkers were also included in fusion vectors where the antigen-binding domain of the fusion protein was at the N-terminus of the fusion to generate expression vectors encoding immunofusions such as V_{L}V_{H}::CCL::Gel.

Several of the fusion proteins with the CCL and CCF linkers were tested for cytotoxicity on the T cell lines HSB2 and PBMC and were comparable in activity to the fusion proteins containing the RMA linker.

### Example 20

### Expression And Purification Of Gelonin Immunofusions

### A. Expression Of Gelonin Immunofusions

Each of the gelonin gene fusions whose construction is described in Example 15 was co-expressed with its pair H65 Fab gene in arabinose-induced *E. coli* strain E104.

Expression products of the gene fusions were detected in the supernatant of induced cultures by ELISA. Typically, a plate was coated with antibody recognizing gelonin. Culture supernatant was applied and bound Fab was detected with antibody recognizing human kappa coupled to horseradish peroxidase. H65 Fab fragment chemically conjugated to gelonin was used a standard. Alternative ELISA protocols involving coating a plate with antibody recognizing either the kappa or Fd or involving a detection step with anti-human Fd rather than anti-human kappa yielded similar results. Only properly assembled fusion protein containing gelonin, kappa and Fd was detected by this assay. Unassociated chains were not detected.

The fusion protein produced from induced cultures containing expression vectors pING4406, 4407, 4408, and 4410 in *E. coli* E104 accumulated at about 20-50 ng/ml. The fusion proteins expressed upon induction of pING3754, 3334, 3758 and 3759 (but not pING3757) were expressed at much higher levels, at about 100 to 500 ng/ml. A fusion protein of about 70,000 Kd was detected in the concentrated *E. coli* culture supernatant by immunostaining of Western blots with either anti-human kappa or anti-gelonin antibodies.

The Gelonin::SLT::Fd' (kappa) fusion protein from pING3754 (ATCC 69102) was purified from induced 10 L fermentation broth. The 10 L fermentation broth was concentrated and buffer exchanged into 10mM phosphate buffer at pH 7.0, using an S10Y10 cartridge (Amicon) and a DC10 concentrator. The supernatant was purified by passing the concentrated supernatant through a DE52 column (20 x 5 cm) equilibrated with 10 mM sodium phosphate buffer at pH 7.0. The flow-through was then further purified and concentrated by column chromatography on CM52 (5 x 10 cm) in 10 mM phosphate buffer. A 0 - 0.2 M linear gradient of MaCl was used to the elute the fusion protein, and fractions containing the fusion protein were pooled and loaded onto a Protein G column (1ml). The fusion protein was eluted from protein G with 0.2 M sodium citrate, pH 5.5 and then 0.2 M sodium acetate, pH 4.5, and finally, 0.2 M glycine, pH 2.5. The Gelonin::RMA::Fd' (kappa) and Gelonin::RMA::kappa (Fd') fusions proteins were purified from fermentation broths by similar methods except that the CM52 column step was eliminated, and the DE52 column was equilibrated with 100mM sodium phosphate buffer at pH 7.0. The fusion proteins were not purified to homogeneity.

Each of the three purified fusion proteins was then assayed for activity in the RLA assay and for cytotoxicity against the T-cell line HSB2. (T cells express the CD5 antigen which is recognized by H65 antibody.) The RLA assay was performed as described in Example 4 and results of the assay are presented below in Table 12.

**Table 12**

| Fusion Protein | IC50(pM) |
|---|---|
| rGelonin | 11 |
| Gelonin::SLT::Fd (kappa) | 19 |
| Gelonin::RMA::Fd (kappa) | 28 |
| Gelonin::RMA::kappa (Fd) | 10 |

Two fusion proteins were tested in whole cell cytotoxicity assays performed as described in Example 6 (Table 13). As shown in Table 13, the fusion proteins were active. Gelonin::SLT::Fd(kappa) killed two T cell lines, HSB2 and CEM, with respective IC₅₀s 2-fold (HSB2) or 10-fold (CEM) higher than that of the gelonin chemically linked to H65. See Table 13 below for results wherein IC₅₀ values were adjusted relative to the amount of fusion protein in each sample.

**Table 13**

| IC₅₀ (pMT) | | |
|---|---|---|
| Fusion Protein | HSB2 Cells | CEM Cells |
| he3Fab-Gel_{A50(C44)} | 165 | 173 |
| Gelonin::SLT::Fd (kappa) | 180 | 1007 |
| Gelonin::RMA::Fd (kappa) | 150 | NT |

These fusion protein showed similar activity on peripheral blood mononuclear cells (data not shown).

### B. Purification of Immunofusions

### (i) Immunofusions Comprising cH65Fab'

Immunofusions comprising a cH65Fab' fragment were purified from cell-free supernatants by passing the supernatant through a CM Spheradex (Sepacor) column (5cm x 3cm), equilibrated in 10 Mm Na phosphate at pH 7.0. Immunofusion proteins bind to the column and are eluted with 10 mM Na phosphate, 200 mM NaCl, pH 7.0. The eluate was diluted two-fold with 20 Mm HEPES, 3 M ammonium sulfate, pH 7.6 and loaded onto a phenyl sepharose fast flow (Pharmacia) column (2.5 x3.5 cm), equilibrated in 20 mM HEPES, 1.2 M ammonium sulfate, pH 7.0. The column was next washed with 20 mM Hepes, 1.2 M ammonium sulfate, pH 7.0 and eluted with 20 mM HEPES, 0.9 M ammonium sulfate, pH 7.0. The phenyl sepharose eluate was concentrated to a volume of 2-4 ml in an Amicon stirred cell fitted with a YM10 membrane. The concentrated sample was loaded onto an S-200 (Pharmacia) column (3.2x 38 cm), equilibrated in 10 mm Na phosphate, 150 mm NaCl, pH 7.0. The column was run in the same buffer and fractions were collected. Fractions containing the fusion protein of desired molecular weight were combined. For example, by selection of appropriate column fractions, both monovalent (gelonin-Fab') and bivalent (gelonin₂-F(ab')₂ forms encoded by pING3758 were purified.

### (ii) Immunofusions Comprising hc3Fab

Immunofusions comprising he3Fab were purified as in the preceding section with the exception that the phenyl sepharose column was eluted with 20 mM HEPES, 1.0 M ammonium sulfate, pH 7.0.

### (iii) Immunofusions Comprising SCA

Cell-free supernatant was passed through a CM spheradex column (5 x 3 cm), equilibrated with 10 mM Na phosphate, pH 7.0. single-chain antibody binds to the column which is then washed with 10 mM Naphosphate, 45 mM NaCl, pH 7.0. The fusion protein was then eluted with 10 mM Naphosphate, 200 mM NaCl, pH 7.0. The eluate was diluted two-fold with 20 mM HePES, 3 M ammonium sulfate, pH 7.0 and loaded onto a butyl sepharose Fast Flow (Pharmacia) column (2.5 x 4.1 cm) equilibrated in 20 mM HEPES, 1.5 M ammonium sulfate, pH 7.0. The column was then washed with 20 mM HEPES, 1.0 M ammonium sulfate, pH 7.0 and eluted with 20 mM HEPES pH 7.0. The butyl sepharose eluate was concentrated to a volume of 2-4 ml in an Amicon stirred cell fitted with a YM10 membrane. The concentrated sample was loaded onto an S-200 (Pharmacia) column (3.2 x 38 cm) equilibrated in 10 mM Na phosphate, 150 mM NaCl, pH 7.0. The column was then run in the same buffer and the fractions were collected. Some of the fractions were analyzed by SDS-PAGE to determine which fractions to pool together for the final product.

### Example 21

### Activity of Gelonin Immunofusions

A concern in constructing immunofusions comprising any RIP is that the targeting and enzymatic activities of the components of the fusion protein may be lost as a result of the fusion. For example, attachment of an RIP to the amino terminus of an antibody may affect the antigen-binding (complementarity-determining regions) of the antibody and may also result in steric hinderance at the active site. Similarly, the activity of an RIP may be hindered by attachment of an antibody or antibody portion. For example, RIPS chemically conjugated to antibodies via a disulfide bridge are typically inactive in the absence of reducing agents. In order to assess the foregoing in immunofusions of the present invention, such proteins were subjected to assays to determine their enzymatic, binding, and cytotoxic activities.

### A. Reticulocyte Lysate Assay

The enzymatic activity of immunofusions comprising gelonin was assayed using the reticulocyte lysate assay (RLA) describe above. As noted in Example 4, the RLA assay measures the inhibition of protein synthesis in a cell-free system using endogenous globin mRNA from a rabbit red blood cell lysate. Decreased incorporation of tritiated leucine (³H-Leu) was measured as a function of toxin concentration. Serial log dilutions of standard toxin (the 30 kD form of ricin A-chain, abbreviated as RTA 30), native gelonin, recombinant gelonin (rGelonin or rGel) and gelonin analogs were tested over a range of 1 µg/ml to 1 pg/ml. Samples were tested in triplicate, prepared on ice, incubated for 30 minutes at 37°C, and then counted on an Inotec Trace 96 cascade ionization counter. By comparison with an uninhibited sample, the picomolar concentration of toxin (pM) which corresponds to 50% inhibition of protein synthesis (IC₅₀) was calculated.

Representative data for various immunotoxins of the invention are shown below in Table 14 .

**Table 14**

| Immunotoxin | Lot No. | IC₅₀ (pM) |
|---|---|---|
| rGel::RMA::SCA(V_{H}-V_{L}) | AB1136 | 12 |
| rGel::RMA::SCA(V_{L}-V_{H}) | AB1137 | 18 |
| rGel::SLT::SCA(V_{H}-V_{L}) | AB1133 | 26 |
| rGel::RMA::SCA(V_{L}-V_{H}) | AB1124 | 33 |
| rGel::RMA::K+Fd'(cH65Fab') | AB1122 | 54 |
| rGel::SLT::K+Fd(he3Fab) | AB1160 | 40 |
| rGel::RMA::K+Fd(he3Fab) | AB1149 | 33 |
| rGel::RMA::Fd+K(he3Fab) | AB1163 | 14 |
| rGel::Fd'+K(cH65Fab') | AB1123 | 45 |

Contrary to the expectations discussed above, gelonin immunofusions of the invention exhibit enzymatic activity which is comparable to the activities of native and recombinant gelonin shown in Example 4. This was true for fusions made with either the reducible (SLT) or non-reducible (RMA) linkers.

### B. Binding Activity of Immunofusions

Several immunofusions according to the present invention were assayed for their ability to compete with labelled antibody for binding to CD5-positive cells. The Kd of the immunofusions was estimated by three different means as shown in Table 15. The first Kd estimation (Kd₁ in Table 15) was obtained by competition with fluorescein-labelled H65 IgG for binding to MOLT-4X cells (ATCC CRL 1582) according to the procedure reported in Knebel, *et al., Cytometry Suppl., 1:* 68 (1987), incorporated by reference herein.

The second Kd measurement (Kd₂ in Table15) was obtained by Scatchard analysis of competition of the immunofusion with ¹²⁵I-cH65 IgG for binding on MOLT-4M cells as follows. A 20 µg aliquot of chimeric H65 IgG (cH65 IgG) was iodinated by exposure to 100 µl lactoperoxidase-glucose oxidase immobilized beads (Enzymobeads, BioRad), 100 µl of PBS, 1.0 mci I¹²⁵ (Amersham, IMS30), 50 µl of 55 mM b-D-glucose for 45 minutes at 23°C. The reaction was quenched by the addition of 20 µl of 105 mM sodium metabisulfite and 120 mM potassium iodine followed by centrifugation for 1 minute to pellet the beads. ¹²⁵I-cH65 IgG was purified by gel filtration using a 7 ml column of sephadex G25, eluted with PBS (137 mM NaCl, 1.47 mM KH₂PO₄, 8.1 mM Na₂HPO₄, 2.68 mM KCl at pH 7.2-7.4) plus 0.1% BSA. ¹²⁵I-cH65 IgG recovery and specific activity were determined by TCA precipitation.

Competitive binding was performed as follows: 100 µl of Molt-4M cells were washed two times in ice-cold DHB binding buffer (Dubellco's modified Eagle's medium (Gibco, 320-1965PJ), 1.0% BSA and 10 mM Hepes at pH 7.2 -7.4). Cells were resuspended in the same buffer, plated into 96 v-bottomed wells (Costar) at 3 x 10⁵ cells per well and pelleted at 4°C by centrifugation for 5 min at 1,000 rpm using a Beckman JS 4.2 rotor; 50 µl of 2X-concentrated 0.1 nM ¹²⁵I-cH65 IgG in DHB was then added to each well and competed with 50 µl of 2X - concentrated cH65 IgG in DHB at final protein concentrations from 100 nM to 0.0017 nM. The concentrations of assayed proteins were determined by measuring absorbance (A₂₈₀ and using an extinction coefficient of 1.0 for fusion proteins, 1.3 for Fab, and 1.22 for Fab conjugated to gelonin. Also, protein concentrations were determined by BCA assay (Pierce Chemical) with bovine serum albumin as the standard. Binding was allowed to proceed at 4°C for 5 hrs and was terminated by washing cells three times with 200 µl of DHB binding buffer by centrifugation for 5 min. at 1,000 rpm. All buffers and operations were at 4°C. Radioactivity was determined by solubilizing cells in 100 µl of 1.0 M NaOH and counting in a Cobra II auto gamma counter (Packard). Data from binding experiments were analyzed by the weighted nonlinear least squares curve fitting program, MacLigand, a Macintosh version of the computer program "Ligand" from Munson, *Analyt. Biochem.*, 107:220 (1980), incorporated by reference herein.

Finally, the Kd (Kd₃ in the Table) was estimated by examination of the ED₅₀ values obtained from separate competition binding assays, performed as described in the previous paragraph. All three measurements are shown in Table 15 below:

**Table 15**

| Molecule Type | Kd₁ | Kd₂ | Kd₃ |
|---|---|---|---|
| H65 IgG | 1.6 | ND | ND |
| cH65 IgG | ND | 3.0 | 2.5 |
| cH65Fab' | 4.0 | 14.0 | ND |
| cH65Fab'-rGel_{A50(C44)} | 3.5 | 13.0 | ND |
| rGel::RMA::K+Fd'(cH65Fab') | 16.0 | ND | 100 |
| he3Fab | 1.20 | 2.60 | ND |
| he3Fab-rGel_{A50(C44)} | 1.10 | 2.70 | ND |
| rGel::RMA::K+Fd'(he3Fab) | 2.60 | ND | 5.0 |
| rGel::SLT::K+Fd(he3Fab) | ND | ND | 30 |
| SCA(V_{L}-V_{H}) | 2.20 | ND | 30 |
| rGel::RMA::SCA(V_{H}-V_{L}) | 3.50 | ND | 20 |
| rGel::RMA::SCA(V_{L}-V_{H}) | 4.70 | ND | 30 |
| SCA ( V_{L}-V_{H}) | ND | ND | 20 |
| rGel::RMA::SCA(V_{L}-V_{H}) | 2.30 | ND | ND |
| ND = not determined | | | |

The results presented in Table 15 suggest that Fab and SCA antibody forms may retain substantial binding activity even when fused to an RIP.

### C. Comparative Cvtotoxicity Assays

Fusion proteins and immunoconjugates according to the present invention were used in a comparative cytoxicity assay. Two types of assays were conducted, one targeting T cell line HSB2, and the other targeting lectin-activated peripheral blood mononuclear cells (PBMC) according to procedures in Example 6. The results of the assays are presented below in Tables 16a, 16b and 16c.

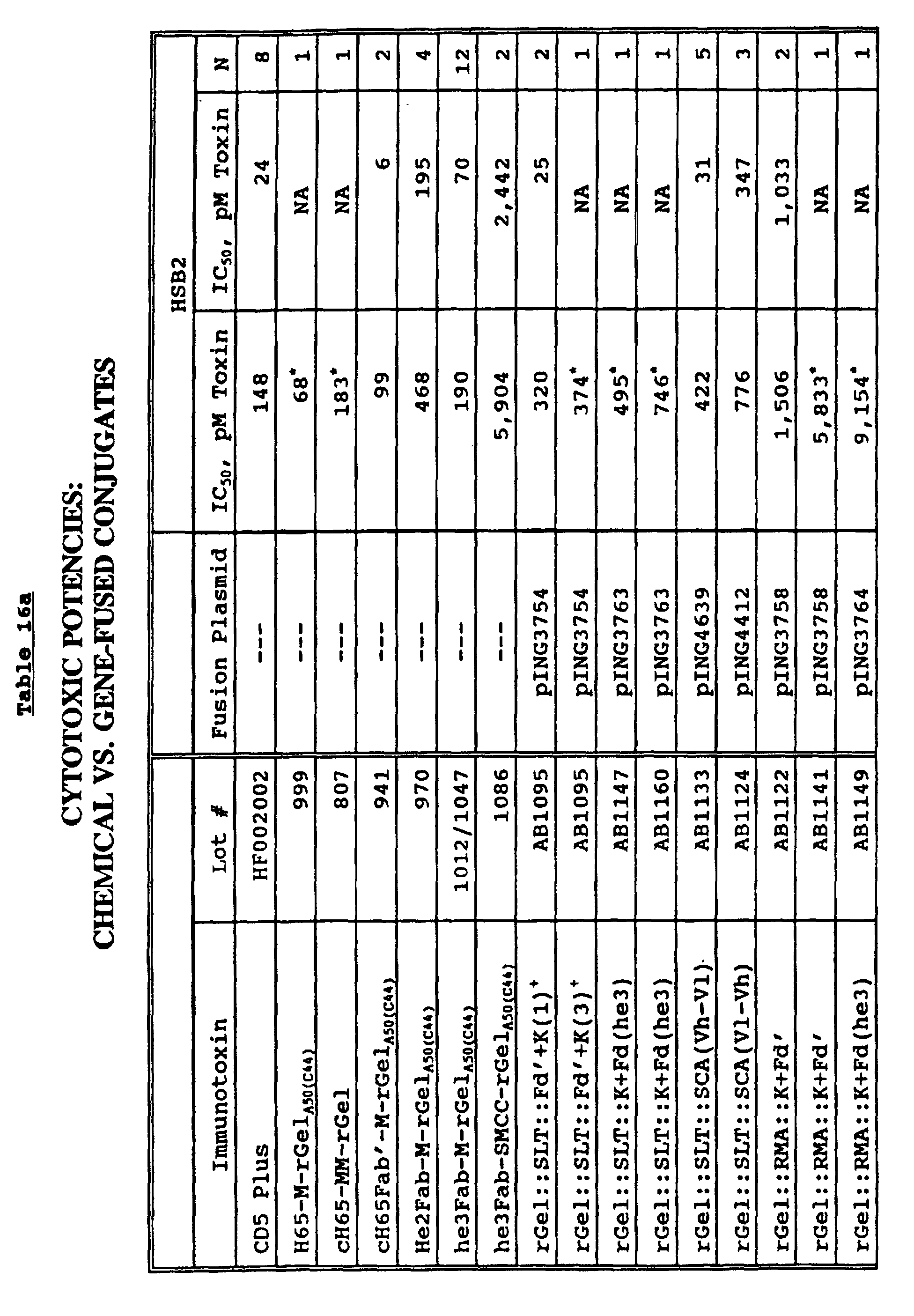

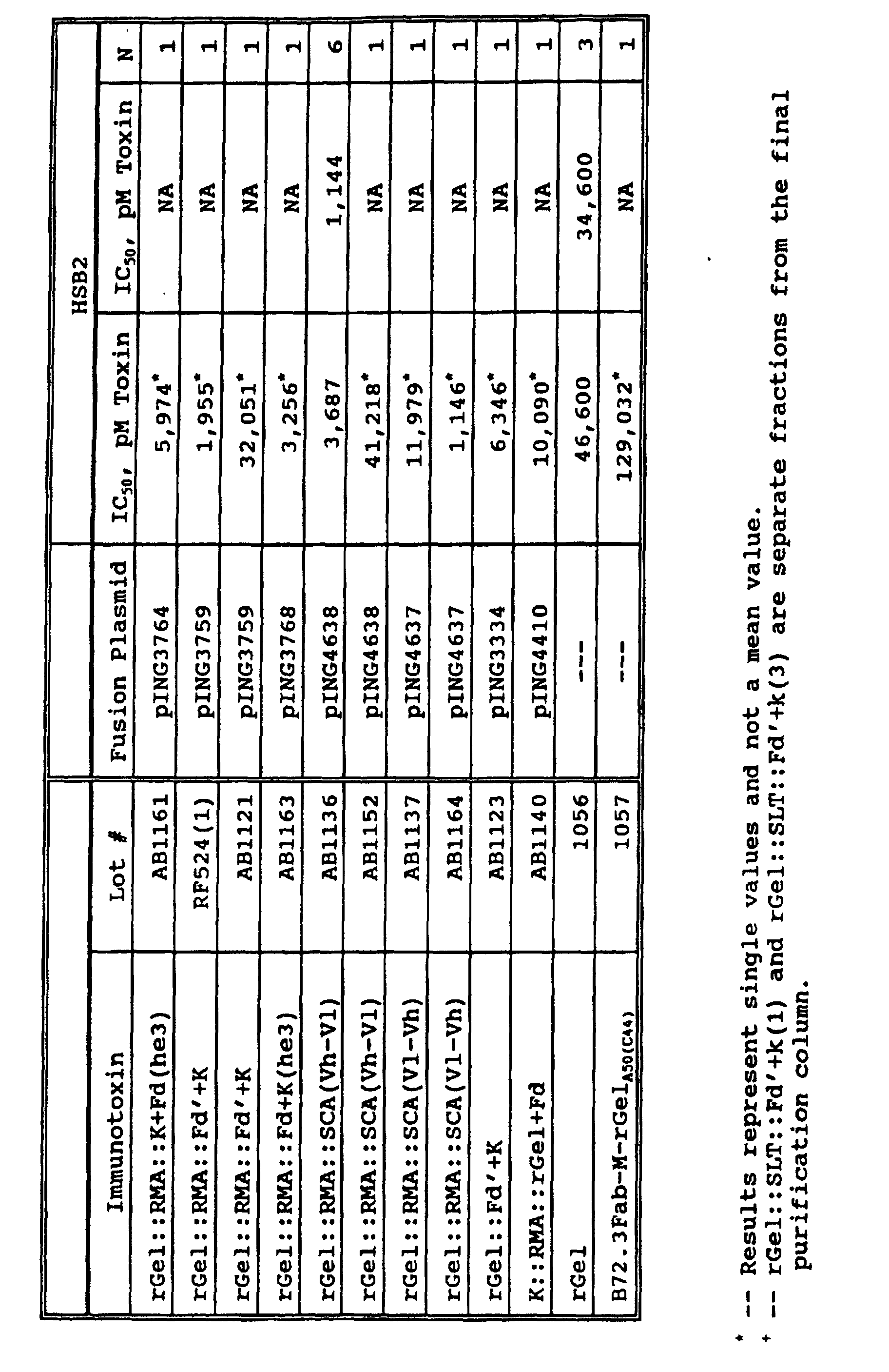

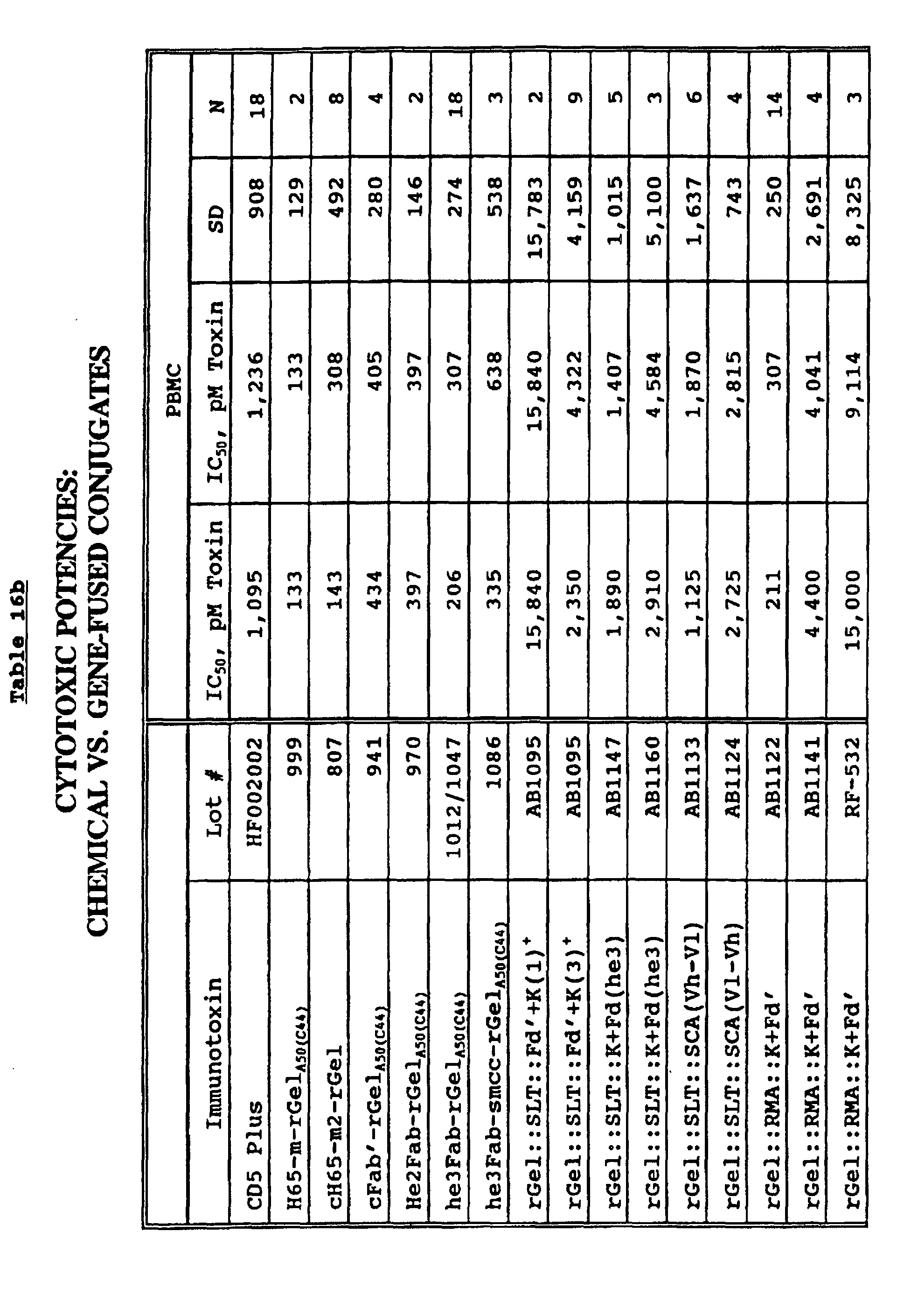

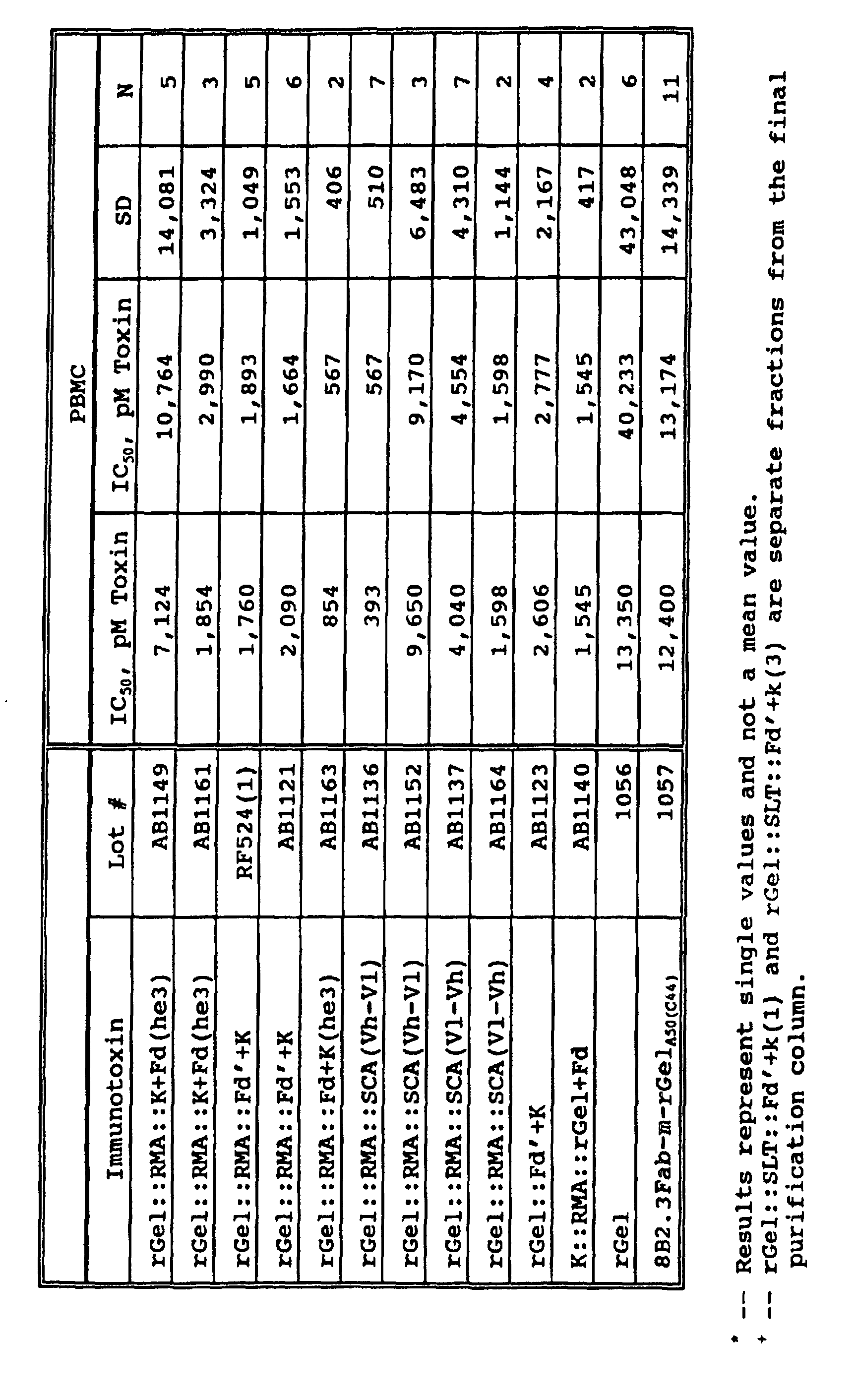

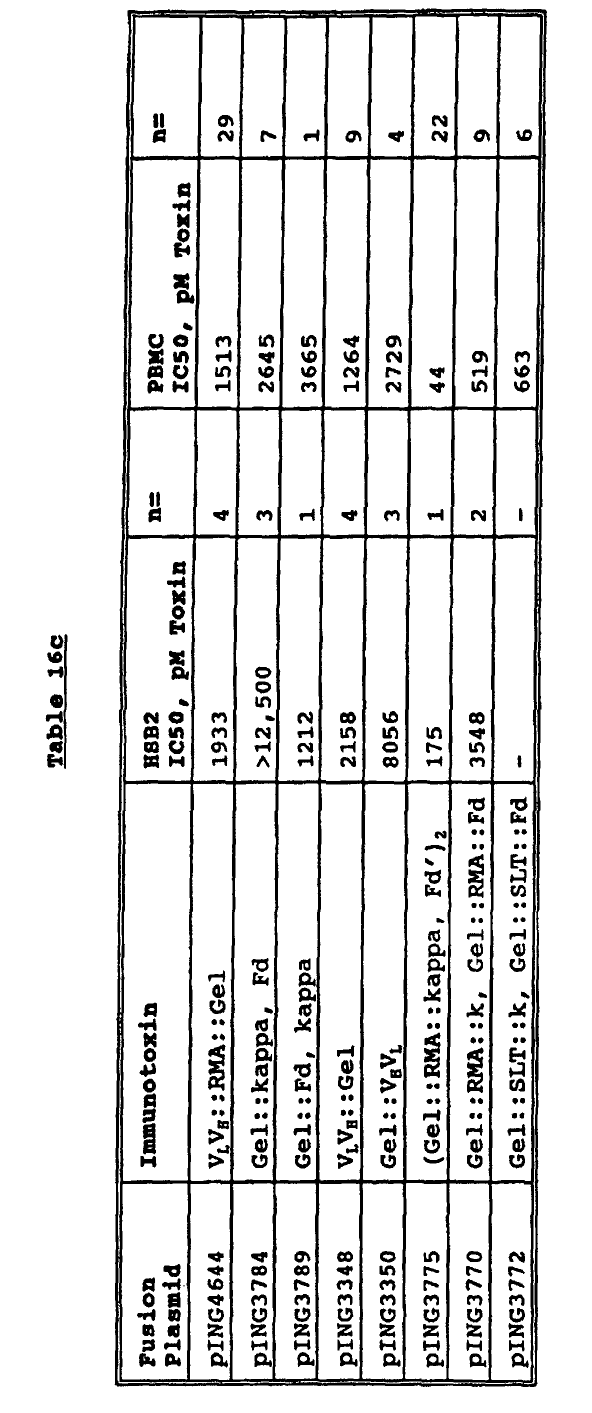

## Claims

1. A fusion protein comprising gelonin fused to the carboxy terminal end of an antigen-binding portion of the antibody designated he3 obtainable from cell line ATTC HB 11206.

2. The fusion protein of Claim 1 wherein said fusion protein lacks a cleavable peptide segment linking said gelonin and said antibody portion.

3. The fusion protein of Claim 1 which is V_{L}V_{H}::Gelonin.

4. The fusion protein of Claim 1 which is V_{H}V_{L}::Gelonin.

5. A fusion protein comprising gelonin fused to the amino terminal end of an antigen-binding portion of the antibody designated he3 obtainable from cell line ATTC HB 11206, said fusion protein having the structure Gelonin::kappa,Fd.

6. A fusion protein comprising gelonin fused to the amino terminal end of an antigen-binding portion of the antibody designated he3 obtainable from cell line ATTC HB 11206, said fusion protein having the structure Gelonin::Fd,kappa.

7. A fusion protein comprising gelonin fused to the amino or carboxy terminal end of an antigen-binding portion of an antibody, said fusion protein further comprising the cleavage site-containing peptide segment KPAKFLRL between said gelonin and said antibody portion.

8. A fusion protein comprising gelonin fused to the amino or carboxy terminal end of an antigen-binding portion of an antibody, said fusion protein further comprising the cleavage site-containing peptide segment KPAKFFRL between said gelonin and said antibody portion.

9. The fusion protein (Gelonin::RMA::kappa,Fd')₂, wherein "RMA" represents the sequence PSGQAGAAASESLFISNHAY.

10. The fusion protein (Gelonin::RMA::Fd',kappa)₂, wherein "RMA" represents the sequence PSGQAGAAASESLFISNHAY.

11. A fusion protein comprising gelonin fused to the amino terminal end of an antigen-binding portion of the antibody designated he3 obtainable from cell line ATTC HB 11206, said fusion protein having the structure Gelonin::RMA::kappa,Fd, wherein "RMA" represents the sequence PSGQAGAAASESLFISNHAY.

12. A fusion protein comprising gelonin fused to the amino terminal end of an antigen-binding portion of the antibody designated he3 obtainable from cell line ATTC HB 11206, said fusion protein having the structure Gelonin::RMA::Fd,kappa, wherein "RMA" represents the sequence PSGQAGAAASESLFISNHAY.

13. A fusion protein comprising gelonin fused to the amino terminal end of an antigen-binding portion of the antibody designated he3 obtainable from cell line ATTC HB 11206, said fusion protein having the structure Gelonin::SLT::kappa,Fd, wherein SLT represents the sequence CHHHASRVARMASDEFPSMC.

14. A fusion protein comprising gelonin fused to the amino terminal end of an antigen-binding portion of the antibody designated he3 obtainable from cell line ATTC HB 11206, said fusion protein having the structure Gelonin::SLT::Fd kappa, wherein SLT represents the sequence CHHHASRVARMASDEFPSMC.

15. The fusion protein V_{H}V_{L}::SLT::Gelonin, wherein SLT represents the sequence CHHHASRVARMASDEFPSMC.

16. The fusion protein V_{L}V_{H}::SLT::Gelonin, wherein SLT represents the sequence CHHHASRVARMASDEFPSMC.

17. The fusion protein V_{H}V_{L}::RMA::Gelonin, wherein "RMA" represents the sequence PSGQAGAAASESLFISNHAY.

18. The fusion protein V_{L}V_{H}::RMA::Gelonin, wherein "RMA" represents the sequence PSGQAGAAASESLFISNHAY.

19. A polynucleotide encoding the fusion protein of any preceding claim.

20. A host cell transformed or transfected with the polynucleotide encoding the fusion protein of Claim 19.

## Patentansprüche

1. Fusionsprotein, umfassend Gelonin, fusioniert mit dem Carboxy-terminalen Ende eines Antigen-bindenden Teils eines Antikörpers, bezeichnet als he3, erhältlich aus der Zelllinie ATTC HB 11206.

2. Fusionsprotein nach Anspruch 1, worin dem Fusionsprotein ein spaltbares Peptidsegment fehlt, welches das Gelonin und den Antikörperteil verbindet.

3. Fusionsprotein nach Anspruch 1, bei dem es sich um V_{L}V_{H}::Gelonin handelt.

4. Fusionsprotein nach Anspruch 1, bei dem es sich um V_{H}V_{L}::Gelonin handelt.

5. Fusionsprotein, umfassend Gelonin, fusioniert an das amino-terminale Ende eines Antigen-bindenden Teils des Antikörpers, bezeichnet als he3, erhältlich aus der Zelllinie ATTC HB 11206, wobei das Fusionsprotein die Struktur Gelonin::kappa,Fd aufweist.

6. Fusionsprotein, umfassend Gelonin, fusioniert an das amino-terminale Ende eines Antigen-bindenden Teils des Antikörpers, bezeichnet als he3, erhältlich aus der Zelllinie ATTC HB 11206, wobei das Fusionsprotein die Struktur Gelonin::Fd,kappa aufweist.

7. Fusionsprotein, umfassend Gelonin, fusioniert an das amino- oder carboxy-terminale Ende eines Antigen-bindenden Teils eines Antikörpers, wobei das Fusionsprotein zusätzlich das die Spaltstelle enthaltende Peptidsegment KPAKFLRL zwischen dem Gelonin und dem Antikörperteil enthält.

8. Fusionsprotein, umfassend Gelonin, fusioniert an das amino- oder carboxy-terminale Ende eines Antigen-bindenden Teils eines Antikörpers, wobei das Fusionsprotein zusätzlich das die Spaltstelle enthaltende Peptidsegment KPAKFFRL zwischen dem Gelonin und dem Antikörperteil enthält.

9. Fusionsprotein (Gelonin::RMA::kappa,Fd')₂, worin "RMA" die Sequenz PSGQAGAAASESLFISNHAY darstellt.

10. Fusionsprotein (Gelonin::RMA::Fd',kappa)₂, worin "RMA" die Sequenz PSGQAGAAASESLFISNHAY darstellt.

11. Fusionsprotein, umfassend Gelonin, fusioniert an das amino-terminale Ende eines Antigen-bindenden Teils des Antikörpers, bezeichnet als he3, erhältlich aus der Zelllinie ATTC HB 11206, wobei das Fusionsprotein die Struktur Gelonin::RMA::kappa,Fd aufweist, worin "RMA" die Sequenz PSGQAGAAASESLFISNHAY darstellt.

12. Fusionsprotein, umfassend Gelonin, fusioniert an das amino-terminale Ende eines Antigen-bindenden Teils des Antikörpers, bezeichnet als he3, erhältlich aus der Zelllinie ATTC HB 11206, wobei das Fusionsprotein die Struktur Gelonin::RMA::Fd,kappa, aufweist, worin "RMA" die Sequenz PSGQAGAAASESLFISNHAY darstellt.

13. Fusionsprotein, umfassend Gelonin, fusioniert an das amino-terminale Ende eines Antigen-bindenden Teils des Antikörpers, bezeichnet als he3, erhältlich aus der Zelllinie ATTC HB 11206, wobei das Fusionsprotein die Struktur Gelonin::SLT::kappa,Fd aufweist, worin SLT die Sequenz CHHHASRVARMASDEFPSMC darstellt.

14. Fusionsprotein, umfassend Gelonin, fusioniert an das amino-terminale Ende eines Antigen-bindenden Teils des Antikörpers, bezeichnet als he3, erhältlich aus der Zelllinie ATTC HB 11206, wobei das Fusionsprotein die Struktur Gelonin::SLT::Fd,kappa aufweist, worin SLT die Sequenz CHHHASRVARMASDEFPSMC darstellt.

15. Fusionsprotein V_{H}V_{L}::SLT::Gelonin, worin SLT die Sequenz CHHHASRVARMASDEFPSMC darstellt.

16. Fusionsprotein V_{L}V_{H}::SLT::Gelonin, worin SLT die Sequenz CHHHASRVARMASDEFPSMC darstellt.

17. Fusionsprotein V_{H}V_{L}::RMA::Gelonin, worin "RMA" die Sequenz PSGQAGAAASESLFISNHAY darstellt.

18. Fusionsprotein V_{L}V_{H}::RMA::Gelonin, worin "RMA" die Sequenz PSGQAGAAASESLFISNHAY darstellt.

19. Polynukleotid, kodierend das Fusionsprotein nach einem der vorstehend Ansprüche.

20. Wirtszelle, transformiert oder transfiziert mit dem Polynukleotid, kodierend das Fusionsprotein, aus Anspruch 19.

## Revendications

1. Protéine de fusion comprenant de la gélonine fusionnée à l'extrémité carboxy-terminale d'une portion de liaison pour antigène de l'anticorps appelé he3 susceptible d'être obtenu à partir de la lignée cellulaire ATTC HB 11206.

2. Protéine de fusion selon la revendication 1, laquelle protéine de fusion ne possède pas de segment peptidique clivable liant ladite gélonine et ladite portion d'anticorps.

3. Protéine de fusion selon la revendication 1, laquelle est V_{L}V_{H}::Gélonine.

4. Protéine de fusion selon la revendication 1, laquelle est V_{H}V_{L}::Gélonine.

5. Protéine de fusion comprenant de la gélonine fusionnée à l'extrémité amino-terminale d'une portion de liaison pour antigène de l'anticorps appelé he3 susceptible d'être obtenu à partir de la lignée cellulaire ATTC HB 11206, ladite protéine de fusion ayant la structure Gélonine::kappa,Fd.

6. Protéine de fusion comprenant de la gélonine fusionnée à l'extrémité amino-terminale d'une portion de liaison pour antigène de l'anticorps appelé he3 susceptible d'être obtenu à partir de la lignée cellulaire ATTC HB 11206, ladite protéine de fusion ayant la structure Gélonine::Fd,kappa.

7. Protéine de fusion comprenant de la gélonine fusionnée à l'extrémité amino ou carboxy-terminale d'une portion de liaison pour antigène d'un anticorps, ladite protéine de fusion comprenant en outre le segment peptidique contenant un site de clivage KPAKFLRL entre ladite gélonine et ladite portion d'anticorps.

8. Protéine de fusion comprenant de la gélonine fusionnée à l'extrémité amino ou carboxy-terminale d'une portion de liaison pour antigène d'un anticorps, ladite protéine de fusion comprenant en outre le segment peptidique contenant un site de clivage KPAKFFRL entre ladite gélonine et ladite portion d'anticorps.

9. Protéine de fusion (Gélonine::RMA::kappa,Fd')₂, dans laquelle "RMA" représente la séquence PSGQAGAAASESLFISNHAY.

10. Protéine de fusion (Gélonine::RMA::Fd',kappa)₂, dans laquelle "RMA" représente la séquence PSGQAGAAASESLFISNHAY.

11. Protéine de fusion comprenant de la gélonine fusionnée à l'extrémité amino-terminale d'une portion de liaison pour antigène de l'anticorps appelé he3 susceptible d'être obtenu à partir de la lignée cellulaire ATTC HB 11206, ladite protéine de fusion ayant la structure Gélonine::RMA::kappa,Fd, dans laquelle "RMA" représente la séquence PSGQAGAAASESLFISNHAY.

12. Protéine de fusion comprenant de la gélonine fusionnée à l'extrémité amino-terminale d'une portion de liaison pour antigène de l'anticorps appelé .he3 susceptible d'être obtenu à partir de la lignée cellulaire ATTC HB 11206, ladite protéine de fusion ayant la structure Gélonine::RMA::Fd,kappa, dans laquelle "RMA" représente la séquence PSGQAGAAASESLFISNHAY.

13. Protéine de fusion comprenant de la gélonine fusionnée à l'extrémité amino-terminale d'une portion de liaison pour antigène de l'anticorps appelé he3 susceptible d'être obtenu à partir de la lignée cellulaire ATTC HB 11206, ladite protéine de fusion ayant la structuré Gélonine::SLT::kappa,Fd, dans laquelle SLT représente la séquence CHHHASRVARMASDEFPSMC.

14. Protéine de fusion comprenant de la gélonine fusionnée à l'extrémité amino-terminale d'une portion de liaison pour antigène de l'anticorps appelé he3 susceptible d'être obtenu à partir de la lignée cellulaire ATTC HB 11206, ladite protéine de fusion ayant la structure Gélonine::SLT::Fd,kappa, dans laquelle SLT représente la séquence CHHHASRVARMASDEFPSMC.

15. Protéine de fusion V_{H}V_{L}::SLT::Gélonine, dans laquelle SLT représente la séquence CHHHASRVARMASDEFPSMC.

16. Protéine de fusion V_{L}V_{H}::SLT::Gélonine, dans laquelle SLT représente la séquence CHHHASRVARMASDEFPSMC.

17. Protéine de fusion V_{H}V_{L}::RMA::Gélonine, dans laquelle "RMA" représente la séquence PSGQAGAAASESLFISNHAY.

18. Protéine de fusion V_{L}V_{H}::RMA::Gélonine, dans laquelle "RMA" représente la séquence PSGQAGAAASESLFISNHAY.

19. Polynucléotide codant pour la protéine de fusion selon l'une quelconque des revendications précédentes.

20. Cellule hôte transformée ou transfectée avec le polynucléotide codant pour la protéine de fusion selon la revendication 19.
